# EUROPEAN PATENT SPECIFICATION

(11) **EP 4 408 823 B1**
(45) Date of publication and mention of the grant of the patent: **10.06.2026**
(21) Application number: 22798244.4
(22) Date of filing: 27.09.2022
(51) Int. Cl.: C07D 209/88, C07D 401/04, C07D 403/04, C07D 409/04, A61K 31/40, A61P 35/02

(54) **NPM1-DEPENDING LEUKEMIA AGENTS**
NPM1-ABHÄNGIGE LEUKÄMIEMITTEL
AGENTS DE LEUCÉMIE DÉPENDANT DE NPM1

(30) Priority: 28.09.2021 EP 21382868
(43) Date of publication of application: 07.08.2024
(73) Proprietor: Universidad Complutense de Madrid, 28040 Madrid (ES); Fundación del Sector Público Estatal Centro Nacional de Investigaciones Oncológicas Carlos III (F.S.P. CNIO), 28029 Madrid (ES)
(72) Inventor: LÓPEZ RODRÍGUEZ, María Luz, 28040 MADRID (ES); BENHAMÚ SALAMA, Bellinda, 28100 ALCOBENDAS (ES); VAZQUEZ VILLA, María Del Henar, 45593 BARGAS (ES); ALGAR LIZANA, Sergio, 28935 MÓSTOLES (ES); SÁNCHEZ MERINO, Anabel, 46160 LLIRIA (ES); GALLARDO DELGADO, Miguel, 28045 MADRID (ES)
(74) Representative: ZBM Patents - Zea, Barlocci & Markvardsen
(86) International application number: PCT/EP2022/076831
(87) International publication number: WO 2023/052354

(56) References cited:
- ASCHE C ET AL: "Synthesis, antitumour activity and structure-activity relationships of 5H-benzo[b]carbazoles", BIOORGANIC, ELSEVIER, AMSTERDAM, NL, vol. 13, no. 3, 1 February 2005 (2005-02-01), pages 819 - 837, XP027637799, ISSN: 0968-0896, [retrieved on 20050201]
- ISSA SAMAR ET AL: "Carbazole scaffolds in cancer therapy: a review from 2012 to 2018", JOURNAL OF ENZYME INHIBITION AND MEDICINAL CHEMISTRY, vol. 34, no. 1, 1 January 2019 (2019-01-01), GB, pages 1321 - 1346, XP055894675, ISSN: 1475-6366, DOI: 10.1080/14756366.2019.1640692
- LIU YANKAI ET AL: "Asymmetric Catalysis of Diels-Alder Reactions with in Situ Generated Heterocyclic ortho -Quinodimethanes", JOURNAL OF THE AMERICAN CHEMICAL SOCIETY, vol. 133, no. 38, 28 September 2011 (2011-09-28), pages 15212 - 15218, XP055894715, ISSN: 0002-7863, DOI: 10.1021/ja206517s
- FALINI B: "Therapy-related acute myeloid leukaemia with mutated NPM1: treatment induced or de novo in origin?", vol. 22, no. 5, 1 May 2008 (2008-05-01), London, pages 891 - 892, XP055894737, ISSN: 0887-6924, Retrieved from the Internet <URL:https://www.nature.com/articles/leu200844.pdf> DOI: 10.1038/leu.2008.44

## Description

.. This application claims the benefit of European Patent Application EP21382868.4 filed on September 28, 2021.

### Technical Field

.. The present disclosure relates to compounds acting as inhibitors of NPM1 protein and, in particular, to their use for the treatment of pathological states for which a downregulation and/or cytoplasmic location reduction of this protein is indicated, such as acute myeloid leukemia (AML) and other cancers. The invention further relates to pharmaceutical compositions containing them, to their use in therapy and to a process for the preparation of such compounds.

### Background

.. The cancer process involves different cells within a tumor, from cancerous cells to normal cells, that contribute to its growth. However, cancer stem cells (CSCs) have been described as potential subtypes of cell that originate the cancer process. There are diverse neoplasms with CSCs involvement, such as breast and colorectal cancer. However, one of the most paradigmatic diseases with critical participation of CSCs is the acute myeloid leukemia (AML). AML CD34+/CD38-CSCs are the only ones capable to induce leukemia in vivo. Thus, a specific compound that abrogates the CSCs population could be an optimal therapeutic strategy against AML.

.. Acute myeloid leukemia is a myeloid haematological disease with a high rate of mortality, around 30% in 5-year rate survival. The most common mutations with an incidence higher than 50% are FLT3 (fms like tyrosine kinase 3) and NPM1 (nucleophosmin 1). In particular, NPM1 mutation is responsible for the stem cell signature characteristic of AML. The most common treatment against AML is 7+3 regimen cytarabine (an antimetabolic agent that generates DNA damage in S phase cells) and anthracycline (an intercalating agent). However, in some situations, depending on the molecular profile of the patient, FLT3 inhibitor midostaurin (for FLT3 mutant patients) can be added in therapy regimen. Unfortunately, no NPM1 inhibitors are currently used in clinical practice, and only a few drug candidates are in clinical trials (NucAnt 6L and ClGB-300) though none of them for acute myeloid leukemia.

.. Thus, in order to improve the dramatic low survival rate of AML, the development of novel targeted therapy treatments is critical for the future of the patients.

.. NPM1 mutation is considered a good prognosis marker; however, inclusive in this scenario, the 5-year rate survival of NPM1 mutation total cases is under 50% of overall survival, and the raw numbers of died AML patients are, in high proportion, NPM1 mutants due to the high incidence of this mutation. NPM1 is a nucleolar phosphoprotein involved in ribosome biogenesis or genomic stability and shuttling from nucleus to cytoplasm to develop its functions. AML mutation confers undifferentiating and gain of function properties to myeloid stem cells transforming to cancer stem cells. The NPM1 mutant form is translocated into the cytoplasm and is designated as NPM1C+ protein, interacting with molecules like cMYC, E3 ubiquitin ligase FBW7γ and inhibiting caspase-6 and capase-8.

.. Falini B. et al (Leukemia (2008), vol 22, no. 5, p. 891-892) raises the controversy of whether acute myeloid leukemia in patients with mutation in the NPM1 gene appears treatment-induced (as a direct consequence of prior chemotherapy or radiotherapy) or is a de novo disease, that is, independent leukemia that simply manifests the NPM1 mutation. This paper is an editorial, opinion piece on the origin of the disease, not on the approach to NPM1 as a treatment for AML. No chemical structures are disclosed in the document.

.. Asche C. et al (Bioorganic (2005) vol 13, no. 3, p819-837) describes the antitumor activity and structure-activity relationships of 5H-benzo[b]carbazoles. The structures therein described differ significatively of those according to the present invention as set out in the appended set of claims.

.. An object of this invention is, therefore, to provide new compounds for the treatment and/or prevention of pathological states for which a downregulation and/or cytoplasmic location reduction of NPM1 protein is indicated, such as leukemias and other cancers.

### Summary

.. Inventors have found new compounds bearing a tetrahydrocarbazole core that inhibit NPM1 expression and trigger acute myeloid cancer stem cells death and therefore can be used in therapy. Since NPM1 protein is considered to be responsible for the stem cell signature characteristic of acute myeloid leukemia (AML), the compounds of the invention, represent a promising approach for the treatment and/or prevention of AML.

.. Thus, in a first aspect the present disclosure provides a compound of formula (I), a pharmaceutically acceptable salt thereof, or any enantiomer or mixtures of enantiomers, either of the compound of formula (I) or of any of its pharmaceutically acceptable salts. 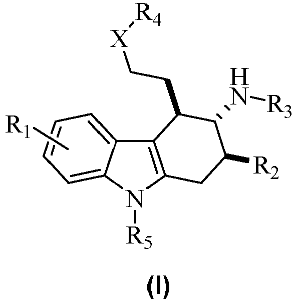 wherein:
in formula (I) the "bold bonds" and "hashed bonds" refer to the relative stereochemistry of the chiral centers;
X is NH or O;
R₁ is selected from the group consisting of H, -(C₁-C₃)alkyl, -O(C₁-C₃)alkyl, halogen, -CF₃, and -OCF₃;
R₂ is phenyl; wherein R₂ is optionally substituted with one group selected from -(C₁-C₃)alkyl, -O-(C₁-C₃)alkyl, halogen, -CF₃, and -OCF₃, -(C₃-C₆)cycloalkyl, -NH-(C₁-C₃)alkyl, -NH-CO-(C₁-C₃)alkyl, and -O(C₃-C₆)cycloalkyl;
R₃ is selected from the group consisting of H, -(C₁-C₄)alkyl, -(C₃-C₆)cycloalkyl, -CO-(C₁-C₆)alkyl, and -CO-(C₃-C₆)cycloalkyl;
R₄ is selected from the group consisting of H, -(C₁-C₄)alkyl, -(C₃-C₆)cycloalkyl, -(CH₂)-(C₃-C₆)cycloalkyl, and -(CH₂CH₂)-(C₃-C₆)cycloalkyl;
and wherein R₄ is optionally substituted with halogen;
and R₅ is selected from the group consisting of H, -CH₃, and -COCH₃.

.. The compounds of the invention may be formulated in different types of compositions. Thus, a second aspect of the disclosure relates to a pharmaceutical composition which comprises a therapeutically effective amount of a compound of formula (I) as defined above, or a pharmaceutically acceptable salt thereof, or any enantiomer or mixtures of enantiomers, either of the compound of formula (I) or of its pharmaceutically acceptable salts, together with one or more pharmaceutically acceptable excipients or carriers.

.. Further, as mentioned above, the compounds of the invention or the pharmaceutical composition as previously defined may be used in the treatment and/or prevention of cancer, in particular in the treatment and/or prevention of cancer mediated by NPM1 dysregulation; more particularly haematological malignancies; more particularly anaplastic large cell lymphoma (ALCL), acute promyelocytic leukemia (APL) and acute myeloid leukemia (AML).

.. Accordingly, an additional aspect of the invention relates to a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use in therapy.

.. It is also provided a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use as a medicament.

.. The present invention further provides a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use in the treatment of a disease associated with NPM1 dysregulation.

.. It is further provided a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use in the treatment and/or prevention of cancer, in particular for use in the treatment and/or prevention of haematological malignancies, being particularly relevant in anaplastic large cell lymphoma (ALCL), acute promyelocytic leukemia (APL) and acute myeloid leukemia (AML).

.. In accordance with an embodiment of the present invention, it is provided a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use in the treatment and/or prevention of cancer, wherein the cancer is selected from the group consisting of mixed lineage leukemia (MLL), MLL-related leukemia, MLL-associated leukemia, MLL-positive leukemia, MLL-induced leukemia, rearranged mixed lineage leukemia (MLL-r), leukemia associated with a MLL rearrangement or a rearrangement of the MLL gene, acute leukemia, chronic leukemia, indolent leukemia, lymphoblastic leukemia, lymphocytic leukemia, myeloid leukemia, myelogenous leukemia, childhood leukemia, acute lymphocytic leukemia (ALL), anaplastic large cell lymphoma (ALCL), acute promyelocytic leukemia (APL), acute myeloid leukemia (AML), acute granulocytic leukemia, acute nonlymphocytic leukemia, chronic lymphocytic leukemia (CLL), chronic myelogenous leukemia (CML), therapy related leukemia, myelodysplastic syndrome (MDS), myeloproliferative disease (MPD), myeloproliferative neoplasia (MPN), plasma cell neoplasm, multiple myeloma, myelodysplasia, cutaneous T-cell lymphoma, lymphoid neoplasm, AIDS-related lymphoma, thymoma, thymic carcinoma, mycosis fungoides, Alibert-Bazin syndrome, granuloma fungoides, Sézary Syndrome, hairy cell leukemia, T-cell prolymphocytic leukemia (T-PLL), large granular lymphocytic leukemia, meningeal leukemia, leukemic leptomeningitis, leukemic meningitis, multiple myeloma, Hodgkin's lymphoma, non Hodgkin's lymphoma (malignant lymphoma), or Waldenstrom's macroglobulinemia.

.. Additionally, NPM1 is overexpressed in a variety of solid tumours of different histological origin including prostate, liver, thyroid, colon, gastric, pancreas, glioma and glioblastoma, astrocytoma and others. NPM1 alteration often correlates with mitotic index and metastatization and it has been proposed as an adverse prognostic marker in a number of such malignancies. Accordingly, the present invention provides a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, for use in the treatment and/or prevention of any of the above-mentioned malignancies.

.. The present invention also relates to a method for the treatment or prevention of a disease associated with NPM1 dysregulation, comprising administering a therapeutically pharmaceutically effective amount of a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, together with pharmaceutically acceptable diluents, excipients or carriers, in a subject in need thereof, including a human.

.. The present invention further provides a method for inhibiting NPM1, comprising administering to a patient in need of said treatment an amount of a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, sufficient to downregulate and/or reduce cytoplasmic location of this protein.

.. Accordingly, the invention further relates to use of a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, in the manufacture of a medicament for the treatment and/or prophylaxis of a disorder mediated by NPM1 dysregulation and associated clinical symptoms in a mammal, including a human; in particular a cancer, more particularly a haematological cancer, especially anaplastic large cell lymphoma (ALCL), acute promyelocytic leukemia (APL) acute myeloid leukemia (AML), or any of the malignancies mentioned above.

.. This aspect may alternatively be formulated as a method of treatment and/or prophylaxis of a mammal, including a human, suffering from or being susceptible to any of the diseases mentioned above which comprises administering to said patient a therapeutically effective amount of a compound of formula (I) as defined above, or a pharmaceutically acceptable salt thereof, or any enantiomer or mixtures of enantiomers, either of the compound of formula (I) or of its pharmaceutically acceptable salts, or the pharmaceutical composition as defined above, in combination with appropriate amounts of pharmaceutically acceptable diluents or carriers.

.. Compounds of the invention, including salts thereof, can be prepared using a number of synthetic routes, including the general synthetic routes described below, starting from commercially available starting materials, compounds known in the literature, or from readily prepared intermediates, by employing standard synthetic methods and procedures. Standard synthetic methods and procedures for the preparation of organic compounds and functional group transformations and manipulations are known in the art and can be found in standard textbooks.

.. A further aspect of the invention relates to a process for the preparation of the compounds of formula (I) or a pharmaceutically acceptable salt thereof, or any enantiomer or mixtures of enantiomers, either of the compound of formula (I) or of any of its pharmaceutically acceptable salts as defined herein. The process may be performed either according to method A, method B or alternatively method C, method D.

.. Methods A and B for the preparation of compounds of formula (I) wherein X is NH are illustrated in Scheme 1.

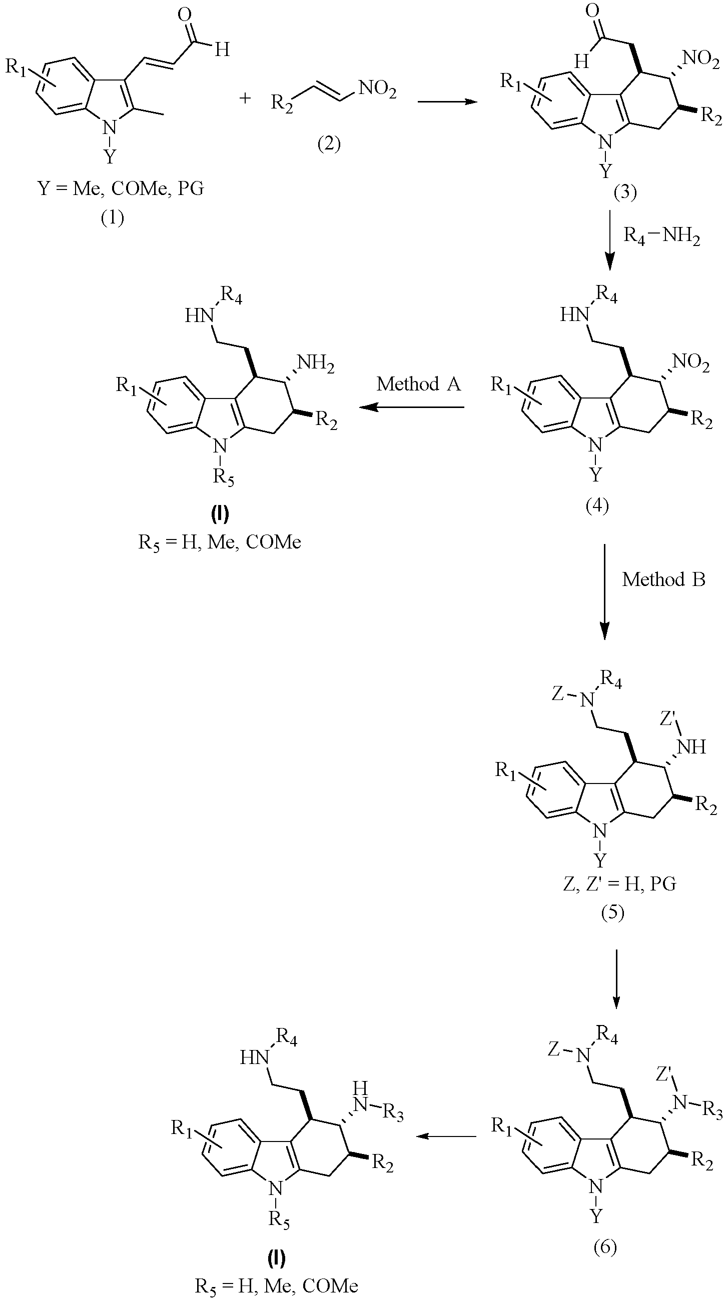

.. **Scheme 1.** General synthetic route for the preparation of compounds of formula (I) wherein X is NH, R₃ is H, R₅ is H, CH₃ or COCH₃, and R₁, R₂ and R₄ are as defined above (Method A), or wherein X is NH, R₃ is different from H, R₅ is H, CH₃ or COCH₃, and R₁, R₂, and R₄ are as defined above (Method B).

.. According to method A, an asymmetric Diels-Alder reaction between 3-(2-methyl-1*H*-indol-3-yl)prop-2-enal derivative (1), wherein R₁ is as defined above and Y is methyl, acetyl or a Protecting-group (PG) such as *tert*-butoxycarbonyl (Boc), and nitroalkene (2), wherein R₂ is as defined above, catalyzed by (*R*)-2-{diphenyl[(trimethylsilyl)oxy]methyl}pyrrolidine, provides the corresponding tetrahydrocarbazole scaffold (3) in a diastereo- and enantioselective manner. Alternatively, when the S enantiomer of the catalyst is used, the enantiomerically opposite Diels-Alder product (3) is obtained. Next, reductive amination of (3) with amine of formula R₄NH₂, wherein R₄ is as defined above, and subsequent reduction of the nitro group of intermediate (4), optionally followed by removal of the Boc group, result in final compounds of formula (**I**) wherein X is NH, R₃ is H, and R₁, R₂, R₄, R₅ are as defined above.

.. Alternatively, according to method B, reduction of the nitro group of derivative (4) previously described in method A, preceded or followed by protection, subsequent alkylation or acylation of intermediate (5), and deprotection of derivative (6) provide final compounds of formula (I) wherein X is NH and R₃ is different from H, and R₁, R₂, R₄, R₅ are as defined above.

.. Methods C and D for the preparation of the compounds of formula (I) wherein X is O are outlined in Scheme 2.

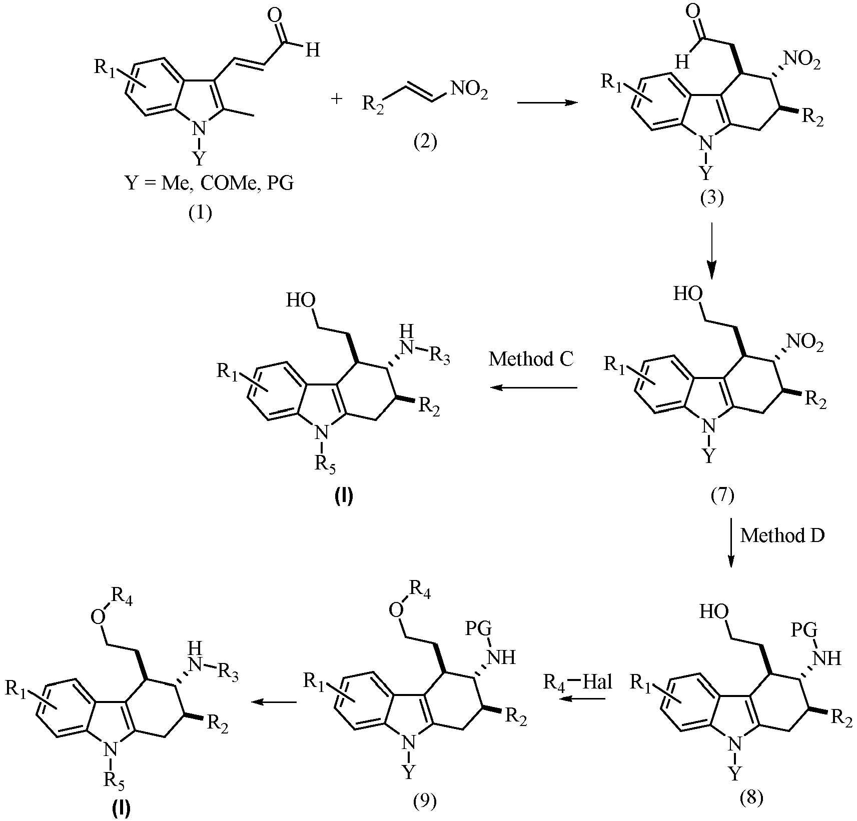

.. **Scheme 2.** General synthetic route for the preparation of compounds of formula (I) wherein X is O, R₄ is H, and R₁, R₂, R₃, R₅ are as defined above (Method C), or wherein X is O, and R₄ is different from H, and R₁, R₂, R₃, R₅ are as defined above (Method D).

.. The method C comprises an initial asymmetric Diels-Alder reaction, analogous to that described in method A, to obtain the corresponding tetrahydrocarbazole scaffold (3), wherein R₁ and R₂ are as defined above, and Y is methyl, acetyl or a Protecting-group (PG) such as *tert*-butoxycarbonyl (Boc). This derivative is transformed into alcohol (7), followed by reduction of the nitro group, optional *N*-alkylation or *N*-acylation, and removal of the Boc group when necessary, to afford final compounds of formula (**I**) wherein X is O, R₄ is H, and R₁, R₂, R₃, R₅ are as defined above.

.. Alternatively, according to method D, reduction of the nitro group and in-situ N-protection in alcohol (7) provide intermediate (8). Next, O-alkylation with a halogenated derivative of formula R₄-halogen, deprotection and optional N-alkylation or N-acylation afford final compounds of formula (**I**) wherein X is O, R₄ is different from H, and R₁, R₂, R₃, R₅ are as defined above.

.. The preparation processes described above include the use of stereospecific reagents in order to give specific stereoisomers that can be isolated as enantiopure compounds or highly enriched mixtures of stereoisomers. In addition, it is possible to separate stereoisomers once the compound has been prepared by standard resolution techniques known to the skilled person.

### Brief description of the drawings

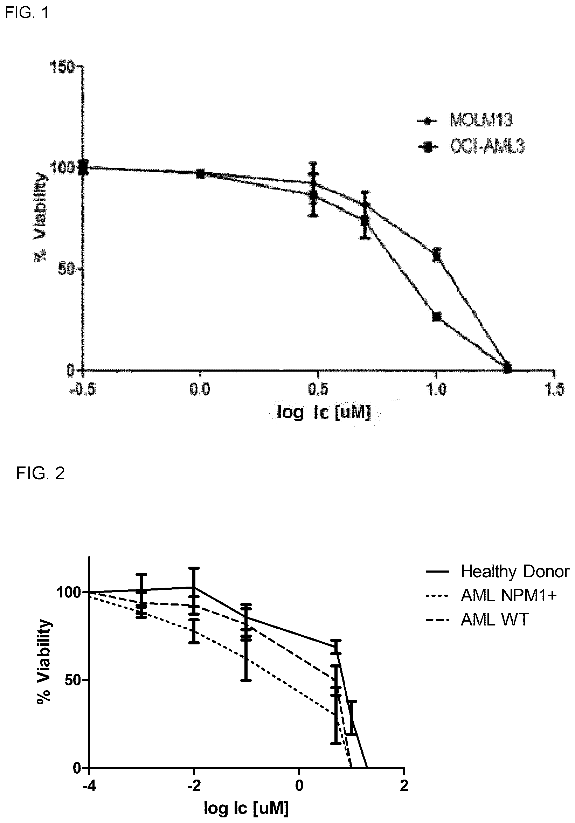

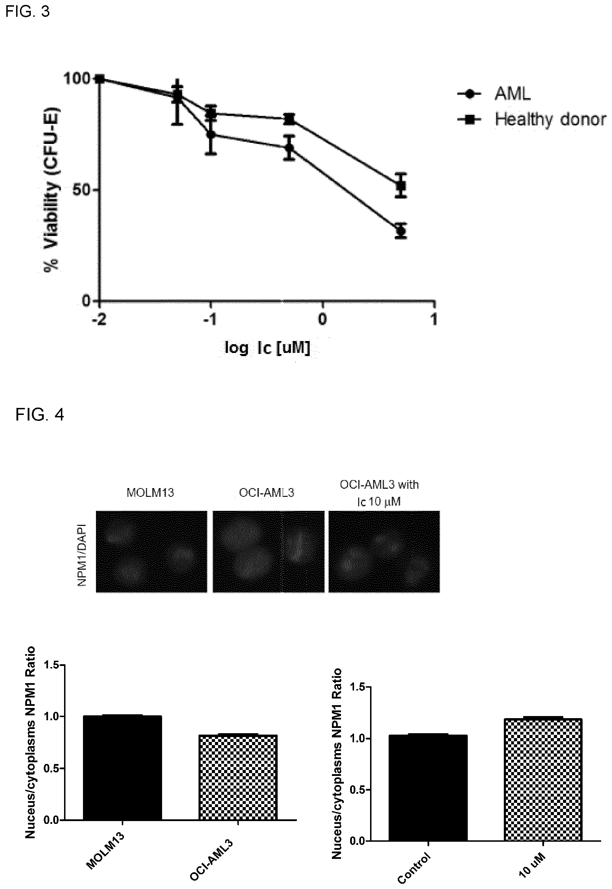

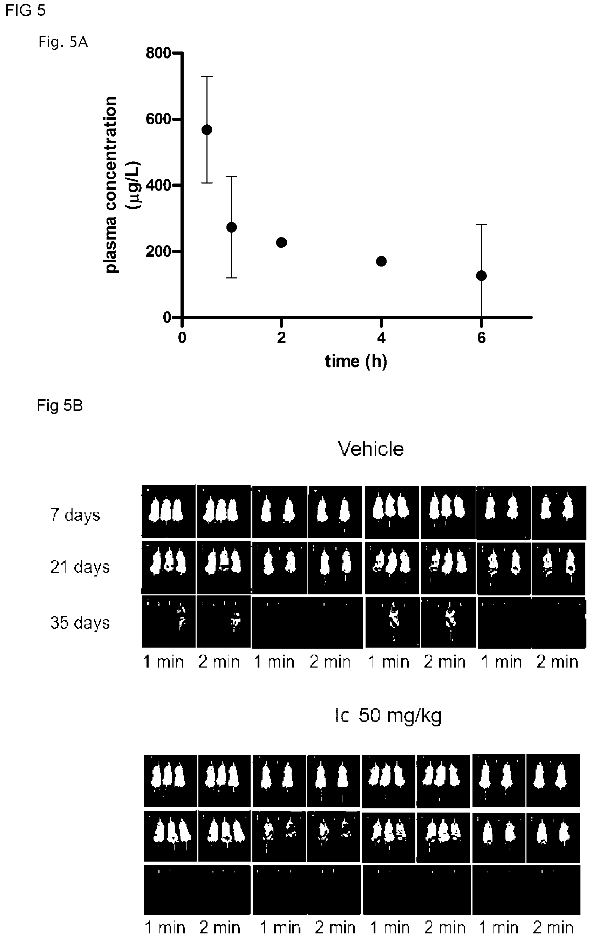

.. Non-limiting examples of the present disclosure will be described in the following, with reference to the appended drawings, in which:
.. FIG. 1 shows dose-response curve in AML cell lines OCI-AML3 (NPM1 mut) and MOLM13 (NPM1 WT) with compound **Ic**. The results showed µM range of the compound and higher efficacy against NPM1 mutant cell line. IC₅₀ MOLM13=41.75 µM. IC₅₀ OCI-AML3=7.589 µM.
.. FIG. 2 shows dose-response curve in AML primary cells NPM1 mut (N=4), NPM1 WT (N=4) and healthy donor bone marrow cells (N=5) with compound **Ic.** The results showed µM range of the compound, higher efficacy against AML cells and especially on NPM1 mutant AML primary cells.
.. FIG. 3 shows dose-response curve in BFU-E cultures with AML primary cells (N=3) and healthy donor bone marrow cells (N=3) with compound Ic. The results showed µM range of the compound, higher efficacy against AML cells.
.. FIG. 4 shows confocal microscopy images of NPM1 and DAPI in AML cell lines OCI-AML3 (NPM1 mut) and MOLM13 (NPM1 WT) and in OCI-AML3 with compound Ic 10 µM. The results showed NPM1C+ in OCI-AML3 but not in MOLM13, and how compound Ic 10 µM deplete NPM1 in OCI-AML3, especially NPM1C+ form.
.. FIG. 5A shows plasma concentration of compound Ic at 30 minutes, 1, 2, 4 and 6 hours after ip administration of 50 mg/kg of the compound (N=3 per timepoint). A correct *in-vivo* diffusion was observed for compound Ic.
.. FIG. 5B shows AML cell infiltration after Ic ip administration for 3 weeks in NSG mice injected with 1 million OCI-AML3-GFP-Luc cells. Tumour infiltration decrease was observed in **Ic**-treated group compared with vehicle at same time of sacrifice.

### Detailed description of the invention

.. All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

.. The terms "compound of the invention", "compound as described herein" and the like are meant to include a compound of formula (I), including their salts, solvates, all enantiomers, and isotopes thereof.

.. The present invention also includes salts of the compounds of the invention. Preferably, said salts are pharmaceutically acceptable salts.

.. As used herein, a "pharmaceutically acceptable salt" is intended to mean a salt that retains the biological effectiveness and properties of the parent compound (i.e. the free acid or free base, as applicable) and that is not biologically or otherwise undesirable. Pharmaceutically acceptable salts include salts formed with inorganic or organic bases, and salts formed with inorganic and organic acids.

.. There is no limitation on the type of salt of the compounds of the invention that can be used, provided that these are pharmaceutically acceptable when they are used for therapeutic purposes.

.. As some of the compounds of formula (I) are basic compounds, salts may be prepared from pharmaceutically acceptable non-toxic acids, including inorganic and organic acids. Such acids include for instance acetic, trifluoroacetic, benzenesulfonic, benzoic, camphorsulfonic, citric, ethanesulfonic, fumaric, gluconic, glutamic, hydrobromic, hydrochloric, lactic, maleic, malic, mandelic, methanesulfonic, phosphoric, succinic, sulfuric, tartaric, p-toluenesulfonic acid, and the like.

.. The preparation of pharmaceutically acceptable salts of the compounds of formula (I) can be carried out by methods known in the art. For instance, they can be prepared from the parent compound, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them. The compounds of formula (I) and their salts may differ in some physical properties but they are equivalent for the purposes of the present invention.

.. Additionally, compounds of the present invention, or salts thereof, may exist in hydrated or unhydrated (anhydrous) form or as solvates with other solvent molecules. The term "solvate" refers to a molecular complex comprising the compound of formula (I) or a salt thereof, and a stoichiometric or non-stoichiometric amount of one or more solvent molecules bound by non-covalent intermolecular forces. Some compounds may have a tendency to trap a fixed molar ratio of solvent molecules in the crystalline solid state, thus forming a solvate. If the solvent is water, the solvate formed is a hydrate. Non-limiting examples of solvates include hydrates and solvates with alcohols (also named alcoholates) such as ethanol (ethanolates). When compounds of the invention (or salts thereof) exist as solvates, all solvates thereof are intended to be included in the scope of the present invention, particularly pharmaceutically acceptable solvates. As used herein a "pharmaceutically acceptable solvate" is a solvate formed with a pharmaceutically acceptable solvent. Pharmaceutically acceptable solvents are well known in the art and include solvents such as water and ethanol. The compounds of the invention may be in crystalline form either as free solvation compounds or as solvates (e.g. hydrates) and it is intended that both forms are within the scope of the present invention. Methods of solvation are generally known within the art.

.. The reaction schemes described above are only meant as illustrative of methods to obtain the compounds of the invention. Other routes known by the ordinary skilled artisan, as well as other reactants and intermediates, can also be used to arrive at the compounds of formula (I).

.. The salts of a compound of formula (I) can be obtained during the final isolation and purification of the compounds of the invention or can be prepared by treating a compound of formula (I) with a sufficient amount of the desired acid or base to give the salt in a conventional manner.

.. In some of the processes described below it may be necessary or advisable to protect reactive or labile groups with conventional protecting groups. Both the nature of these protecting groups and the procedures for their introduction and removal are well known in the art. Whenever a protecting group is present, a subsequent deprotection step will be required, which can be performed under standard conditions well known in the art.

.. "Protecting group" (PG) refers to a group of atoms that when attached to a reactive group in a molecule masks, reduces or prevents that reactivity. Representative amino protecting groups include, formyl, acetyl, trifluoroacetyl, benzyl, benzyloxycarbonyl (CBZ), *tert*-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitro- veratryloxycarbonyl (NVOC), and the like. Representative hydroxy protecting groups include those where the hydroxy group is either acylated or alkylated such as benzyl, and trityl ethers as well as alkyl ethers, tetrahydropyranyl ethers, trialkylsilyl ethers and allyl ethers. The protecting group may be removed at a suitable later stage in the synthesis.

.. The term "halo" or "halogen" refers to bromo, chloro, fluoro or iodo.

.. As used herein, the term "hydroxyl" or "hydroxy" refers to -OH.

.. Combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., therapeutic administration to a subject).

.. The expression "substituted with one or more" means that a group can be substituted with one or more, preferably with 1, 2, 3 or 4 substituents, provided that this group has enough positions susceptible of being substituted.

.. Some compounds of the invention can have chiral centers that can give rise to various stereoisomers. As used herein, the term "stereoisomer" refers to all isomers of individual compounds that differ only in the orientation of their atoms in space. The term stereoisomer includes mirror image isomers (enantiomers), mixtures of mirror image isomers (racemates, racemic mixtures), geometric (cis/trans or syn/anti or E/Z) isomers, and isomers of compounds with more than one chiral center that are not mirror images of one another (diastereoisomers). The present invention relates to each of these stereoisomers and also mixtures thereof.

.. The compounds of the invention may have one or more asymmetric centers and may thus give rise to stereoisomers. All single optical isomers and stereoisomers, such as enantiomers, diastereoisomers and mixtures thereof, are considered within the scope of the present invention. Compounds of the present invention that contain asymmetrically substituted carbon atoms can be isolated in optically active form or highly enriched mixtures. Thus, any given compound referred to herein is intended to represent any one of a racemate, one or more enantiomeric forms, one or more atropoisomeric forms, and mixtures thereof.

.. Compounds of the invention include unlabeled forms as well as isotopically labeled forms thereof. Isotopically labeled forms of the compounds are compounds that differ only in the replacement of one or more atoms by a corresponding isotopically enriched atom. Examples of isotopes that can be incorporated into compounds of the invention include for example isotopes of hydrogen, carbon, nitrogen, oxygen, fluorine, chlorine, and iodine, such as ²H, ³H, ¹¹C, ¹³C, ¹⁴C, ¹⁵N, ¹⁸O, ¹⁷O, ³⁵S, ¹⁸F, ³⁶Cl, and ¹²⁵I. Such isotopically labelled compounds are useful for example as probes in biological assays, as analytical tools, or as therapeutic agents.

.. Diastereoisomers and enantiomers can be separated by conventional techniques such as chromatography or fractional crystallization. Optical isomers can be resolved by conventional techniques of optical resolution to give optically pure isomers. This resolution can be carried out on any chiral synthetic intermediates or on compounds of the invention. Optically pure isomers can also be individually obtained using enantiospecific synthesis.

.. In all embodiments of the invention referring to the compounds of formula (I), the pharmaceutically acceptable salts thereof and the enantiomers or mixtures of enantiomers, either of any of the compounds of formula (I) or of any of their pharmaceutically acceptable salts are always contemplated even if they are not specifically mentioned.

.. The term (C₁-Cₙ)alkyl refers to a saturated branched or linear hydrocarbon chain which contains from 1 to n carbon atoms and only single bonds.

.. The term "cycloalkyl" or "cycloalkane," as used herein, refers to an unsubstituted or substituted, saturated, stable, non-aromatic, chemically-feasible carbocyclic ring, having from three to six carbon atoms. Examples of cycloalkyl radicals include cyclopropyl, cyclobutyl, cyclopentyl, and cyclohexyl.

.. For the purposes of the invention, room temperature (rt) is 20-25 °C.

.. In the embodiments of the invention referring to the compounds of formula (I), where the substitution or unsubstitution of a certain group is not specified, e.g. either because it is not indicated a certain substitution for that group or that the group is unsubstituted, it has to be understood that the possible substitution of this group is the one as in the definition of the formula (I). The same applies when in specific group is said to be "optionally substituted" without specifically indicating the substitution.

.. The term "excipient" means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of giving a shape, to facilitate administration and preserve the active ingredient. Furthermore, they contribute to characterize the pharmaceutical preparation from the point of view of appearance, stability, biopharmaceutical profile and acceptability by the patient.

.. Unless otherwise indicated, in the context of the present invention the percentage and amount of a certain component in a composition are to be referred to the weight of said component with respect to the total weight of the composition.

.. Unless otherwise indicated, in the context of the present invention a range of values indicated for a certain parameter, for example the weight of a component in a mixture, includes the upper and the lower limits of the range, e.g. if the content in weight, or in volume, of a component A in a mixture is indicated as "X to Y", the content of A can be X, Y or any of the intermediate values.

.. Unless otherwise indicated, the data relative to the peaks in the NMR, IR and MS spectra are meant within the common uncertainty due to the instrument measurement.

.. With regard to the specific conditions for carrying out the preparation process of the invention, the skilled person would know how to adjust the parameters of each of the steps indicated above in the light of the description and examples of the present invention.

.. In accordance with an embodiment, optionally in combination with one or more features of the particular embodiments defined herein,
R₂ is phenyl;
wherein R₂ is optionally substituted with methyl, ethyl, -OCH₃, -OCH₂CH₃, -OCH(CH₃)₂, F, Cl, Br, -CF₃, -OCF₃, -(C₃-C₆)cycloalkyl, -NHCH₃, -NHCOCH₃ , and -O(C₃-C₆)cycloalkyl;
R₄ is selected from the group consisting of H, methyl, ethyl, propyl, isopropyl, isobutyl, cyclopropyl, cyclobutyl, cyclopentyl, cyclohexyl, and 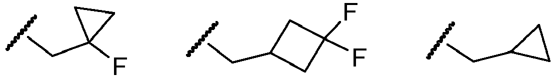 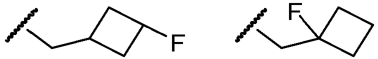
wherein the wavy line represents the attaching point.

.. In accordance with another embodiment, optionally in combination with one or more features of the particular embodiments defined herein,
R₁ is selected from the group consisting of H, methyl, ethyl, -OCH₃, -OCH₂CH₃; F, Cl, Br, -CF₃, and -OCF₃;
R₂ is phenyl optionally substituted with methyl, ethyl, -OCH₃, -OCH₂CH₃, -OCH(CH₃)₂, F, Cl, Br, -CF₃, -OCF₃, -NHCH₃, and -NHCOCH₃;
R₃ is selected from the group consisting of H, methyl, ethyl, -COCH₃, and cyclopropyl;
R₄ is selected from the group consisting of H, methyl, ethyl, propyl, isopropyl, isobutyl, cyclopropyl, cyclobutyl, and 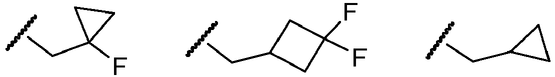 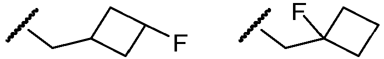
wherein the wavy line represents the attaching point.

.. In accordance with another embodiment, optionally in combination with one or more features of the particular embodiments defined herein,
R₁ is selected from the group consisting of H, methyl, ethyl, -OCH₃, -OCH₂CH₃, F, Cl, Br, -CF₃, and -OCF₃;
R₂ is phenyl optionally substituted with methyl, ethyl, -OCH₃, -OCH₂CH₃, F, Cl, -CF₃, and -OCF₃ ;
R₃ is selected from the group consisting of H and methyl;
R₄ is selected from the group consisting of H, methyl, ethyl, propyl, isopropyl, isobutyl, cyclopropyl, cyclobutyl, and 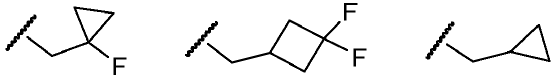 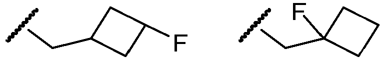
wherein the wavy line represents the attaching point.

.. In accordance with another embodiment, optionally in combination with one or more features of the particular embodiments defined herein,
X is NH;
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃ ;
R₃ is selected from the group consisting of H and methyl;
R₄ is selected from the group consisting of H, methyl, ethyl, propyl, isopropyl, isobutyl, cyclopropyl, cyclobutyl, and 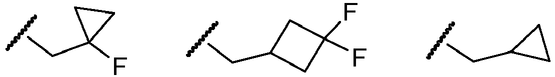 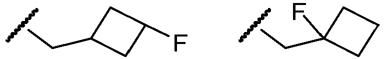
wherein the wavy line represents the attaching point;
and R₅ is selected from the group consisting of H, CH₃ and -COCH₃.

.. In accordance with another embodiment, optionally in combination with one or more features of the particular embodiments defined herein,
X is NH.
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃ ;
R₃ is selected from the group consisting of H and methyl;
R₄ is selected from the group consisting of 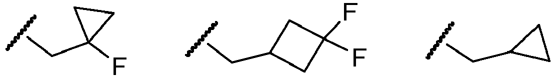 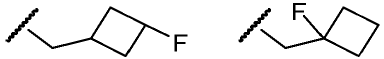
wherein the wavy line represents the attaching point;
and R₅ is selected from the group consisting of H, CH₃ and -COCH₃.

.. In accordance with another embodiment, optionally in combination with one or more features of the particular embodiments defined herein,
X is O,
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃ ;
R₃ is selected from the group consisting of H and methyl;
R₄ is selected from the group consisting of H, methyl, ethyl, propyl, isopropyl, isobutyl, cyclopropyl, cyclobutyl, and 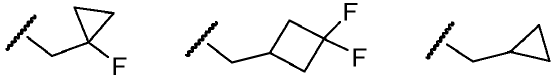 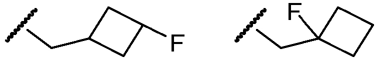
wherein the wavy line represents the attaching point;
and R₅ is selected from the group consisting of H, CH₃ and -COCH₃.

.. In accordance with another embodiment, optionally in combination with one or more features of the particular embodiments defined herein,
X is O;
R₁ is selected from the group consisting of H, F, Cl, methyl, and -OCH₃;
R₂ is phenyl optionally substituted with methyl, ethyl, -OCH₃, -OCH₂CH₃, F, Cl, -CF₃, and -OCF₃;
R₃ is selected from the group consisting of H and methyl;
R₄ is selected from the group consisting of 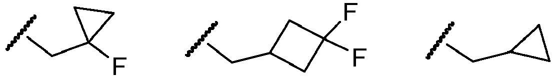 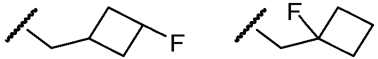
wherein the wavy line represents the attaching point;
and R₅ is selected from the group consisting of H, CH₃ and -COCH₃.

.. The following compounds are particularly preferred:
(2*R*,3*R*,4*R*)-4-{2-[(Cyclopropylmethyl)amino]ethyl}-2-phenyl-2,3,4,9-tetrahydro-1H-carbazol-3-amine (**Ia**);
(2*R*,3*R*,4*R*)-4-{2-[(Cyclopropylmethyl)amino]ethyl}-2-(4-methylphenyl)-2,3,4,9-tetrahydro-1*H*-carbazol-3-amine (**Ib**);
(2*R*,3*R*,4*R*)-4-{2-[(Cyclopropylmethyl)amino]ethyl}-2-(4-methoxyphenyl)-2,3,4,9-tetrahydro-1*H*-carbazol-3-amine (**Ic**);
(2*S*,3*S*,4*S*)-4-{2-[(Cyclopropylmethyl)amino]ethyl}-2-(4-methoxyphenyl)-2,3,4,9-tetrahydro-1*H*-carbazol-3-amine (**Id**);
(2*R*,3*R*,4*R*)-4-{2-[(Cyclopropylmethyl)amino]ethyl}-2-[4-(trifluoromethyl)phenyl]-2,3,4,9-tetrahydro-1*H*-carbazol-3-amine (**Ie**);
(2*R*,3*R*,4*R*)-4-{2-[(Cyclopropylmethyl)amino]ethyl}-2-[4-(trifluoromethoxy)phenyl]-2,3,4,9-tetrahydro-1*H*-carbazol-3-amine (**If**);
(2*R*,3*S*,4*R*)-4-{2-[(Cyclopropylmethyl)amino]ethyl}-2-(2-fluorophenyl)-2,3,4,9-tetrahydro-1*H*-carbazol-3-amine (**Ig**);
(2*R*,3*R*,4*R*)-2-(3-Chlorophenyl)-4-{2-[(cyclopropylmethyl) amino]ethyl}-2,3,4,9-tetrahydro-1*H*-carbazol-3-amine (**Ih**);
(2*R*,3*R*,4*R*)-2-(4-Chlorophenyl)-4-{2-[(cyclopropylmethyl)amino]ethyl}-2,3,4,9-tetrahydro-1*H*-carbazol-3-amine (**Ii**);
(2*R*,3*R*,4*R*)-4-{2-[(Cyclopropylmethyl)amino]ethyl}-2-(3-methoxyphenyl)-2,3,4,9-tetrahydro-1*H*-carbazol-3-amine (**Ij**);
(2*R*,3*S*,4*R*)-4-{2-[(Cyclopropylmethyl)amino]ethyl}-2-(2-methoxyphenyl)-2,3,4,9-tetrahydro-1*H*-carbazol-3-amine (**Ik**);
(2*R*,3*R*,4*R*)-4-{2-[(Cyclopropylmethyl)amino]ethyl}-2-(3-methylphenyl)-2,3,4,9-tetrahydro-1*H*-carbazol-3-amine (**Il**);
(2*R*,3*R*,4*R*)-4-{2-[(Cyclopropylmethyl)amino]ethyl}-2-(2-methylphenyl)-2,3,4,9-tetrahydro-1*H*-carbazol-3-amine (**Im**);
(2*R*,3*S*,4*R*)-2-(2-Chlorophenyl)-4-{2-[(cyclopropylmethyl)amino]ethyl}-2,3,4,9-tetrahydro-1*H*-carbazol-3-amine (**In**);
(2*R*,3*R*,4*R*)-4-{2-[(Cyclopropylmethyl)amino]ethyl}-2-(4-fluorophenyl)-2,3,4,9-tetrahydro-1*H*-carbazol-3-amine (**Io**);
(2*S*,3*S*,4*S*)-4-{2-[(Cyclopropylmethyl)amino]ethyl}-2-(4-fluorophenyl)-2,3,4,9-tetrahydro-1*H*-carbazol-3-amine (**Ip**);
(2*R*,3*R*,4*R*)-4-{2-[(Cyclopropylmethyl)amino]ethyl}-2-(3-fluorophenyl)-2,3,4,9-tetrahydro-1*H*-carbazol-3-amine (**Iq**);
(2*R*,3*R*,4*R*)-4-{2-[(Cyclopropylmethyl)amino]ethyl}-2-(4-methoxyphenyl)-N-methyl-2,3,4,9-tetrahydro-1*H*-carbazol-3-amine (**Ir**);
(2*S*,3*S*,4*S*)-4-{2-[(Cyclopropylmethyl)amino]ethyl}-2-(4-methoxyphenyl)-N-methyl-2,3,4,9-tetrahydro-1*H*-carbazol-3-amine (**Is**);
*N*-[(2*R*,3*R*,4*R*)-4-{2-[(Cyclopropylmethyl)amino]ethyl}-2-(4-methoxyphenyl)-2,3,4,9-tetrahydro-1*H*-carbazol-3-yl]acetamide (**It**);
(2*R*,3*R*,4*R*)-4-(2-{[(3,3-Difluorocyclobutyl)methyl]amino}ethyl)-2-(4-methylphenyl)-2,3,4,9-tetrahydro-1*H*-carbazol-3-amine (**Iu**);
(2*R*,3*R*,4*R*)-9-Methyl-2-(4-methylphenyl)-4-(2-{[(oxetan-3-yl)methyl]amino}ethyl)-2,3,4,9-tetrahydro-1*H*-carbazol-3-amine (**Iv**);
(2*R*,3*R*,4*R*)-2-(4-Methoxyphenyl)-4-[2-(methylamino)ethyl]-2,3,4,9-tetrahydro-1*H-*carbazol-3-amine (**Iw**);
(*2R,3R,4R*)-2-(4-Methylphenyl)-4-{2-[(2-methylpropyl)amino]ethyl}-2,3,4,9-tetrahydro-1*H*-carbazol-3-amine (**Ix**);
(2*R*,3*R*,4*R*)-2-(4-Methylphenyl)-4-{2-[(propan-2-yl)amino]ethyl}-2,3,4,9-tetrahydro-1*H-*carbazol-3-amine (**Iy**);
(2*R*,3*R*,4*R*)-4-[2-(Ethylamino)ethyl]-2-(4-methylphenyl)-2,3,4,9-tetrahydro-1*H-*carbazol-3-amine (**Iz**);
(2*R*,3*R*,4*R*)-4-[2-(Cyclopropylamino)ethyl]-2-(4-methylphenyl)-2,3,4,9-tetrahydro-1*H-*carbazol-3-amine (**Iaa**);
(2*R*,3*R*,4*R*)-6-Chloro-4-(2-{[(3,3-difluorocyclobutyl)methyl]amino}ethyl)-2-(4-methylphenyl)-2,3,4,9-tetrahydro-1*H*-carbazol-3-amine (**Iab**);
(2*R*,3*R*,4*R*)-6-Chloro-4-(2-{[(3,3-difluorocyclobutyl)methyl]amino}ethyl)-*N*-methyl-2-(4-methylphenyl)-2,3,4,9-tetrahydro-1*H*-carbazol-3-amine (**Iac**);
2-[(2*R*,3*R*,4*R*)-3-Amino-2-(4-methylphenyl)-2,3,4,9-tetrahydro-1*H*-carbazol-4-yl]ethan-1-ol (**Iad**);
(2*R*,3*R*,4*R*)-4-(2-Methoxyethyl)-2-(4-methoxyphenyl)-2,3,4,9-tetrahydro-1*H*-carbazol-3-amine (**Iae**);
(2*R*,3*R*,4*R*)-4-{2-[(Cyclopropylmethyl)amino]ethyl}-6-methoxy-2-(4-methylphenyl)-2,3,4,9-tetrahydro-1*H*-carbazol-3-amine (**Iaf**);
(2*R*,3*R*,4*R*)-6-Chloro-4-{2-[(cyclopropylmethyl)amino]ethyl}-2-(4-methylphenyl)-2,3,4,9-tetrahydro-1*H*-carbazol-3-amine (**Iag**);
(2*R*,3*R*,4*R*)-6-Chloro-4-{2-[(cyclopropylmethyl)amino]ethyl}-2-(4-methoxyphenyl)-2,3,4,9-tetrahydro-1*H*-carbazol-3-amine (**Iah**);
(2*R*,3*R*,4*R*)-4-{2-[(Cyclopropylmethyl)amino]ethyl}-6-methyl-2-(4-methylphenyl)-2,3,4,9-tetrahydro-1*H*-carbazol-3-amine (**Iai**);
(2*R*,3*R*,4*R*)-4-{2-[(Cyclopropylmethyl)amino]ethyl}-6-fluoro-2-(4-methylphenyl)-2,3,4,9-tetrahydro-1*H*-carbazol-3-amine (**Iaj**);
(2*R*,3*R*,4*R*)-6-Chloro-4-{2-[(cyclopropylmethyl)amino]ethyl}-N-methyl-2-(4-methylphenyl)-2,3,4,9-tetrahydro-1*H*-carbazol-3-amine (**Iak**); and
(*2R,3R,4R*)-4-{2-[(Cyclopropylmethyl)amino]ethyl}-9-methyl-2-(4-methylphenyl)-2,3,4,9-tetrahydro-1*H*-carbazol-3-amine (**Ial**).

.. For the purposes of the invention, the term "treatment" of the disease refers to stopping or delaying of the disease progress, when the drug is used in the subject exhibiting symptoms of disease onset. The term "prevention" refers to stopping or delaying of symptoms of disease onset, when the drug is used in the subject exhibiting no symptoms of disease onset but having high risk of disease onset.

.. Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention.

.. As it is shown in the Examples, compounds of the invention potently inhibit NPM1 dysregulation. Taken together, the results show that compounds of the invention are potent and specific inhibitors of NPM1 in human cells, especially against NPM1 mutant cell lines.

.. It is highlighted that in the field of drug design, any structural modification of a pharmacologically active compound was, in the absence of an established correlation between structural features and activity, expected a priori to disturb the pharmacological activity profile of the initial structure.

.. The compounds of the invention are structurally different from the NPM1 regulators described in the prior art because of the novel combination of substituents of the formula, and particularly in the specific selection of the tetrahydrocarbazole moiety present in their structure. These structural variations are neither disclosed nor suggested in the prior art. These structural variations result in compounds that are useful as inhibitors of the NPM1 protein, with a remarkable inhibition of the NPM1 dysregulation in human cell lines.

.. The expression "therapeutically effective amount" as used herein refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations. Accordingly, a therapeutically effective amount of a compound may be an amount which is sufficient to treat a disease or disorder, delay the onset or progression of a disease or disorder, and/or alleviate one or more symptoms of the disease or disorder, when administered to a subject suffering from said disease or disorder. The precise effective amount for a subject will depend upon a variety of factors such as the subject's body weight, size and health, the nature and extent of the condition to be treated, and the therapeutic or combination of therapeutics selected for administration. Therapeutically effective amounts for a given situation can be determined by routine experimentation that is within the skill and judgement of the clinician.

.. For any compound, the therapeutically effective amount can be estimated initially either in in vitro assays, e.g. cell culture assays, or in animal models, e.g. mice, rats or dogs. The animal model may also be used to determine the appropriate concentration range and route of administration. Such information can then be used to determine useful doses and routes for administration in humans. Therapeutic efficacy and toxicity may be determined by standard procedures in cell cultures or experimental animals, e.g. ED₅₀ and LD₅₀ values can be determined and the ratio between toxic and therapeutic effects, also known as therapeutic index, may be calculated and used to determine suitable doses for use in humans.

.. As used herein, unless otherwise stated, the term "treating" and "treatment" in relation to a disease, disorder or condition refers to the management and care of a patient for the purpose of combating a disease, disorder or condition, such as to reverse, alleviate, inhibit the process of, or prevent the disease, disorder or condition to which such term applies, or one or more symptoms of such disease, disorder or condition, and includes the administration of a compound of the invention (or a pharmaceutically acceptable salt thereof) to prevent the onset of the symptoms or the complications, or alleviating the symptoms or complications, or eliminating the disease, condition or disorder. Preferably, treatment is curative or ameliorating.

.. While it is possible that a compound of the invention may be administered for use in therapy directly as such, it is typically administered in the form of a pharmaceutical composition. These compositions comprise a compound of the invention (or a pharmaceutically acceptable salt thereof) as active pharmaceutical ingredient together with one or more pharmaceutically acceptable carriers. For the purposes of the invention, a carrier is suitable for use in the pharmaceutical compositions described herein if it is compatible with the other ingredients of the composition and not deleterious to the recipient of the composition. The expression "pharmaceutically acceptable diluents, excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. A "pharmaceutically acceptable carrier" includes non-API (API refers to Active Pharmaceutical Ingredient) substances, such as disintegrators, binders, fillers, lubricants and the like, used in formulating pharmaceutical products and regarded as safe for administering to subjects (particularly humans) according to established governmental standards, including those promulgated by the United States Food and Drug Administration and the European Medical Agency. Pharmaceutically acceptable carriers are well known to those skilled in the art and are selected on the basis of the chosen type of formulation and route of administration, according to standard pharmaceutical practice.

.. The election of the pharmaceutical formulation will depend upon the nature of the active compound and its route of administration. Any route of administration may be used, for example oral and parenteral administration.

.. Parenteral administration includes intravenous, intra-arterial, subcutaneous, intraperitoneal or intramuscular. Parenteral administration can be in the form of a single bolus dose, or may be, for example, by a continuous perfusion pump.

.. The compositions can be formulated as to provide quick (immediate), sustained or delayed release of the active ingredient after administration to the patient by using methods known in the art.

.. Examples of pharmaceutically acceptable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, and methyl cellulose. The pharmaceutical compositions can additionally include further pharmaceutically acceptable excipients including: lubricating agents such as talc, magnesium stearate and mineral oil; wetting agents; emulsifying and suspending agents; preserving agents such as methyl- and propylhydroxybenzoates; sweetening agents; flavouring agents; and colouring agents.

.. Pharmaceutical compositions suitable for parenteral administration include sterile aqueous solutions or suspensions, or can be alternatively prepared in lyophilized form for extemporaneous preparation of a solution or suspension using a sterile aqueous carrier prior to use. In such formulations, diluents or pharmaceutically acceptable carriers such as sterile water and physiological saline buffer can be used. Other conventional solvents, pH buffers, stabilizers, anti-bacterial agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

.. The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

.. In therapeutic applications, pharmaceutical compositions are to be administered in a manner appropriate to the disease to be treated, as determined by a person skilled in the medical arts. An appropriate dose and suitable duration and frequency of administration will be determined by such factors as the condition of the patient, the type and severity of the disease, the particular form of the active ingredient and the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objectively identifiable improvement as noted by the clinician. Effective doses may generally be assessed or extrapolated using experimental models like dose-response curves derived from in vitro or animal model test systems.

.. The pharmaceutical compositions of the invention can be included in a container, pack or dispenser together with instructions for administration.

.. The compounds of the invention can also be administered in combination with another active agent as long as the other active agent does not interfere with or adversely affect the effects of the active compounds of this invention. Combination therapy includes administration of a single pharmaceutical dosage formulation which contains a compound of the invention and one or more additional active agents, as well as administration of the compound of the invention and each additional active agent in its own separate pharmaceutical dosage formulation. If administered separately, the administration can be simultaneous, sequential or separate, and the compound of the invention and the additional therapeutic agent(s) can be administered via the same administration route or using different administration routes, for example one compound can be administered orally and the other intravenously.

.. Although only a number of examples have been disclosed herein, other alternatives, modifications, uses and/or equivalents thereof are possible. Thus, the scope of the present disclosure should not be limited by particular examples, but should be determined only by a fair reading of the claims that follow.

### Examples

### EXAMPLE 1. General procedure for the synthesis of compounds of formula (I) wherein X is NH and R₃ is H (Method A, Scheme 1).

.. To a solution of (R)-2-{diphenyl[(trimethylsilyl)oxy]methyl} pyrrolidine (0.2 eq) in toluene (2 mL/mmol), benzoic acid (0.2 eq) was added and the mixture was stirred at rt for 10 min under air. Then, compound (1, Y = Me, COMe, Boc) (1.5 eq) and the corresponding nitroalkene (2) (1.0 eq) were added and the reaction was stirred at 70 °C for 48 h or under MW irradiation at 110 °C for 5.5 h. Afterward, the crude was flushed through a short plug of silica, using a 1:1 mixture of dichloromethane (DCM)/diethyl ether, and the solvent was evaporated under reduced pressure. The residue was purified by flash chromatography (toluene/diethyl ether, 98:2) to obtain the corresponding derivative (3).

.. To a solution of (3) (1.0 eq) in anhydrous methanol (5 mL/mmol) under nitrogen atmosphere, the appropriate amine (2.0 eq) was added, and the reaction mixture was stirred at rt for 4 h to form the corresponding imine (confirmed by¹H-NMR analysis). Then, NaBH₄ (2.0 eq) was added at 0 °C and the mixture was allowed to reach rt and stirred overnight. The reaction was quenched with a saturated aqueous solution of NaHCO₃, and the solvent was evaporated under reduced pressure. The residue was suspended in water and extracted with EtOAc (x2). The combined organic layers were washed with brine, dried over Na₂SO₄, filtered and concentrated under reduced pressure. The crude was purified by flash chromatography (DCM/methanol, 20:1 to 5:1) to provide derivative (4).

.. Next, (4) (1.0 eq) was dissolved in a 1:1 mixture of acetic acid and anhydrous methanol (4 mL/mmol) under nitrogen atmosphere, and zinc (10.0 eq) was added. The reaction was stirred until complete conversion of starting material (thin layer chromatography). Then, the mixture was filtered, washing with methanol, and the filtrate was evaporated under reduced pressure. The residue was suspended in a saturated aqueous solution of NaHCO₃ and extracted with DCM (x3). The combined organic layers were washed with brine, dried over Na₂SO₄, filtered and concentrated under reduced pressure. The crude was purified by flash chromatography (DCM/methanol, 20:1 to 5:1, with 0.5% NH₃) to afford the corresponding final compound of formula (I) wherein R₅ group is CH₃, COCH₃, or the Boc protected derivative of final compound of formula (I) wherein R₅ group is H. The Boc protected derivative (1.0 eq) was dissolved in methanol (2.5 mL/mmol), concentrated HCI (35.0 eq) was added and the reaction mixture was stirred at rt for 4 h. Then, the solvent was evaporated under reduced pressure and the resulting solid was washed with anhydrous diethyl ether and dried under vacuum to afford the hydrochloride salt of the corresponding final compound of formula (I) wherein R₅ group is H.

### EXAMPLE 2. General procedure for the synthesis of compounds of formula (I) wherein X is NH and R₃ is different from H (Method B, Scheme 1).

.. To a solution of derivative (4) (1.0 eq), synthesized according to the procedure described in Example 1, in anhydrous methanol (7 mL/mmol) at 0 °C under nitrogen atmosphere, nickel chloride hexahydrate (0.15 eq) was added, and the reaction was stirred at this temperature for 10 min. Then, NaBH₄ (12.0 eq) was added portionwise over 30 min and the reaction was stirred at 0 °C for 30 min. Next, di-tert-butyl dicarbonate (3.0 eq) was added and the reaction was allowed to reach rt and stirred overnight. The reaction was then quenched with a 2:1:1 mixture of brine, saturated aqueous solution of NaHCO₃ and EtOAc, and extracted with EtOAc. The organic phase was concentrated under reduced pressure and the residue was dissolved in CHCl₃ and filtered through celite. The solvent was evaporated under reduced pressure and the crude product was purified by flash chromatography (DCM/methanol, 20:1 to 5:1) to afford derivative (5, Z = Z' = Boc). Next, to a solution of (5) (1.0 eq) in anhydrous DMF (6.5 mL/mmol) under a nitrogen atmosphere, NaH (4.0 eq) was added at 0 °C, and the mixture was stirred at rt for 1 h. The appropriate halogenated derivative (10.0 eq) was then added and the reaction was stirred at rt for 18 h. The mixture was neutralized with a saturated aqueous solution of NH₄Cl, diluted with water, and extracted with EtOAc (x2). The combined organic layers were dried over Na₂SO₄, filtered, and concentrated under reduced pressure. The crude product was purified by flash chromatography (hexane/EtOAc, 20:1) to afford derivative (6, Z = Z' = Boc).

.. Alternatively, di-tert-butyl dicarbonate (2.0 eq) was added to a solution of (4) (1.0 eq) and Et₃N (1.3 eq) in anhydrous DCM (10 mL/mmol) at 0 °C under nitrogen atmosphere. The mixture was stirred at 0 °C for 30 min, and then 1 h at rt. The reaction was quenched with a saturated aqueous solution of NaHCO₃ and extracted with DCM (x2). The combined organic layers were dried over Na₂SO₄, filtered, and concentrated under reduced pressure. The crude product was purified by flash chromatography (hexane/EtOAc, 20:1 to 5:1). The resulting N-Boc intermediate (4) (1.0 eq) was dissolved in anhydrous methanol (12 mL/mmol) at 0 °C under a nitrogen atmosphere, nickel chloride hexahydrate (0.05 eq) was added, and the mixture was stirred at this temperature for 5 min. NaBH₄ (4.0 eq) was then added portionwise over 30 min and the reaction was stirred at 0 °C for 2 h. After this time, additional portions of NiCl₂ (0.05 eq) and NaBH₄ (2.5 eq) were added and the mixture was allowed to reach rt and stirred overnight. The reaction was then quenched with a saturated aqueous solution of NH₄CI solution, filtered through celite, and evaporated. The residue was dissolved in EtOAc and washed with water, a saturated aqueous solution of NaHCO₃, and brine. The organic layer was dried over Na₂SO₄, filtered, and evaporated under reduced pressure. The crude was purified by flash chromatography (hexane/EtOAc, 20:1 to 5:1) to afford derivative (5, Z = Boc, Z' = H). Next, the appropriate acid anhydride (1.2 eq) was added at 0 °C to a solution of (5) (1.0 eq) in a 3:1 mixture of DCM and pyridine (20 mL/mmol) under a nitrogen atmosphere. The mixture was stirred at 0 °C for 1 h, and then at rt for 12 h. After this time, the solvent was removed by azeotropic distillation with 1,2-dichloroethane (x3) under reduced pressure, and the residue was dissolved in DCM and washed with a saturated aqueous solution of NaHCO₃ solution. The organic layer was washed with brine, dried over MgSO₄, filtered and concentrated under vacuum. The crude product was purified by flash chromatography (DCM/methanol, 25:1) to afford derivative (6, Z = Boc, Z' = H).
Finally, the corresponding protected derivative (6) (1.0 eq) was dissolved in methanol (2.5 mL/mmol), concentrated HCI (35.0 eq) was added and the reaction mixture was stirred at rt for 4 h. Then, solvents were evaporated under reduced pressure and the resulting solid was washed with anhydrous diethyl ether and dried under vacuum to afford the hydrochloride salt of the corresponding final compound of formula (I).

### EXAMPLE 3. General procedure for the synthesis of compounds of formula (I) wherein X is O and R₄ is H (Method C, Scheme 2).

.. To a solution of intermediate (3) (1.0 eq), synthesized according to the general procedure described in Example 1, in anhydrous methanol (5 mL/mmol) at 0 °C under nitrogen atmosphere, NaBH₄ (2.0 eq) was added and the mixture was allowed to reach rt and stirred for 3 h. Then, the reaction was quenched with a saturated aqueous solution of NaHCO₃. The solvent was evaporated under reduced pressure, and the residue was suspended in water and extracted with EtOAc (x2). The combined organic layers were washed with brine, dried over Na₂SO₄, filtered and evaporated under reduced pressure to afford derivative (7), which was used without further purification.

.. Next, (7) (1.0 eq) was dissolved in a 1:1 mixture of acetic acid and anhydrous methanol (4 mL/mmol) under nitrogen atmosphere, and zinc (10.0 eq) was added. The reaction was stirred until complete conversion of starting material (thin layer chromatography). Then, the mixture was filtered, washing with methanol, and the filtrate was evaporated under reduced pressure. The residue was suspended in a saturated aqueous solution of NaHCO₃ and extracted with DCM (x3). The combined organic layers were washed with brine, dried over Na₂SO₄, filtered and concentrated under reduced pressure. The crude was purified by flash chromatography (DCM/methanol, 20:1 to 5:1, with 0.5% NH₃) to afford the corresponding final compound of formula (I) wherein R₃, R₄ are H and R₅ group is CH₃, COCH₃, or the Boc protected derivative of final compound of formula (I) wherein R₅ group is H. The Boc protected derivative (1.0 eq) was dissolved in methanol (2.5 mL/mmol), concentrated HCI (35.0 eq) was added and the reaction mixture was stirred at rt for 4 h. Then, the solvent was evaporated under reduced pressure and the resulting solid was washed with anhydrous diethyl ether and dried under vacuum to afford the hydrochloride salt of the corresponding final compound of formula (I) wherein R₃, R₄ are H and R₅ group is H.

.. In the case of final compounds of formula (I) wherein R₃ is different from H and R₄ is H, *N*-alkylation or *N*-acylation was performed as described in Example 2.

### EXAMPLE 4. General procedure for the synthesis of compounds of formula (I) wherein X is O and and R₄ is different from H (Method D, Scheme 2).

.. To a solution of derivative (7) (1.0 eq), synthesized according to the procedure described in Example 3, in anhydrous methanol (7 mL/mmol) at 0 °C under nitrogen atmosphere, nickel chloride hexahydrate (0.15 eq) was added, and the reaction was stirred at this temperature for 10 min. Then, NaBH₄ (12.0 eq) was added portionwise over 30 min and the reaction was stirred at 0 °C for 30 min. Next, di-*tert*-butyl dicarbonate (2.0 eq) was added and the reaction was allowed to reach rt and stirred overnight. The reaction was then quenched with a 2:1:1 mixture of brine, saturated aqueous solution of NaHCO₃ and EtOAc, and extracted with EtOAc (x2). The organic phase was concentrated under reduced pressure and the residue was dissolved in CHCl₃ and filtered through celite. The solvent was evaporated under reduced pressure and the crude product was purified by flash chromatography (hexane/EtOAc, 8:2 to 7:3) to afford derivative (8).

.. Next, cesium hydroxide (1.2 equiv) was added at rt to a solution of the obtained *N*-Boc protected derivative (8) (1.0 equiv) in DMF (2.25 mL/mmol) and the reaction was stirred at rt during 1 h. Then, the corresponding halogenated derivative (6.0 equiv) was added dropwise and the reaction was heated at 50 °C for 24 h. After this time, water was added to quench the reaction and the mixture was extracted with EtOAc (x2). The organic phase was dried over Na₂SO₄ and concentrated under reduced pressure. Finally the crude product was purified by flash chromatography (hexane to hexane:EtOAc 7:3) to provide derivative (9), which was *N*-deprotected by treatment with concentrated HCI, as described in Example 2, to afford the hydrochloride salt of the corresponding final compound of formula (**I**) wherein X is O, R₃ is H, and R₄ is different from H.

.. In the case of final compounds of formula (**I**) wherein X is O and R₃, R₄ are different from H, *N*-alkylation or *N*-acylation was performed as described in Example 2.

### EXAMPLE 5. (2R,3R,4R)-4-{2-[(Cyclopropylmethyl)amino]ethyl}-2-phenyl-2,3,4,9-tetrahydro-1H-carbazol-3-amine (Ia).

Obtained following general procedure described in Example 1. [α]_{D}²⁰ = +11 (c 1.00 g/100 mL, methanol). IR (ATR): v (cm⁻¹) 3363, 2949. ¹H-NMR (300 MHz, methanol-*d*₄): δ (ppm) 0.16-0.44 (m, 2H, CH₂), 0.61 (d, *J =* 7.2 Hz, 2H, CH₂), 0.87-1.08 (m, 1H, CH), 2.22-2.46 (m, 2H, CH₂), 2.58-2.88 (m, 3H, ½CH₂, CH₂), 3.00-3.26 (m, 3H, ½CH₂, CH₂), 3.37-3.48 (m, 1H, CH), 3.48-3.63 (m, 1H, CH), 3.94-4.10 (m, 1H, CH), 7.05-7.12 (m, 2H, 2CH_{Ar}), 7.33-7.46 (m, 6H, 6CH_{Ar}), 7.57 (d, *J =* 7.5 Hz, 1H, CH_{Ar}).¹³C-NMR (75 MHz, methanol-*d*₄): δ (ppm) 4.5, 4.7, 8.2, 27.9, 29.4, 37.7, 45.2, 45.3, 53.8, 56.2, 106.7, 112.4, 118.8, 120.5, 122.6, 127.3, 129.3, 130.6, 134.7, 138.4, 141.0. MS (ESI, *m*/*z):* 360.3 [M+H]⁺.

### EXAMPLE 6. (2R,3R,4R)-4-{2-[(Cyclopropylmethyl)amino]ethyl}-2-(4-methylphenyl)-2,3,4,9-tetrahydro-1H-carbazol-3-amine (Ib).

Obtained following general procedure described in Example 1. [α]_{D}²⁰ = +12 (c 1.00 g/100 mL, methanol). IR (ATR): v (cm⁻¹) 3358, 2920. ¹H-NMR (300 MHz, methanol-*d*₄): δ (ppm) 0.22-0.41 (m, 2H, CH₂), 0.62 (d, *J =* 7.8 Hz, 2H, CH₂), 0.89-1.08 (m, 1H, CH), 2.23-2.45 (m, 5H, CH₂, CH₃), 2.62-2.82 (m, 3H, CH₂, ½CH₂), 3.02-3.28 (m, 3H, CH₂, ½CH₂), 3.36-3.42 (m, 1H, CH), 3.46-3.57 (m, 1H, CH), 3.97 (t, *J =* 7.7 Hz, 1H, CH), 7.01-7.17 (m, 2H, 2CH_{Ar}), 7.22-7.38 (m, 5H, 5CH_{Ar}), 7.57 (d, *J =* 7.6 Hz, 1H, CH_{Ar}). ¹³C-NMR (75 MHz, methanol-*d*₄): δ (ppm) 4.5, 4.6, 8.1, 21.1, 27.9, 29.2, 37.6, 44.8, 45.1, 53.7, 56.1, 106.7, 112.4, 118.8, 120.5, 122.6, 127.4, 129.1, 131.1, 134.7, 138.0, 138.4, 139.2. MS (ESI, *m*/*z):* 374.3 [M+H]⁺.

### EXAMPLE 7. (2R,3R,4R)-4-{2-[(Cyclopropylmethyl)amino]ethyl}-2-(4-methoxyphenyl)-2,3,4,9-tetrahydro-1H-carbazol-3-amine (Ic).

Obtained following general procedure described in Example 1. [α]_{D}²⁰ = +6.8 (c 0.30 g/100 mL, methanol). IR (ATR): v (cm⁻¹) 3375, 1458. ¹H-NMR (300 MHz, methanol-*d*₄): δ (ppm) 0.26-0.36 (m, 2H, CH₂), 0.55-0.65 (m, 2H, CH₂), 0.93-1.02 (m, 1H, CH), 2.21-2.43 (m, 2H, CH₂), 2.64-2.82 (m, 3H, CH₂, ½CH₂), 3.01-3.24 (m, 3H, CH₂, ½CH₂), 3.29-3.38 (m, 1H, CH), 3.48-3.55 (m, 1H, CH), 3.79 (s, 3H, CH₃), 3.92-3.97 (m, 1H, CH), 6.96 (d, *J =* 8.3 Hz, 2H, 2CH_{Ar}), 7.01-7.11 (m, 2H, 2CH_{Ar}), 7.32 (d, *J =* 7.7 Hz, 1H, CH_{Ar}), 7.37 (d, *J =* 8.5 Hz, 2H, 2CH_{Ar}), 7.56 (d, *J =* 7.7 Hz, 1H, CH_{Ar}). 13C-NMR (75 MHz, methanol-*d*₄): δ (ppm) 4.4, 4.6, 8.1, 27.8, 29.4, 37.6, 44.4, 45.1, 53.7, 55.8, 56.1, 106.7, 112.3, 115.8, 118.8, 120.4, 122.5, 127.4, 130.3, 132.7, 134.8, 138.3, 161.0. MS (ESI, *m*/*z):* 390.1 [M+H]⁺.

### EXAMPLE 8. (2S,3S,4S)-4-{2-[(Cyclopropylmethyl)amino]ethyl}-2-(4-methoxyphenyl)-2,3,4,9-tetrahydro-1H-carbazol-3-amine (Id).

Obtained following general procedure described in Example 1 using (S)-2-{diphenyl[(trimethylsilyl)oxy]methyl} pyrrolidine. [α]^{D}₂₀ = -5.4 (c = 0.24 g/100 mL, methanol). For spectroscopic data, see compound (**Ic**) in Example 7.

### EXAMPLE 9. (2R,3R,4R)-4-{2-[(Cyclopropylmethyl)amino]ethyl}-2-[4-(trifluoromethyl)phenyl]-2,3,4,9-tetrahydro-1H-carbazol-3-amine (Ie).

Obtained following general procedure described in Example 1. [α]_{D}²⁰ = +13 (c 1.00 g/100 mL, methanol). IR (ATR): v (cm⁻¹) 3387, 1459. ¹H-NMR (300 MHz, methanol-*d*₄): δ (ppm) 0.33 (dd, *J* = 10.3, 4.9 Hz, 2H, CH₂), 0.56-0.67 (m, 2H, CH₂), 0.95-1.05 (m, 1H, CH), 2.28-2.49 (m, 2H, CH₂), 2.65-2.82 (m, 3H, CH₂, ½CH₂), 3.08-3.27 (m, 3H, ½CH₂, CH₂), 3.48-3.58 (m, 2H, 2CH), 4.12 (dd, *J* = 9.5, 6.9 Hz, 1H, CH), 7.04-7.09 (m, 1H, CH_{Ar}), 7.11-7.16 (m, 1H, CH_{Ar}), 7.35 (d, *J =* 8.2 Hz, 1H, CH_{Ar}), 7.59 (d, *J* = 8.0 Hz, 1H, CH_{Ar}), 7.74 (app d, *J =* 2.8 Hz, 4H, 4CH_{Ar}). ¹³C-NMR (75 MHz, methanol-*d*₄): δ (ppm) 4.5, 4.6, 8.2, 27.8, 29.4, 37.8, 45.0, 45.2, 53.7, 55.8, 106.8, 112.4, 118.9, 120.5, 122.7, 125.6 (q, *J =* 275.0 Hz), 127.3, 127.34 (q, *J =* 3.7 Hz), 130.2, 131.3 (q, *J* = 32.4 Hz), 134.4, 138.4, 145.7. MS (ESI, *m*/*z):* 428.2 [M+H]⁺.

### EXAMPLE 10. (2R,3R,4R)-4-{2-[(Cyclopropylmethyl)amino]ethyl}-2-[4-(trifluoromethoxy)phenyl]-2,3,4,9-tetrahydro-1H-carbazol-3-amine (If).

Obtained following general procedure described in Example 1. [α]_{D}²⁰ = +12 (c 1.00 g/100 mL, methanol). IR (ATR): v (cm⁻¹) 3229, 1458. ¹H-NMR (700 MHz, methanol-*d*₄): δ (ppm) 0.33 (d, *J =* 32.1 Hz, 2H, CH₂), 0.61 (d, *J =* 6.8 Hz, 2H, CH₂), 1.01 (br s, 1H, CH), 2.35 (br s, 1H, ½CH₂), 2.49 (br s, 1H, ½CH₂), 2.68-2.79 (m, 3H, CH₂, ½CH₂), 3.16 (d, *J =* 12.6 Hz, 1H, ½CH₂), 3.16-3.24 (m, 2H, CH₂), 3.47 (br s, 1H, CH), 3.57 (br s, 1H, CH), 4.06 (br s, 1H, CH), 7.06 (t, *J =* 7.3 Hz, 1H, CH_{Ar}), 7.12 (t, *J* = 7.5 Hz, 1H, CH_{Ar}), 7.33-7.37 (m, 3H, 3CH_{Ar}), 7.60 (d, *J =* 7.8 Hz, 1H, CH_{Ar}), 7.63 (br s, 2H, 2CH_{Ar}). ¹³C-NMR (175 MHz, methanol-*d*₄): δ (ppm) 4.5, 4.7, 8.2, 27.7, 30.0, 37.8, 45.0, 45.1, 53.8, 56.0, 106.7, 112.4, 118.9, 120.5, 121.9 (q, *J =* 275.0 Hz), 122.7, 123.0, 127.3, 131.3, 134.6, 138.3, 140.3, 150.2 (q, *J =* 2.1 Hz). MS (ESI, *m*/*z*): 444.2 [M+H]⁺.

### EXAMPLE 11. (2R,3S,4R)-4-{2-[(Cyclopropylmethyl)amino]ethyl}-2-(2-fluorophenyl)-2,3,4,9-tetrahydro-1H-carbazol-3-amine (Ig).

Obtained following general procedure described in Example 1. [α]_{D}²⁰ = +8 (c 1.00 g/100 mL, methanol). IR (ATR): v (cm⁻¹) 3357, 2921. ¹H-NMR (300 MHz, methanol-*d*₄): δ (ppm) 0.23-0.43 (m, 2H, CH₂), 0.62 (d, *J =* 7.4 Hz, 2H, CH₂), 0.91-1.08 (m, 1H, CH), 2.20-2.42 (m, 2H, CH₂), 2.64-2.86 (m, 3H, CH₂, ½CH₂), 3.03-3.27 (m, 3H, CH₂, ½CH₂), 3.50-3.61 (m, 1H, CH), 3.70-3.86 (m, 1H, CH), 3.98-4.17 (m, 1H, CH), 7.02-7.17 (m, 2H, 2CH_{Ar}), 7.18-7.29 (m, 2H, 2CH_{Ar}), 7.35 (d, *J =* 7.9 Hz, 1H, CH_{Ar}), 7.38-7.55 (m, 2H, 2CH_{Ar}), 7.58 (d, *J =* 7.6 Hz, 1H, CH_{Ar}). ¹³C-NMR (75 MHz, methanol-*d*₄): δ (ppm) 4.6, 4.7, 8.1, 28.1, 28.3, 37.5, 38.5, 45.4, 53.8, 54.5, 106.7, 112.4, 117.3 (d, *J = 21.8* Hz), 118.9, 120.5, 122.7, 126.5 (d, *J =* 3.1 Hz_{'}), 127.3, 127.6 (d, *J =* 13.1 Hz), 130.6 (d, *J =* 7.1 Hz), 131.3 (d, *J =* 8.1 Hz), 134.1, 138.4, 162.6 (d, *J =* 245.2 Hz). MS (ESI, *m*/*z):* 378.2 [M+H]⁺.

### EXAMPLE 12. (2R,3R,4R)-2-(3-Chlorophenyl)-4-{2-[(cyclopropylmethyl) amino]ethyl}-2,3,4,9-tetrahydro-1H-carbazol-3-amine (Ih).

Obtained following general procedure described in Example 1. [α]_{D}²⁰ = +12 (c 1.00 g/100 mL, methanol). IR (ATR): v (cm⁻¹) 3382, 2921. ¹H-NMR (300 MHz, methanol-*d*₄): δ (ppm) 0.22-0.41 (m, 2H, CH₂), 0.61 (d, *J =* 7.7 Hz, 2H, CH₂), 0.93-1.08 (m, 1H, CH), 2.28-2.52 (m, 2H, CH₂), 2.63-2.88 (m, 3H, CH₂, ½CH₂), 3.02-3.25 (m, 3H, CH₂, ½CH₂), 3.37-3.49 (m, 1H, CH), 3.49-3.59 (m, 1H, CH), 3.96-4.11 (m, 1H, CH), 7.58 (d, *J* = 9.2 Hz, 2H, 2CH_{Ar}), 7.34 (d, *J =* 7.8 Hz, 1H, CH_{Ar}), 7.37-7.49 (m, 3H, 3CH_{Ar}), 7.58 (d, *J* = 9.2 Hz, 2H, 2CH_{Ar}). ¹³C-NMR (75 MHz, methanol-*d*₄): δ (ppm) 4.5, 4.7, 8.2, 27.8, 29.6, 37.8, 45.1, 45.2, 53.8, 55.9, 106.7, 112.4, 118.9, 120.5, 122.7, 127.2, 127.9, 129.5 (2C), 132.1, 134.5, 136.3, 138.4, 143.4. MS (ESI, *m*/*z):* 394.2 [M+H]⁺

### EXAMPLE 13. (2R,3R,4R)-2-(4-Chlorophenyl)-4-{2-[(cyclopropylmethyl)amino]ethyl}-2,3,4,9-tetrahydro-1H-carbazol-3-amine (Ii).

Obtained following general procedure described in Example 1. [α]_{D}²⁰ = +12 (c 1.00 g/100 mL, methanol). IR (ATR): v (cm⁻¹) 3388, 1458. ¹H-NMR (300 MHz, methanol-*d*₄): δ (ppm) 0.33 (d, *J =* 8.6 Hz, 2H, CH₂), 0.62 (d, *J =* 7.0 Hz, 2H, CH₂), 1.00 (br s, 1H, CH), 2.34 (br s, 2H, CH₂), 2.73 (br s, 3H, CH₂, ½CH₂), 3.07-3.19 (m, 3H, ½CH₂, CH₂), 3.46 (br s, 1H, CH), 3.53 (br s, 1H, CH), 4.03 (br s, 1H, CH), 7.05 (t, *J =* 7.0 Hz, 1H, CH_{Ar}), 7.12 (d, *J =* 7.4 Hz, 1H, CH_{Ar}), 7.34 (d, *J =* 7.8 Hz, 1H, CH_{Ar}), 7.39-7.53 (m, 4H, 4CH_{Ar}), 7.58 (d, *J =* 7.5 Hz, 1H, CH_{Ar}). ¹³C-NMR (75 MHz, methanol-*d*₄): δ (ppm) 4.6, 4.7, 8.2, 28.0, 29.3, 37.6, 44.6, 45.4, 53.9, 56.0, 106.7, 112.4, 118.9, 120.5, 122.7, 127.3, 130.6, 131.1, 134.4, 135.0, 138.4, 139.9. MS (ESI, *m*/*z):* 394.2 [M+H]⁺.

### EXAMPLE 14. (2R,3R,4R)-4-{2-[(Cyclopropylmethyl)amino]ethyl}-2-(3-methoxyphenyl)-2,3,4,9-tetrahydro-1H-carbazol-3-amine (Ij).

Obtained following general procedure described in Example 1. [α]_{D}²⁰ = +14 (c 1.00 g/100 mL, methanol). IR (ATR): v (cm⁻¹) 3380, 2928. ¹H-NMR (300 MHz, methanol-*d*₄): δ (ppm) 0.23-0.42 (m, 2H, CH₂), 0.61 (d, *J =* 7.9 Hz, 2H, CH₂), 0.87-1.04 (m, 1H, CH), 2.24-2.53 (m, 2H, CH₂), 2.64-2.83 (m, 3H, CH₂, ½CH₂), 2.97-3.25 (m, 3H, ½CH₂, CH₂), 3.31-3.41 (m, 1H, CH), 3.46-3.57 (m, 1H, CH), 3.83 (s, 3H, CH₃), 4.01 (t, *J =* 8.2 Hz, 1H, CH), 6.93 (d, *J =* 9.7 Hz, 1H, CH_{Ar}), 7.00-7.16 (m, 4H, 4CH_{Ar}), 7.34 (d, *J =* 7.8 Hz, 2H, 2CH_{Ar}), 7.58 (d, *J =* 7.7 Hz, 1H, CH_{Ar}). ¹³C-NMR (75 MHz, methanol-*d*₄): δ (ppm) 4.4, 4.6, 8.1, 27.7, 29.5, 37.7, 45.0, 45.5, 53.7, 55.8, 56.0, 106.6, 112.4, 114.6, 115.0, 118.8, 120.5, 121.3, 122.6, 127.3, 131.6, 134.8, 138.4, 142.5, 162.0. MS (ESI, *m*/*z):* 390.3 [M+H]⁺.

### EXAMPLE 15. (2R,3S,4R)-4-{2-[(Cyclopropylmethyl)amino]ethyl}-2-(2-methoxyphenyl)-2,3,4,9-tetrahydro-1H-carbazol-3-amine (Ik).

Obtained following general procedure described in Example 1. [α]_{D}²⁰ = -24 (c 1.00 g/100 mL, methanol). IR (ATR): v (cm⁻¹) 3224, 2927. ¹H-NMR (300 MHz, methanol-*d*₄): δ (ppm) 0.25-0.41 (m, 2H, CH₂), 0.61 (d, *J =* 7.6 Hz, 2H, CH₂), 0.86-1.10 (m, 1H, CH), 2.05-2.33 (m, 2H, CH₂), 2.54-2.83 (m, 3H, CH₂, ½CH₂), 3.00-3.16 (m, 2H, ½CH₂, ½CH₂), 3.23-3.29 (m, 1H, ½CH₂), 3.44-3.55 (m, 1H, CH), 3.73-3.85 (m, 1H, CH), 3.94 (s, 3H, CH₃), 4.10-4.24 (m, 1H, CH), 6.96 (t, *J* = 7.0 Hz, 1H, CH_{Ar}), 7.00-7.16 (m, 3H, 3CH_{Ar}), 7.25-7.41 (m, 3H, 3CH_{Ar}), 7.57 (d, *J =* 7.6 Hz, 1H, CH_{Ar}). ¹³C-NMR (75 MHz, methanol-*d*₄): δ (ppm) 4.5, 4.7, 8.1, 27.1, 28.4, 37.3, 39.3, 45.5, 53.7, 53.8, 56.2, 106.7, 112.3, 112.5, 118.8, 120.4, 122.4, 122.5, 127.5, 128.3, 129.9, 130.5, 134.5, 138.4, 159.0. MS (ESI, *m*/*z):* 390.2 [M+H]⁺.

### EXAMPLE 16. (2R,3R,4R)-4-{2-[(Cyclopropylmethyl)amino]ethyl}-2-(3-methylphenyl)-2,3,4,9-tetrahydro-1H-carbazol-3-amine (II).

Obtained following general procedure described in Example 1. [α]_{D}²⁰ = +35 (c 1.00 g/100 mL, methanol). IR (ATR): v (cm⁻¹) 3393, 2955. ¹H-NMR (300 MHz, methanol-*d*₄): δ (ppm) 0.25-0.39 (m, 2H, CH₂), 0.61 (d, *J =* 7.8 Hz, 2H, CH₂), 0.91-1.06 (m, 1H, CH), 2.24-2.35 (m, 1H, ½CH₂), 2.38 (s, 3H, CH₃), 2.41-2.52 (m, 1H, ½CH₂), 2.61-2.85 (m, 3H, ½CH₂, CH₂), 3.02-3.28 (m, 3H, ½CH₂, CH₂), 3.33-3.40 (m, 1H, CH), 3.49-3.57 (m, 1H,CH), 4.01 (t, *J =* 8.1 Hz, 1H, CH), 7.06 (td, *J =* 7.5, 1.3 Hz, 1H, CH_{Ar}), 7.12 (ddd, *J =* 8.1, 7.1, 1.3 Hz, 1H, CH_{Ar}), 7.18-7.39 (m, 5H, 5CH_{Ar}), 7.58 (d, *J* = 7.2 Hz, 1H, CH_{Ar}). ¹³C-NMR (75 MHz, methanol-*d*₄): δ (ppm) 4.5, 4.6, 8.2, 21.6, 27.8, 29.6, 37.7, 45.0, 45.4, 53.7, 56.1, 106.6, 112.4, 118.8, 120.5, 122.6, 126.3, 127.3, 129.8, 130.0, 130.5, 134.9, 138.4, 140.5, 140.9. MS (ESI, *m*/*z):* 374.2 [M+H]⁺.

### EXAMPLE 17. (2R,3R,4R)-4-{2-[(Cyclopropylmethyl)amino]ethyl}-2-(2-methylphenyl)-2,3,4,9-tetrahydro-1H-carbazol-3-amine (Im).

Obtained following general procedure described in Example 1. [α]_{D}²⁰ = +31 (c 1.00 g/100 mL, methanol). IR (ATR): v (cm⁻¹) 3391, 2937. ¹H-NMR (700 MHz, methanol-*d*₄): δ (ppm) 0.29-0.32 (m, 1H, ½CH₂), 0.35-0.38 (m, 2H, ½CH₂), 0.61 (d, *J =* 8.1 Hz, 2H, CH₂), 0.98-1.02 (m, 1H, CH), 2.36 (t, *J =* 12.8 Hz, 1H, ½CH₂), 2.47 (s, 3H, CH₃), 2.51-2.59 (m, 1H, ½CH₂), 2.66-2.74 (m, 2H, ½CH₂, ½CH₂), 2.78 (dd, *J =* 12.9, 7.4 Hz, 1H, ½CH₂), 3.09 (app d, *J =* 6.7 Hz, 2H, ½CH₂, ½CH₂), 3.16 (td, *J =* 12.0, 2.9 Hz, 1H, ½CH₂), 3.59-3.63 (m, 1H, CH), 3.69 (dd, *J =* 16.6, 7.5 Hz, 1H, CH), 4.10 (dd, *J* = 9.1, 7.2 Hz, 1H, CH), 7.07 (t, *J =* 8.1 Hz, 1H, CH_{Ar}), 7.12 (t, *J =* 7.0 Hz, 1H, CH_{Ar}), 7.25 (t, *J =* 7.3 Hz, 1H, CH_{Ar}),7.29 (app t, *J =* 7.1 Hz, 2H, 2CH_{Ar}), 7.35 (d, *J =* 8.0 Hz, 1H, CH_{Ar}), 7.51 (d, *J =* 7.6 Hz, 1H, CH_{Ar}), 7.61 (d, *J =* 7.9 Hz, 1H, CH_{Ar}). ¹³C-NMR (175 MHz, methanol-*d*₄): δ (ppm) 4.5, 4.6, 8.2, 19.7, 27.6, 30.0, 37.8, 40.7, 45.0, 53.7, 54.9, 106.7, 112.3, 118.9, 120.5, 122.6, 127.3, 127.5, 128.4, 128.9, 132.5, 134.9, 138.2, 138.3, 139.1. MS (ESI, *m*/*z):* 374.2 [M+H]⁺.

### EXAMPLE 18. (2R,3S,4R)-2-(2-Chlorophenyl)-4-{2-[(cyclopropylmethyl)amino]ethyl}-2,3,4,9-tetrahydro-1H-carbazol-3-amine (In).

Obtained following general procedure described in Example 1. [α]_{D}²⁰ = +5 (c 1.00 g/100 mL, methanol). IR (ATR): v (cm⁻¹) 3389, 2953. ¹H-NMR (300 MHz, methanol-*d*₄): δ (ppm) 0.27-0.42 (m, 2H, CH₂), 0.63 (dd, *J =* 8.2, 1.9 Hz, 2H, CH₂), 0.93-1.05 (m, 1H, CH), 2.16-2.32 (m, 2H, CH₂), 2.68-2.82 (m, 3H, ½CH₂, CH₂), 3.12-3.23 (m, 3H, ½CH₂, CH₂), 3.56 (q, *J =* 5.3 Hz, 1H, CH), 3.99 (q, *J =* 7.1 Hz, 1H, CH), 4.22 (dd, *J* = 8.3, 5.8 Hz, 1H, CH), 7.04-7.10 (m, 1H, CH_{Ar}), 7.11-7.17 (m, 1H, CH_{Ar}), 7.33-7.40 (m, 3H, 3CH_{Ar}), 7.51-7.60 (m, 3H, 3CH_{Ar}). ¹³C-NMR (75 MHz, methanol-*d*₄): δ (ppm) 4.5, 4.6, 8.1, 28.0, 28.5, 37.4, 41.7, 45.4, 53.7, 53.8, 106.9, 112.4, 118.9, 120.5, 122.8, 127.4, 129.3, 130.7, 131.5, 131.6, 133.8, 135.5, 138.4, 138.5. MS (ESI, *m*/*z):* 394.2 [M+H]⁺.

### EXAMPLE 19. (2R,3R,4R)-4-{2-[(Cyclopropylmethyl)amino]ethyl}-2-(4-fluorophenyl)-2,3,4,9-tetrahydro-1H-carbazol-3-amine (Io).

Obtained following general procedure described in Example 1. [α]_{D}²⁰ = +9 (c 1.00 g/100 mL, methanol). IR (ATR): v (cm⁻¹) 3384, 2949. ¹H-NMR (300 MHz, methanol-*d*₄): δ (ppm) 0.26-0.40 (m, 2H, CH₂), 0.62 (dd, *J =* 8.0, 1.9 Hz, 2H, CH₂), 0.92-1.06 (m, 1H, CH), 2.24-2.48 (m, 2H, CH₂), 2.65-2.86 (m, 3H, ½CH₂, CH₂), 3.04-3.26 (m, 3H, ½CH₂, CH₂), 3.37-3.47 (m, 1H, CH), 3.50-3.54 (m, 1H, CH), 4.00 (dd, *J =* 9.4, 7.0 Hz, 1H, CH), 7.03-7.20 (m, 4H, 4CH_{Ar}), 7.34 (d, *J =* 7.6 Hz, 1H, CH_{Ar}), 7.51 (dd, J = 8.7, 5.2 Hz, 2H, 2CH_{Ar}), 7.58 (d, *J =* 7.6 Hz, 1H, CH_{Ar}). ¹³C-NMR (75 MHz, methanol-*d*₄): δ (ppm) 4.5, 4.6, 8.2, 27.9, 29.6, 37.7, 44.7, 45.1, 53.8, 56.1, 106.7, 112.4, 117.2 (d, *J =* 21.6 Hz), 118.8, 120.5, 122.7, 127.3, 131.2 (d, *J =* 8.1 Hz), 134.6, 137.1 (d, *J =* 3.1 Hz), 138.4, 163.3 (d, *J =* 267.0 Hz). MS (ESI, *m*/*z):* 378.2 [M+H]⁺.

### EXAMPLE 20. (2S,3S,4S)-4-{2-[(Cyclopropylmethyl)amino]ethyl}-2-(4-fluorophenyl)-2,3,4,9-tetrahydro-1H-carbazol-3-amine (Ip).

Obtained following general procedure described in Example 1 using (S)-2-{diphenyl[(trimethylsilyl)oxy]methyl} pyrrolidine. [α]^{D}₂₀ = -11 (c = 1.00 g/100 mL, methanol). For spectroscopic data, see compound (**Io**) in Example 19.

### EXAMPLE 21. (2R,3R,4R)-4-{2-[(Cyclopropylmethyl)amino]ethyl}-2-(3-fluorophenyl)-2,3,4,9-tetrahydro-1H-carbazol-3-amine (Iq).

Obtained following general procedure described in Example 1. [α]_{D}²⁰ = +11 (c 1.00 g/100 mL, methanol). IR (ATR): v (cm⁻¹) 3238, 2969. ¹H-NMR (300 MHz, methanol-*d*₄): δ (ppm) 0.26-0.40 (m, 2H, CH₂), 0.62 (dd, *J =* 8.1, 1.8 Hz, 2H, CH₂), 0.94-1.05 (m, 1H, CH), 2.27-2.52 (m, 2H, CH₂), 2.65-2.87 (m, 3H, ½CH₂, CH₂), 3.06-3.28 (m, 3H, ½CH₂, CH₂), 3.43 (td, *J =* 9.2, 5.0 Hz, 1H, CH), 3.50-3.56 (m, 1H, CH), 4.04 (dd, *J =* 9.5, 7.1 Hz, 1H, CH), 7.03-7.16 (m, 3H, 3CH_{Ar}), 7.28-7.36 (m, 3H, 3CH_{Ar}), 7.47 (td, *J =* 8.2, 6.2 Hz, 1H, CH_{Ar}), 7.59 (d, *J =* 7.2 Hz, 1H, CH_{Ar}).¹³C-NMR (75 MHz, methanol-*d*₄): δ (ppm) 4.5, 4.6, 8.2, 27.8, 29.6, 37.8, 45.0, 45.3, 53.8, 56.0, 106.6, 112.4, 116.1 (d, *J =* 22.3 Hz), 118.9, 120.5, 122.7, 125.3 (d, *J =* 3.0 Hz), 127.3, 132.4 (d, *J* = 8.9 Hz), 134.5, 138.4, 143.8 (d, *J* = 7.3 Hz), 164.8 (d, *J =* 247.2 Hz). MS (ESI, *m*/*z):* 378.2 [M+H]⁺.

### EXAMPLE 22. (2R,3R,4R)-4-{2-[(Cyclopropylmethyl)amino]ethyl}-2-(4-methoxyphenyl)-N-methyl-2,3,4,9-tetrahydro-1H-carbazol-3-amine (Ir).

Obtained following general procedure described in Example 2. [α]_{D}²⁰ = +12 (c 1.00 g/100 mL, methanol). IR (ATR): v (cm⁻¹) 3379, 2956. ¹H-NMR (300 MHz, methanol-*d*₄): δ (ppm) 0.22-0.41 (m, 2H, CH₂), 0.63 (d, *J =* 6.7 Hz, 2H, CH₂), 0.88-1.04 (m, 1H, CH), 1.50-1.67 (m, 1H, ½CH₂), 1.94-2.09 (m, 1H, ½CH₂), 2.52-2.76 (m, 2H, CH₂), 2.84 (br s, 3H, CH₃), 3.05-3.22 (m, 4H, CH₂, CH₂), 3.51-3.60 (m, 1H, CH), 3.77 (br s, 4H, CH₃, CH), 3.85-3.91 (m, 1H, CH), 6.85-6.98 (m, 2H, 2CH_{Ar}), 6.99-7.16 (m, 2H, 2CH_{Ar}), 7.28-7.43 (m, 3H, 3CH_{Ar}), 7.50-7.59 (m, 1H, CH_{Ar}). ¹³C-NMR (75 MHz, methanol-*d*₄): δ (ppm) 4.6, 4.7, 8.1, 25.4, 29.7, 32.6, 34.8, 39.7, 46.5, 53.8, 55.8, 63.5, 107.2, 112.3, 115.6, 118.9, 120.4, 122.7, 127.7, 130.5, 132.8, 133.1, 138.4, 160.7. MS (ESI, *m*/*z):* 404.3 [M+H].

### EXAMPLE 23. (2S,3S,4S)-4-{2-[(Cyclopropylmethyl)amino]ethyl}-2-(4-methoxyphenyl)-N-methyl-2,3,4,9-tetrahydro-1H-carbazol-3-amine (Is).

Obtained following general procedure described in Example 2 using (S)-2-{diphenyl[(trimethylsilyl)oxy]methyl} pyrrolidine. [α]^{D}₂₀ = +10 (c = 1.00 g/100 mL, methanol). For spectroscopic data, see compound (**Ir**) in Example 22.

### EXAMPLE 24. N-[(2R,3R,4R)-4-{2-[(Cyclopropylmethyl)amino]ethyl}-2-(4-methoxyphenyl)-2,3,4,9-tetrahydro-1H-carbazol-3-yl]acetamide (It).

Obtained following general procedure described in Example 2. [α]_{D}²⁰ = +12 (c 1.00 g/100 mL, methanol). IR (ATR): v (cm⁻¹) 3254, 2921, 1633. ¹H-NMR (300 MHz, methanol-*d*₄): δ (ppm) 0.31 (d, *J* = 4.6 Hz, 2H, CH₂), 0.62 (d, *J* = 7.7 Hz, 2H, CH₂), 0.85-1.08 (m, 1H, CH), 1.72 (s, 3H, CH₃), 2.01-2.25 (m, 1H, ½CH₂), 2.61-2.86 (m, 4H, ½CH₂, ½CH₂, CH₂), 2.92-3.23 (m, 5H, ½CH₂, CH₂, 2CH), 3.78 (s, 3H, CH₃), 4.36 (t, *J* = 10.2 Hz, 1H, CH), 6.87 (d, *J =* 8.0 Hz, 2H, 2CH_{Ar}), 6.96-7.15 (m, 2H, 2CH_{Ar}), 7.24 (d, *J =* 8.2 Hz, 2H, 2CH_{Ar}), 7.30 (d, *J =* 7.7 Hz, 1H, CH_{Ar}), 7.58 (d, *J =* 7.6 Hz, 1H, CH_{Ar}). ¹³C-NMR (75 MHz, methanol-*d*₄): δ (ppm) 4.4, 4.5, 8.2, 22.5, 26.7, 32.7, 40.8, 44.9, 47.1, 54.0, 54.3, 55.7, 108.2, 112.2, 114.8, 119.1, 120.2, 122.0, 127.6, 129.9, 135.8, 136.1, 138.2, 160.0, 176.8. MS (ESI, *m*/*z):* 432.2 [M+H]⁺.

### EXAMPLE 25. (2R,3R,4R)-4-(2-{[(3,3-Difluorocyclobutyl)methyl]amino}ethyl)-2-(4-methylphenyl)-2,3,4,9-tetrahydro-1H-carbazol-3-amine (Iu).

Obtained following general procedure described in Example 1. [α]_{D}²⁰ = +13 (c 1.00 g/100 mL, methanol). IR (ATR): v (cm⁻¹) 3389, 1458. ¹H-NMR (300 MHz, methanol-*d*₄): δ (ppm) 2.27-2.42 (m, 5H, 2CH₂, CH), 2.36 (s, 3H, CH₃), 2.62-2.71 (m, 3H, ½CH₂, CH₂), 3.01-3.21 (m, 5H, ½CH₂, 2CH₂), 3.35-3.37 (m, 1H, CH), 3.53 (br s, 1H, CH), 3.99 (t, *J =* 8.1 Hz, 1H, CH), 7.06 (t, *J =* 7.0 Hz, 1H, CH_{Ar}), 7.13 (t, *J =* 7.2 Hz, 1H, CH_{Ar}), 7.26 (d, *J =* 7.2 Hz, 2H, 2CH_{Ar}), 7.33-7.37 (m, 3H, 3CH_{Ar}), 7.58 (d, *J =* 7.5 Hz, 1H, CH_{Ar}). ¹³C-NMR (75 MHz, methanol-*d*₄): δ (ppm) 21.2, 25.6, 27.7, 29.6, 37.7, 39.9 (t, *J =* 23.6 Hz), 45.0, 45.8, 53.0, 56.1, 106.6, 112.4, 112.6 (t, *J =* 170.4 Hz), 118.8, 120.5, 122.6, 127.3, 129.1, 131.2, 134.9, 137.9, 138.4, 139.2. MS (ESI, *m*/*z):* 424.2 [M+H]⁺.

### EXAMPLE 26. (2R,3R,4R)-9-methyl-2-(4-methylphenyl)-4-(2-{[(oxetan-3-yl)methyl]amino}ethyl)-2,3,4,9-tetrahydro-1H-carbazol-3-amine (Iv).

Obtained following general procedure described in Example 1. [α]_{D}²⁰ = +13 (c 1.00 g/100 mL, methanol). IR (ATR): v (cm⁻¹) 3288, 1469. ¹H-NMR (300 MHz, methanol-*d*₄): δ (ppm) 2.12-2.50 (m, 6H, CH₂, ½ CH₂, CH₃), 2.55-2.70 (m, 1H, ½ CH₂), 2.78 (dd, *J* = 7.5, 3.2 Hz, 2H, CH₂), 2.81-3.11 (m, 5H, CH₂, CH, CH, CH), 3.18-3.31 (m, 1H, CH), 3.57 (s, 3H, CH₃), 4.28 (dt, *J =* 10.5, 6.1 Hz, 2H, CH₂), 4.68 (td, *J* = 7.8, 6.1 Hz, 2H, CH₂), 7.02 (t, *J =* 6.9 Hz, 1H, CH_{Ar}), 7.11 (t, *J* = 7.2 Hz, 1H, CH_{Ar}), 7.18 = 7.33 (m, 5H, 5 CH_{Ar}), 7.61 (d, *J =* 7.8 Hz, 1H, CH_{Ar}). ¹³C-NMR (75 MHz, methanol-*d*₄): δ (ppm) 21.1, 29.3, 30.7, 31.1, 35.9, 42.1, 46.9, 53.6, 56.6, 77.2, 77.2, 110.0, 110.3, 119.7, 119.9, 121.7, 127.6, 129.2, 130.7, 136.6, 138.0, 139.0, 141.2. MS (ESI, *m*/*z):* 404.2 [M+H]⁺.

### EXAMPLE 27. (2R,3R,4R)-2-(4-Methoxyphenyl)-4-[2-(methylamino)ethyl]-2,3,4,9-tetrahydro-1H-carbazol-3-amine (Iw).

Obtained following general procedure described in Example 1. [α]_{D}²⁰ = +8 (c 1.00 g/100 mL, methanol). IR (ATR): v (cm⁻¹) 3336, 2943. ¹H-NMR (300 MHz, methanol-*d*₄): δ (ppm) 2.21-2.44 (m, 2H, CH₂), 2.57 (s, 3H, CH₃), 2.60-2.72 (m, 1H, ½CH₂), 3.01-3.28 (m, 3H, ½CH₂, CH₂), 3.34-3.42 (m, 1H, CH), 3.49-3.58 (m, 1H, CH), 3.81 (s, 3H, CH₃), 3.96 (t, *J =* 7.9 Hz, 1H, CH), 6.98 (d, *J =* 7.9 Hz, 2H, 2CH_{Ar}), 7.06 (t, *J =* 7.4 Hz, 1H, CH_{Ar}), 7.12 (t, *J* = 6.9 Hz, 1H, CH_{Ar}), 7.31-7.45 (m, 3H, 3CH_{Ar}), 7.58 (d, *J =* 7.8 Hz, 1H, CH_{Ar}).¹³C-NMR (75 MHz, methanol-*d*₄): δ (ppm) 27.7, 29.5, 33.6, 37.5, 44.4, 46.9, 55.8, 56.1, 106.6, 112.3, 115.8, 118.8, 120.5, 122.5, 127.3, 130.3, 132.7, 134.8, 138.4, 161.0.MS (ESI, *m*/*z):* 350.2 [M+H]⁺.

### EXAMPLE 28. (2R,3R,4R)-2-(4-Methylphenyl)-4-{2-[(2-methylpropyl)amino]ethyl}-2,3,4,9-tetrahydro-1H-carbazol-3-amine (Ix).

Obtained following general procedure described in Example 1. [α]_{D}²⁰ = +10 (c 1.00 g/100 mL, methanol). IR (ATR): v (cm⁻¹) 3223, 2959. ¹H-NMR (300 MHz, methanol-*d*₄): δ (ppm) 0.96 (d, *J =* 6.7 Hz, 3H, CH₃), 0.97 (d, *J =* 6.7 Hz, 3H, CH₃), 1.92 (dp, *J* = 13.7, 6.8 Hz, 1H, CH), 2.36 (s, 3H, CH₃), 2.34-2.52 (m, 2H, CH₂), 2.60-2.73 (m, 3H, CH₂, ½CH₂), 3.03-3.28 (m, 3H, ½CH₂, CH₂), 3.34-3.43 (m, 1H, CH), 3.49-3.56 (m, 1H, CH), 3.99 (dd, *J* = 9.2, 7.0 Hz, 1H, CH), 7.06 (td, *J* = 7.5, 1.3 Hz, 1H, CH_{Ar}), 7.12 (td, *J* = 7.6, 1.3 Hz, 1H, CH_{Ar}), 7.26 (d, *J* = 7.9 Hz, 2H, 2CH_{Ar}), 7.32 - 7.39 (m, 3H, 3CH_{Ar}), 7.59 (d, *J* = 7.9 Hz, 1H, CH_{Ar}). ¹³C-NMR (75 MHz, methanol-*d*₄): δ (ppm) 20.2, 20.3, 21.2, 27.3, 27.5, 29.5, 37.7, 44.9, 46.0, 56.0, 56.05, 106.7, 112.4, 118.9, 120.5, 122.6, 127.3, 129.1, 131.1, 134.8, 138.0, 138.4, 139.1. MS (ESI, *m*/*z*): 376.3 [M+H]⁺.

### EXAMPLE 29. (2R,3R,4R)-2-(4-Methylphenyl)-4-{2-[(propan-2-yl)amino]ethyl}-2,3,4,9-tetrahydro-1H-carbazol-3-amine (Iy).

Obtained following general procedure described in Example 1. [α]_{D}²⁰ = +7 (c 1.00 g/100 mL, methanol). IR (ATR): v (cm⁻¹) 3169, 2925. ¹H-NMR (300 MHz, methanol-*d*₄): δ (ppm) 1.18 (d, *J* = 6.5 Hz, 3H, CH₃), 1.23 (d, *J* = 6.5 Hz, 3H, CH₃),2.22-2.33 (m, 2H, CH₂), 2.36 (s, 3H, CH₃), 2.68 (td, *J =* 11.6, 5.8 Hz, 1H, ½CH₂), 3.02-3.29 (m, 4H, ½CH₂, CH₂, CH), 3.39 (td, *J* = 8.6, 5.9 Hz, 1H, CH), 3.49-3.56 (m, 1H, CH), 3.99 (t, *J* = 7.9 Hz, 1H, CH), 7.06 (t, *J* = 6.9 Hz, 1H, CH_{Ar}), 7.13 (t, *J* = 7.6 Hz, 1H, CH_{Ar}), 7.25 (d, *J* = 7.8 Hz, 2H, 2CH_{Ar}), 7.31-7.41 (m, 3H, 3CH_{Ar}), 7.58 (d, *J* = 7.7 Hz, 1H, CH_{Ar}). ¹³C-NMR (75 MHz, methanol-*d*₄): δ (ppm) 19.2, 19.2, 21.1, 28.1, 29.2, 37.7, 42.6, 44.8, 51.8, 56.0, 106.7, 112.4, 118.8, 120.5, 122.6, 127.4, 129.1, 131.1, 134.8, 138.0, 138.4, 139.1. MS (ESI, *m*/*z*): 362.2 [M+H]⁺.

### EXAMPLE 30. (2R,3R,4R)-4-[2-(Ethylamino)ethyl]-2-(4-methylphenyl)-2,3,4,9-tetrahydro-1H-carbazol-3-amine (Iz).

Obtained following general procedure described in Example 1. [α]_{D}²⁰ = +5 (c 1.00 g/100 mL, methanol). IR (ATR): v (cm⁻¹) 2925, 2955. ¹H-NMR (300 MHz, methanol-*d*₄): δ (ppm) 1.22 (t, *J =* 7.3 Hz, 3H, CH₃), 2.23-2.38 (m, 2H, CH₂), 2.36 (s, 3H, CH₃), 2.64 (td, *J* = 11.7, 5.7 Hz, 1H, ½CH₂), 2.81-2.97 (m, 2H, CH₂), 3.03-3.29 (m, 3H, ½CH₂, CH₂), 3.33-3.42 (m, 1H, CH), 3.48-3.56 (m, 1H, CH), 3.98 (dd, *J* = 9.1, 6.8 Hz, 1H, CH), 7.06 (t, *J* = 7.5 Hz, 1H, CH_{Ar}), 7.12 (t, *J* = 8.2 Hz, 1H, CH_{Ar}), 7.25 (d, *J* = 7.6 Hz, 2H, 2CH_{Ar}), 7.33-7.37 (m, 3H, 3CH_{Ar}), 7.58 (d, *J =* 7.3 Hz, 1H, CH_{Ar}). ¹³C-NMR (75 MHz, methanol-*d*₄): δ (ppm) 11.5, 21.1, 27.9, 29.3, 37.6, 44.0, 44.8, 45.0, 56.0, 106.6, 112.3, 118.8, 120.5, 122.6, 127.3, 129.1, 131.1, 134.8, 137.9, 138.4, 139.2. MS (ESI, *m*/*z*): 348.2 [M+H]⁺.

### EXAMPLE 31. (2R,3R,4R)-4-[2-(Cyclopropylamino)ethyl]-2-(4-methylphenyl)-2,3,4,9-tetrahydro-1H-carbazol-3-amine (Iaa).

Obtained following general procedure described in Example 1. [α]_{D}²⁰ = +10 (c 1.00 g/100 mL, methanol). IR (ATR): v (cm⁻¹) 3233, 2925. ¹H-NMR (300 MHz, methanol-*d*₄): δ (ppm) 0.76-0.89 (m, 4H, 2CH₂), 2.35 (s, 3H, CH₃), 2.30-2.47 (m, 2H, CH₂), 2.52-2.60 (m, 1H, CH), 2.76 (td, *J* = 11.9, 5.4 Hz, 1H, ½CH₂), 3.08 (dd, *J* = 16.7, 5.4 Hz, 1H, ½CH₂), 3.18-3.30 (m, 2H, ½CH₂, ½CH₂), 3.35-3.44 (m, 1H, CH), 3.48-3.57 (m, 1H, CH), 4.00 (dd, *J* = 8.9, 7.0 Hz, 1H, CH), 7.06 (t, *J* = 7.4 Hz, 1H, CH_{Ar}), 7.13 (t, *J* = 8.2 Hz, 1H, CH_{Ar}), 7.25 (d, *J* = 7.8 Hz, 2H, 2CH_{Ar}), 7.33-7.41 (m, 3H, 3CH_{Ar}), 7.57 (d, *J* = 7.7 Hz, 1H, CH_{Ar}). ¹³C-NMR (75 MHz, methanol-*d*₄): δ (ppm) 4.0, 4.3, 21.1, 27.7, 29.3, 31.2, 37.6, 44.8, 46.0, 56.0, 106.6, 112.4, 118.8, 120.5, 122.6, 127.4, 129.1, 131.1, 134.8, 137.9, 138.4, 139.1. MS (ESI, *m*/*z*): 360.2 [M+H]⁺.

### EXAMPLE 32. (2R,3R,4R)-6-Chloro-4-(2-{[(3,3-difluorocyclobutyl)methyl]amino}ethyl)-2-(4-methylphenyl)-2,3,4,9-tetrahydro-1H-carbazol-3-amine (Iab).

Obtained following general procedure described in Example 1. [α]_{D}²⁰ = +26 (c 1.00 g/100 mL, methanol). IR (ATR): v (cm⁻¹) 3183, 2926. ¹H-NMR (300 MHz, methanol-*d*₄): δ (ppm) 2.26-2.49 (m, 5H, CH, 2CH₂), 2.36 (s, 3H, CH₃), 2.57-2.75 (m, 3H, CH₂, ½CH₂), 2.96-3.26 (m, 5H, ½CH₂, 2CH₂), 3.34-3.41 (m, 1H, CH), 3.46-3.53 (m, 1H, CH), 4.00 (dd, *J* = 9.0, 6.7 Hz, 1H, CH), 7.10 (dd, *J* = 8.6, 1.9 Hz, 1H, CH_{Ar}), 7.25 (d, *J* = 7.9 Hz, 2H, 2CH_{Ar}), 7.33 = 7.37 (m, 3H, 3CH_{Ar}), 7.59 (d, *J =* 2.0 Hz, 1H, CH_{Ar}). ¹³C-NMR (75 MHz, methanol-*d*₄): δ (ppm) 21.1, 21.7, 27.7, 29.2, 37.3, 39.9 (t, *J* = 23.6 Hz), 44.6, 45.7, 53.1, 55.7, 106.6, 113.5, 116.6 (t, *J* = 170.4 Hz), 118.3, 122.7, 126.1, 128.4, 129.1, 131.2, 136.8, 137.7, 139.2. MS (ESI, *m*/*z):* 458.2 [M+H]⁺.

### EXAMPLE 33. (2R,3R,4R)-6-Chloro-4-(2-{[(3,3-difluorocyclobutyl)methyl]amino}ethyl)-N-methyl-2-(4-methylphenyl)-2,3,4,9-tetrahydro-1H-carbazol-3-amine (Iac).

Obtained following general procedure described in Example 2. [α]_{D}²⁰ = +5 (c 1.00 g/100 mL, methanol). IR (ATR): v (cm⁻¹) 3229, 2951. ¹H-NMR (500 MHz, methanol-*d*₄): δ (ppm) 1.96 - 2.02 (m, 1H, CH), 2.32 (s, 3H, CH₃), 2.26 - 2.50 (m, 4H, 2CH₂), 2.70 - 2.98 (m, 5H, ½CH₂, 2CH₂), 2.85 (s, 3H, CH₃), 3.15-3.21 (m, 2H, ½CH₂, ½CH₂), 3.37 (m, 1H, ½CH₂), 3.51 - 3.56 (m, 1H, CH), 3.73 - 3.77 (m, 1H, CH), 3.96 - 4.02 (m, 1H, CH), 7.11 (dd, *J* = 8.7, 1.8 Hz, 1H, CH_{Ar}), 7.20 (d, *J* = 7.4 Hz, 2H, 2CH_{Ar}), 7.30 (d, *J =* 7.9 Hz, 2H, 2CH_{Ar}), 7.34 (d, *J* = 8.6 Hz, 1H, CH_{Ar}), 7.56 (d, *J* = 2.0 Hz, 1H, CH_{Ar}). ¹³C-NMR (125 MHz, methanol-*d*₄): δ (ppm) 21.1, 21.6, 25.2, 29.5, 32.0, 34.6, 39.8, 39.9 (t, *J* = 23.6 Hz), 46.6, 52.9, 62.9, 107.1, 113.5, 115.9 (t, *J* = 170.4 Hz), 118.3, 122.9, 126.1, 128.8, 129.1, 130.9, 135.2, 136.8, 137.8, 139.0. MS (ESI, *m*/*z*): 472.2 [M+H]⁺.

### EXAMPLE 34. 2-[(2R,3R,4R)-3-Amino-2-(4-methylphenyl)-2,3,4,9-tetrahydro-1H-carbazol-4-yl]ethan-1-ol (Iad).

Obtained following general procedure described in Example 3. [α]_{D}²⁰ = +17 (c 1.00 g/100 mL, methanol). IR (ATR): v (cm⁻¹) 3393, 3247, 1459. ¹H-NMR (300 MHz, methanol-*d*₄): δ (ppm) 1.81-1.99 (m, 1H, ½CH₂), 2.35 (s, 3H, CH₃), 2.61 (d, *J* = 15.7 Hz, 1H, ½CH₂), 2.99 (d, *J* = 11.4 Hz, 1H, ½CH₂), 3.12-3.26 (m, 3H, ½CH₂, 2CH), 3.71-3.91 (m, 3H, CH, CH₂), 6.99 (t, *J* = 7.4 Hz, 1H, CH_{Ar}), 7.07 (t, *J* = 7.5 Hz, 1H, CH_{Ar}), 7.20-7.37 (m, 5H, 5CH_{Ar}), 7.42 (d, *J* = 7.7 Hz, 1H, CH_{Ar}). ¹³C NMR (75 MHz, methanol-*d*₄) δ (ppm) 21.1, 31.0, 37.7, 40.3, 46.0, 59.9, 61.5, 109.7, 112.1, 119.1, 119.9, 122.1, 127.4, 129.0, 131.1, 134.0, 138.0, 138.4, 139.1. MS (ESI, *m*/*z):* 321.2 [M+H]⁺.

### EXAMPLE 35. (2R,3R,4R)-4-(2-Methoxyethyl)-2-(4-methoxyphenyl)-2,3,4,9-tetrahydro-1H-carbazol-3-amine (Iae).

Obtained following general procedure described in Example 4. [α]_{D}²⁰ = +18 (c 1.00 g/100 mL, methanol). IR (ATR): v (cm⁻¹) 3247, 2924. ¹H-NMR (700 MHz, methanol-*d*₄): δ (ppm) 2.04-2.10 (m, 1H, ½CH₂), 2.47-2.51 (m, 1H, ½CH₂), 3.02 (dd, *J* = 16.3, 5.2 Hz, 1H, ½CH₂), 3.17 (ddd, *J* = 16.4, 10.2, 1.9 Hz, 1H, ½CH₂), 3.23 (td, *J* = 10.2, 5.0 Hz, 1H, CH), 3.30-3.33 (m, 1H, CH), 3.37 (s, 3H, CH₃), 3.55-3.61 (m, 2H, CH₂), 3.82 (s, 3H, CH₃), 3.82-3.85 (m, 1H, CH), 6.98-7.02 (m, 3H, 3CH_{Ar}), 7.09 (ddd, *J* = 8.1, 7.1, 1.1 Hz, 1H, CH_{Ar}), 7.31 (d, *J* = 8.1 Hz, 1H, CH_{Ar}), 7.34 (d, *J* = 8.7 Hz, 2H, 2CH_{Ar}), 7.44 (d, *J* = 7.9 Hz, 1H, CH_{Ar}).¹³C-NMR (175 MHz, methanol-*d*₄): δ (ppm) 30.7, 34.1, 39.6, 45.3, 55.8, 59.1, 59.4, 72.2, 109.1, 112.2, 115.8, 119.1, 120.0, 122.2, 127.5, 130.2, 132.9, 134.2, 138.4, 161.0. MS (ESI, *m*/*z):* 351.2 [M+H]⁺.

### EXAMPLE 36. (2R,3R,4R)-4-{2-[(Cyclopropylmethyl)amino]ethyl}-6-methoxy-2-(4-methylphenyl)-2,3,4,9-tetrahydro-1H-carbazol-3-amine (Iaf).

Obtained following general procedure described in Example 1. [α]_{D}²⁰ = +26 (c 1.00 g/100 mL, methanol). IR (ATR): v (cm⁻¹) 3387, 1455. ¹H-NMR (300 MHz, methanol-*d*₄): δ (ppm) 0.33 (dd, *J* = 9.5, 4.5 Hz, 2H, CH₂), 0.62 (d, *J =* 7.8 Hz, 2H, CH₂), 0.95-1.06 (m, 1H, CH), 2.22-2.32 (m, 2H, CH₂), 2.35 (s, 3H, CH₃), 2.65-2.79 (m, 3H, ½CH₂, CH₂), 3.01-3.25 (m, 3H, ½CH₂, CH₂), 3.35-3.40 (m, 1H, CH), 3.42-3.52 (m, 1H, CH), 3.84 (s, 3H, OCH₃), 3.95 (t, *J* = 7.2 Hz, 1H, CH), 6.79 (dd, *J* = 8.8, 1.5 Hz, 1H, CH_{Ar}), 7.05 (d, *J* = 2.2 Hz, 1H, CH_{Ar}), 7.22-7.26 (m, 3H, 3CH_{Ar}), 7.35 (d, *J* = 7.8 Hz, 2H, 2CH_{Ar}). ¹³C-NMR (75 MHz, methanol-*d*₄): δ (ppm) 4.5, 4.7, 8.2, 21.1, 27.8, 29.2, 37.5, 44.7, 45.2, 53.7, 56.1, 56.5, 102.0, 106.5, 111.8, 112.9, 127.7, 129.1, 131.1, 133.5, 135.5, 138.0, 139.1, 155.4. MS (ESI, *m*/*z*): 404.3 [M+H]⁺.

### EXAMPLE 37. (2R,3R,4R)-6-Chloro-4-{2-[(cyclopropylmethyl)amino]ethyl}-2-(4-methylphenyl)-2,3,4,9-tetrahydro-1H-carbazol-3-amine (Iag).

Obtained following general procedure described in Example 1. [α]_{D}²⁰ = +19 (c 1.00 g/100 mL, methanol). IR (ATR): v (cm⁻¹) 3383, 1454. ¹H-NMR (300 MHz, methanol-*d*₄): δ (ppm) 0.19-0.46 (m, 2H, CH₂), 0.59-0.66 (m, 2H, CH₂), 0.89-1.09 (m, 1H, CH), 2.20-2.30 (m, 2H, CH₂), 2.34 (s, 3H, CH₃), 2.53-2.88 (m, 3H, ½CH₂, CH₂), 2.99-3.24 (m, 3H, ½CH₂, CH₂), 3.38-3.57 (m, 2H, 2CH), 3.86-4.09 (m, 1H, CH), 7.07 (d, *J* = 8.1 Hz, 1H, CH_{Ar}), 7.20-7.40 (m, 5H, 5CH_{Ar}), 7.56 (s, 1H, CH_{Ar}). ¹³C-NMR (75 MHz, methanol-*d*₄): δ (ppm) 4.7, 4.9, 8.2, 21.2, 28.0, 29.1, 37.2, 44.4, 45.8, 54.0, 56.1, 106.7, 113.5, 118.3, 122.6, 126.1, 128.4, 129.2, 131.2, 136.6, 136.7, 137.7, 139.1. MS (ESI, *m*/*z*): 408.2 [M+H]⁺.

### EXAMPLE 38. (2R,3R,4R)-6-Chloro-4-{2-[(cyclopropylmethyl)amino]ethyl}-2-(4-methoxyphenyl)-2,3,4,9-tetrahydro-1H-carbazol-3-amine (Iah).

Obtained following general procedure described in Example 1. [α]_{D}²⁰ = +50 (c 1.00 g/100 mL, methanol). IR (ATR): v (cm⁻¹) 3222, 1456. ¹H-NMR (300 MHz, methanol-*d*₄): δ (ppm) 0.26-0.42 (m, 2H, CH₂), 0.63 (d, *J =* 7.7 Hz, 2H, CH₂), 0.93-1.08 (m, 1H, CH), 2.67-2.33 (m, 2H, CH₂), 2.58-2.83 (m, 3H, ½CH₂, CH₂), 2.99-3.28 (m, 3H, ½CH₂, CH₂), 3.33-3.41 (m, 1H, CH), 3.42-3.55 (m, 1H, CH), 3.80 (s, 3H, OCH₃), 3.96 (t, *J* = 7.4 Hz, 1H, CH), 6.97 (d, *J* = 7.7 Hz, 2H, 2CH_{Ar}), 7.09 (dd, *J* = 8.6, 1.9 Hz, 1H, CH_{Ar}), 7.31 (d, *J* = 8.6 Hz, 1H, CH_{Ar}), 7.37 (d, *J* = 8.2 Hz, 2H, 2CH_{Ar}), 7.58 (d, *J =* 1.9 Hz, 1H, CH_{Ar}). ¹³C-NMR (75 MHz, methanol-*d*₄): δ (ppm) 4.5, 4.6, 8.2, 27.9, 28.9, 37.2, 44.0, 45.1, 53.8, 55.8, 55.9, 106.7, 113.5, 115.8, 118.3, 122.7, 126.1, 128.5, 130.3, 132.5, 136.6, 136.7, 161.0. MS (ESI, *m*/*z*): 424.2 [M+H]⁺.

### EXAMPLE 39. (2R,3R,4R)-4-{2-[(Cyclopropylmethyl)amino]ethyl}-6-methyl-2-(4-methylphenyl)-2,3,4,9-tetrahydro-1H-carbazol-3-amine (Iai).

Obtained following general procedure described in Example 1. [α]_{D}²⁰ = +11 (c 1.00 g/100 mL, methanol). IR (ATR): v (cm⁻¹) 3237, 2925. ¹H-NMR (300 MHz, methanol-*d*₄): δ (ppm) 0.23-0.44 (m, 2H, CH₂), 0.54-0.71 (m, 2H, CH₂), 0.93-1.06 (m, 1H, CH), 2.22-2.36 (m, 2H, CH₂), 2.35 (s, 3H, CH₃), 2.43 (s, 3H, CH₃), 2.61-2.83 (m, 3H, CH₂, ½CH₂), 3.01-3.25 (m, 3H, ½ CH₂, CH₂), 3.33-3.40 (m, 1H, CH), 3.46-3.52 (m, 1H, CH), 3.97 (dd, *J* = 9.1, 6.7 Hz, 1H, CH), 6.96 (d, *J* = 8.8 Hz, 1H, CH_{Ar}), 7.21-7.26 (m, 3H, 3CH_{Ar}), 7.35 (app d, *J* = 8.0 Hz, 3H, 3CH_{Ar}). ¹³C-NMR (75 MHz, methanol-*d*₄): δ (ppm) 4.5, 4.6, 8.2, 21.1, 21.7, 27.9, 29.2, 37.6, 44.8, 45.1, 53.7, 56.1, 106.2, 112.0, 118.6, 124.1, 127.6, 129.1, 129.6, 131.1, 134.7, 136.7, 138.0, 139.1. MS (ESI, *m*/*z*): 388.2 [M+H]⁺.

### EXAMPLE 40. (2R,3R,4R)-4-{2-[(Cyclopropylmethyl)amino]ethyl}-6-fluoro-2-(4-methylphenyl)-2,3,4,9-tetrahydro-1H-carbazol-3-amine (Iaj).

Obtained following general procedure described in Example 1. [α]_{D}²⁰ = +3 (c 1.00 g/100 mL, methanol). IR (ATR): v (cm⁻¹) 3193, 2925. ¹H-NMR (300 MHz, methanol-*d*₄): δ (ppm) 0.34 (dd, *J* = 9.8, 4.4 Hz, 2H, CH₂), 0.63 (d, *J =* 7.8 Hz, 2H, CH₂), 0.95-1.07 (m, 1H, CH), 2.22-2.31 (m, 2H, CH₂), 2.35 (s, 3H, CH₃), 2.64-2.83 (m, 3H, CH₂, ½CH₂), 3.02-3.22 (m, 3H, ½CH₂, CH₂), 3.35-3.40 (m, 1H, CH), 3.45-3.54 (m, 1H, CH), 3.99 (t, *J* = 8.0 Hz, 1H, CH), 6.89 (td, *J* = 9.2, 2.4 Hz, 1H, CH_{Ar}), 7.24-7.36 (m, 6H, 6CH_{Ar}). ¹³C-NMR (75 MHz, methanol-*d*₄): δ (ppm) 4.5, 4.7, 8.2, 21.1, 27.7, 29.2, 37.4, 44.6, 45.1, 53.8, 55.9, 103.94 (d, *J* = 24.1 Hz), 107.0 (d, *J* = 4.6 Hz), 110.3 (d, *J* = 26.0 Hz), 113.0 (d, *J* = 10.0 Hz), 127.6 (d, *J* = 9.6 Hz), 129.1, 131.1, 134.9, 136.9, 137.8, 139.2, 159.1 (d, *J* = 233.0 Hz). MS (ESI, *m*/*z):* 392.2 [M+H]⁺.

### EXAMPLE 41. (2R,3R,4R)-6-Chloro-4-{2-[(cyclopropylmethyl)amino]ethyl}-N-methyl-2-(4-methylphenyl)-2,3,4,9-tetrahydro-1H-carbazol-3-amine (Iak).

Obtained following general procedure described in Example 2. [α]_{D}²⁰ = +1 (c 1.00 g/100 mL, methanol). IR (ATR): v (cm⁻¹) 3382, 2953. ¹H-NMR (300 MHz, methanol-*d*₄): δ (ppm) 0.29-0.39 (m, 2H, CH₂), 0.65 (dd, *J* = 8.1, 1.8 Hz, 2H, CH₂), 0.96-1.03 (m, 1H, CH), 1.45-1.54 (m, 1H, ½CH₂), 1.95-2.04 (m, 1H, ½CH₂), 2.31 (s, 3H, CH₃), 2.55-2.72 (m, 2H, CH₂), 2.85 (s, 4H, CH₃, ½CH₂), 3.10-3.23 (m, 2H, ½CH₂, ½CH₂), 3.33-3.39 (m, 1H, ½CH₂), 3.52-3.57 (m, 1H, CH), 3.76-3.81 (m, 1H, CH), 4.01 (br s, 1H, CH), 7.10 (dd, *J* = 8.6, 1.8 Hz, 1H, CH_{Ar}), 7.19 (d, *J* = 7.5 Hz, 2H, 2CH_{Ar}), 7.31 (d, *J* = 7.5 Hz, 2H, 2CH_{Ar}), 7.34 (d, *J* = 8.6 Hz, 1H, CH_{Ar}), 7.57 (d, *J* = 1.8 Hz, 1H, CH_{Ar}). ¹³C-NMR (75 MHz, methanol-*d*₄): δ (ppm) 4.6, 4.7, 8.1, 21.1, 25.1, 29.5, 32.0, 34.6, 39.8, 45.9, 53.6, 62.9, 107.3, 113.5, 118.3, 122.8, 126.1, 128.8, 129.1, 130.9, 135.1, 136.8, 137.9, 138.9. MS (ESI, *m*/*z):* 422.0 [M+H]⁺.

### EXAMPLE 42. (2R,3R,4R)-4-{2-[(Cyclopropylmethyl)amino]ethyl}-9-methyl-2-(4-methylphenyl)-2,3,4,9-tetrahydro-1H-carbazol-3-amine (Ial).

Obtained following general procedure described in Example 1. [α]_{D}²⁰ = +13 (c 1.00 g/100 mL, methanol). ¹H-NMR (300 MHz, methanol-*d*₄): δ (ppm) 0.18 (d, *J* = 4.4 Hz, 2H, CH₂), 0.53 (dd, *J* = 8.1, 1.5 Hz, 2H, CH₂), 0.88-0.97 (m, 1H, CH), 2.12-2.19 (m, 1H, ½CH₂), 2.28-2.31 (m, 1H, ½CH₂), 2.36 (s, 3H, CH₃), 2.57 (dd, *J* = 7.2, 1.7 Hz, 2H, CH₂), 2.72-2.81 (m, 1H, ½CH₂), 2.87-3.01 (m, 4H, ½CH₂,CH, CH₂), 3.07-3.18 (m, 2H, 2CH), 3.60 (s, 3H, CH₃), 7.00-7.05 (m, 1H, CH_{Ar}), 7.12 (ddd, *J* = 8.2, 7.1, 1.1 Hz, 1H, CH_{Ar}), 7.22-7.32 (m, 5H, 5CH_{Ar}), 7.57 (d, *J* = 7.8 Hz, 1H, CH_{Ar}). ¹³C-NMR (75 MHz, methanol-*d*₄): δ (ppm) 4.2, 9.7, 21.1, 29.3, 30.8, 32.1, 42.8, 45.0, 47.0, 54.1, 58.0, 110.0, 110.4, 119.6, 119.9, 121.8, 127.4, 129.2, 130.7, 136.6, 138.1, 139.1, 140.9. MS (ESI, *m*/*z):* 388.3 [M+H]⁺.

### EXAMPLE 43. Viability assays in OCI-AML3 (NPM1 C+) and MOLM13 (NPM1 C-) cell lines

.. Two different acute myeloid leukemia cell lines, OCI-AML3 (NPM1 C+) and MOLM13 (NPM1 C-) were obtained from the DSMZ cell repository (Braunschweig, Germany). Cell lines were expanded and cryogenically frozen upon acquisition to establish stocks in liquid nitrogen until use. Cultured cells were maintained at 37 °C in a humidified incubator in an atmosphere of 5% CO₂ and passaged every 2-3 days. RPMI media (Gibco/Life Technologies; Grand Island, NY) supplemented with 10% fetal bovine serum (FBS) (Hyclone) and 1% penicillin/streptomycin was used.

.. The sensitivity of the compounds on OCI-AML3 (NPM1 C+) and MOLM13 (NPM1 C-) cell viability was assessed as an in-vitro acute myeloid leukemia (AML) model. Briefly, cells were seeded at a density of 2×10⁴ cells/100 µL/well in 96-well plates. Cell lines were treated for 24 h with the test compound diluted in DMSO at the concentration of 5 µM or vehicle (DMSO). Then, 10 µL of WST-1 solution ((2-(4-iodophenyl)-3-(4-nitrophenyl)-5-(2,4-disulfophenyl)-2H-tetrazolium monosodium salt, Merck) were added and the cells were incubated for 4 h at 37 °C. The absorbance was measured at 450 nm using an Asys UVM 340 microplate reader (Biochrom Ltd., Cambridge, UK). The background absorbance of blank wells containing only medium was subtracted from each test well. The data were reported as viability percentage relative to vehicle, obtained from two or three independent experiments performed in triplicate (Table 1).

.. The results obtained from these viability assays show that novel compounds (I) have efficacy against both AML cell lines in µM range, especially against NPM1 mutant cell line.

**Table 1. Viability of novel compounds (I) in OCI-AML3 (NPM1 C+) and MOLM13 (NPM1 C-) cell lines**

| **Compd** | **OCI-AML3 (%)** | **MOLM13 (%)** |
|---|---|---|
| **lb** | 16±0 | 20±5 |
| **lc** | 40±2 | 55±6 |
| **ld** | 33±5 | 40±0 |
| **le** | 14±5 | 13±3 |
| **lf** | 11±4 | 12±3 |
| **lh** | 25±7 | 28±10 |
| **li** | 13±4 | 13±1 |
| **lk** | 23±3 | 30±2 |
| **ll** | 19±7 | 32±4 |
| **lm** | 36±8 | 61±7 |
| **ln** | 29±1 | 28±4 |
| **lo** | 16±1 | 13±5 |
| **lp** | 7±3 | 7±2 |
| **lq** | 18±6 | 34±4 |
| **lr** | 11±3 | 15±0 |
| **ls** | 1±0 | 6±1 |
| **lu** | 4±3 | 6±1 |
| **lw** | 27±2 | 40±15 |
| **lx** | 0±0 | 0±0 |
| **ly** | 8±0 | 4±4 |
| **lz** | 8±4 | 8±3 |
| **laa** | 1±1 | 2±2 |
| **lab** | 17±4 | 14±8 |
| **lac** | 6±0 | 5±1 |
| **laf** | 39±6 | 60±2 |
| **lag** | 9±5 | 10±6 |
| **lah** | 26±12 | 16±2 |
| **lai** | 12±2 | 6±2 |
| **laj** | 7±1 | 4±1 |
| **lak** | 1±1 | 7±2 |
| **lal** | 22±2 | 17±0 |

### EXAMPLE 44. Viability assays in primary CD34+ cells from AML patients

.. The sensitivity of compounds (**I**) on primary CD34+ cells from AML patients as a short-term ex-vivo AML model.

.. Primary cells from AML bone marrow patients CD34+ were isolated by anti-CD34+ microbeads (Miltenyi Biotech) from bone marrow of healthy donors and AML patients. Briefly, bone marrow samples were collected from AML and healthy patient pelvic bone, and were subjected to Ficoll-Paque density gradient separation to isolate mononuclear cells. Then, CD34⁺ cells were separated using high-gradient magnetic-activated cell sorting according to the manufacturer's manual, and maintained in DMEM medium supplemented with 10% FBS until their use.

.. Sorted CD34⁺ cells were plated in 96-well plates at a density of 5 x 10⁴ per well. Then, the assay was performed as described in Example 43.

.. The results obtained from these viability assays show that compound **Ic** exhibited higher efficacy against AML primary cells than healthy donor cells (IC₅₀ AML NPM1+= 1.24 µM; IC₅₀ AML NPM1 WT= 5.03 µM IC₅₀ healthy donors = 7.89 µM, p-value: 0.0003, Figure 2)

### EXAMPLE 45. Colony formation unit assays in primary CD34+ cells from AML patients

.. The sensitivity of compounds (I) on primary CD34+ cells from AML patients as a mid-term ex-vivo AML model.

.. Sorted CD34⁺ cells were plated in 24-well plates at a density of 3 × 10⁴ cells per well in methylcellulose-based medium MethoCult GF H4434 (StemCell Technologies) containing stem cell factor (SCF), interleukin-3 (IL-3), erythropoietin (EPO) and granulocyte macrophage colony stimulating factor (GM-CSF). Test compound was added directly to the cells within the methylcellulose medium, and incubated at 37 °C in a humidified atmosphere in the presence of 5% CO₂ for 14 days. Total cells from colonies formed were counted manually using an inverted microscope/phase contrast microscopy with trypan-blue. The data were presented as percentage of colonies or percentage of viable cells relative to DMSO, obtained from two or three independent experiments performed in duplicate.

.. The results obtained from these viability assays show that compound **Ic** induced a decrease in viability (CFU-E: IC₅₀ AML = 1.255 µM; IC₅₀ healthy donors = 4.596 µM; p-value: 0.0209; Figure 3).

### EXAMPLE 46. Effect on the NPM1 cytoplasmic form (NPM1 C+) of the protein using confocal microscopy

.. OCI-AML3 (NPM1 C+) cells were plated onto glass coverslips or Greiner Bio-One µClear black 96-well plate (7.5 × 104 cells per well) and treated with test compound for 48 h. Then, cells were fixed with 4% paraformaldehyde for 15 min, permeabilized with 0.1% Triton X-100 for 30 min, and blocked in 5% bovine serum albumin during 1 h at rt. Incubation with primary antibodies against NPM1 (7H10B9, NOVUS Biologicals) was carried out overnight at 4 °C. Alexa-488 conjugated antimouse or anti-rabbit antibodies (Molecular Probes) were used for detection of the reported proteins by confocal microscopy. Nuclei were counterstained with 4',6-diamidino-2-phenylindole (DAPI) bath. A total of 35 fields per well were acquired with a ×40 magnification lens for those samples run in the Opera HCS system (Perkin Elmer). Images were segmented using the DAPI staining and the coefficient of variation of NPM1 intensity per nucleus/cytoplasm was calculated using Definiens Developer XD software.

.. As shown in Figure 4, the inhibition of the NPM1 cytoplasmic form (NPM1 C+) of the protein, typically associated to NPM1 mutation, was visualized, which confirms the effect of compound Ic in NPM1 biology.

### EXAMPLE 47. Animal models

.. Pharmacokinetic studies for compound Ic were performed. After injecting 25 mg/kg dose of the compound, samples of blood are withdrawn at different times (30, 60, 120, 240, 360 min), and the presence of tested compound is analysed by HPLC-MS. The area of the molecular peak allows to calculate the concentration of the compound present in mice serum at each time.

.. An AML xenograft mouse model was used for the study of *in vivo* efficacy. Female 6-8 week-old NOD.Cg-Prkdcscid Il2rgtm1Wjl/SzJ (NSG) mice (Vivotecnia, Madrid, Spain) were inoculated intravenously by tail vein injection with OCI-AML3-ffLuc-GFP cells (1×10⁶). The animal experiment was performed according to the guidelines of the Institutional Animal Care and Use Committees of the Comunidad de Madrid, Spain, under approved protocol (PROEX 023/17). Four days later, mice were randomized into three groups of 10 animals: the first group received intraperitoneal injections of vehicle (0.9% saline solution) five days a week; the second group received 25 mg/kg of compound **Ic** intraperitoneally five times a week for four weeks; the third group received intraperitoneal injections of 50 mg/kg of compound **Ic** five times weekly for three weeks. Mice were monitored for tumor burden, distribution and engraftment every week by IVIS whole-body bioluminescence imaging. Bioimaging of MM burden in vivo was performed by IVIS Imaging system (Caliper Life Science). Before performing, Luciferin (D-luciferin monosodium salt, Thermofisher) was prepared in PBS and 150 mg/kg of Luciferin was injected i.p.; after 10 min, luminescence signal can be detected. The signal data was analyzed by the Living Image 4.2 (Caliper Life Science) software.

.. The correct *in-vivo* diffusion of compound **Ic** was confirmed in the model. 25 mg/kg via intraperitoneal showed levels between 200-600 µg/L in mouse peripheral blood during the first 6 h post-injection (Figure 5A).

.. Tumour infiltration decreased was observed in treated group compared with vehicle at same time of sacrifice (Figure 5B).

## Claims

1. A compound of formula (**I**), a pharmaceutically acceptable salt thereof, or any enantiomer or mixtures of enantiomer, either of the compound of formula (**I**) or of any of its pharmaceutically acceptable salts 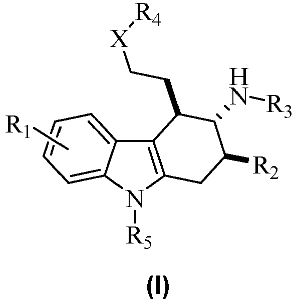 wherein:
in formula (**I**) the "bold bonds" and "hashed bonds" refer to the relative stereochemistry of the chiral centers;
X is NH or O;
R₁ is selected from the group consisting of H, -(C₁-C₃)alkyl, -O(C₁-C₃)alkyl, halogen, -CF₃, and -OCF₃;
R₂ is phenyl optionally substituted with one group selected from -(C₁-C₃)alkyl, -O-(C₁-C₃)alkyl, halogen, -CF₃, -OCF₃, -(C₃-C₆)cycloalkyl, -NH-(C₁-C₃)alkyl, -NH-CO-(C₁-C₃)alkyl, and -O(C₃-C₆)cycloalkyl;
R₃ is selected from the group consisting of H, -(C₁-C₄)alkyl, -(C₃-C₆)cycloalkyl, -CO-(C₁-C₆)alkyl, and -CO-(C₃-C₆)cycloalkyl;
R₄ is selected from the group consisting of H, -(C₁-C₄)alkyl, -(C₃-C₆)cycloalkyl, -(CH₂)-(C₃-C₆)cycloalkyl, -(CH₂CH₂)-(C₃-C₆)C₃-C₆cycloalkyl;
and wherein R₄ is optionally substituted with halogen;
and R₅ is selected from the group consisting of H, -CH₃, and -COCH₃.

2. The compound of formula (**I**) according to claim 1, wherein
R₂ is phenyl, optionally substituted with methyl, ethyl, -OCH₃, -OCH₂CH₃, -OCH(CH₃)₂, F, Cl, Br, -CF₃, -OCF₃, -(C₃-C₆)cycloalkyl, -NHCH₃, -NHCOCH₃ , and -O(C₃-C₆)cycloalkyl;
R₄ is selected from the group consisting of H, methyl, ethyl, propyl, isopropyl, isobutyl, cyclopropyl, cyclobutyl, cyclopentyl, cyclohexyl, and 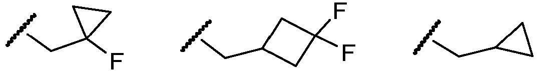 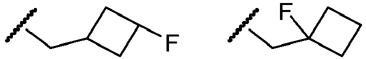
wherein the wavy line represents the attaching point.

3. The compound of formula (**I**) according to any of the claims 1-2, wherein
R₁ is selected from the group consisting of H, methyl, ethyl, -OCH₃, -OCH₂CH₃; F, Cl, Br, -CF₃, and -OCF₃;
R₂ is phenyl optionally substituted with methyl, ethyl, -OCH₃, -OCH₂CH₃, -OCH(CH₃)₂, F, Cl, Br, -CF₃, -OCF₃, -NHCH₃, and -NHCOCH₃;
R₃ is selected from the group consisting of H, methyl, ethyl, -COCH₃, and cyclopropyl;
R₄ is selected from the group consisting of H, methyl, ethyl, propyl, isopropyl, isobutyl, cyclopropyl, cyclobutyl, and 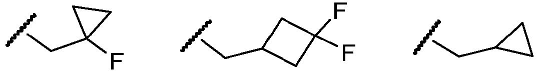 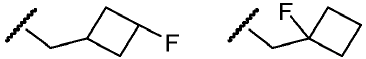
wherein the wavy line represents the attaching point.

4. The compound of formula (**I**) according to any of the claims 1-3, wherein
R₁ is selected from the group consisting of H, methyl, ethyl, -OCH₃, -OCH₂CH₃, F, Cl, Br, -CF₃, and -OCF₃;
R₂ is phenyl optionally substituted with methyl, ethyl, -OCH₃, -OCH₂CH₃, F, Cl, -CF₃, and -OCF₃ ;
R₃ is selected from the group consisting of H and methyl;
R₄ is selected from the group consisting of H, methyl, ethyl, propyl, isopropyl, isobutyl, cyclopropyl, cyclobutyl, and 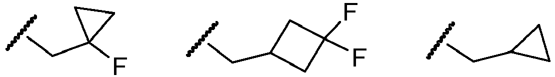 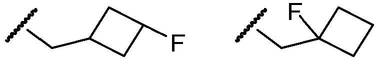
wherein the wavy line represents the attaching point.

5. The compound of formula (**I**) according to any of the claims 1-4, wherein
X is NH.
R₁ is selected from the group consisting of H, F, Cl, methyl, and -OCH₃;
R₂ is phenyl optionally substituted with methyl, ethyl, -OCH₃, -OCH₂CH₃, F, Cl, -CF₃, and -OCF₃ ;
R₃ is selected from the group consisting of H and methyl;
R₄ is selected from the group consisting of H, methyl, ethyl, propyl, isopropyl, isobutyl, cyclopropyl, cyclobutyl, and 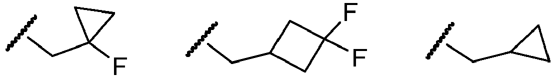 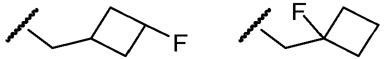
wherein the wavy line represents the attaching point;
and R₅ is selected from the group consisting of H, CH₃ and -COCH₃.

6. The compound of formula (**I**) according to any of the claims 1-5, wherein
X is NH;
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃;
R₃ is selected from the group consisting of H and methyl;
R₄ is selected from the group consisting of 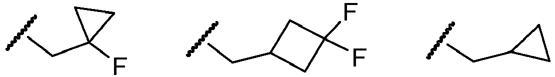 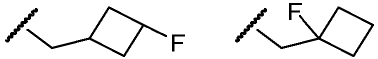
wherein the wavy line represents the attaching point;
and R₅ is selected from the group consisting of H, CH₃ and -COCH₃.

7. The compound of formula (**I**) according to any of the claims 1-6, wherein
X is O;
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃;
R₃ is selected from the group consisting of H and methyl;
R₄ is selected from the group consisting of H, methyl, ethyl, propyl, isopropyl, isobutyl, cyclopropyl, cyclobutyl, and 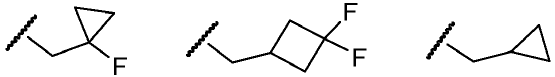 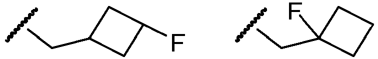
wherein the wavy line represents the attaching point;
and R₅ is selected from the group consisting of H, CH₃ and -COCH₃.

8. The compound of formula (**I**) according to any of the claims 1-7, wherein
X is O;
R₁ is selected from the group consisting of H, F, Cl, methyl, and -OCH₃;
R₂ is phenyl optionally substituted with methyl, ethyl; -OCH₃, -OCH₂CH₃, F, Cl, -CF₃, and -OCF₃ ;
R₃ is selected from the group consisting of H and methyl;
R₄ is selected from the group consisting of 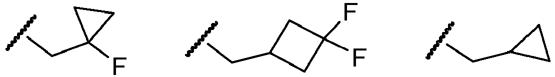 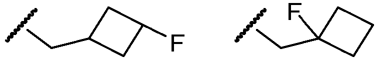
wherein the wavy line represents the attaching point;
and R₅ is selected from the group consisting of H, CH₃ and -COCH₃.

9. The compound of formula (**I**) according to any of the claims 1-8, which is selected from
(2*R*,3*R*,4*R*)-4-{2-[(Cyclopropylmethyl)amino]ethyl}-2-phenyl-2,3,4,9-tetrahydro-1*H-*carbazol-3-amine (**Ia**);
(2*R*,3*R*,4*R*)-4-{2-[(Cyclopropylmethyl)amino]ethyl}-2-(4-methylphenyl)-2,3,4,9-tetrahydro-1*H*-carbazol-3-amine (**Ib**);
(2*R*,3*R*,4*R*)-4-{2-[(Cyclopropylmethyl)amino]ethyl}-2-(4-methoxyphenyl)-2,3,4,9-tetrahydro-1*H*-carbazol-3-amine (**Ic**);
(2*S*,3*S*,4*S*)-4-{2-[(Cyclopropylmethyl)amino]ethyl}-2-(4-methoxyphenyl)-2,3,4,9-tetrahydro-1*H*-carbazol-3-amine (**Id**);
(2*R*,3*R*,4*R*)-4-{2-[(Cyclopropylmethyl)amino]ethyl}-2-[4-(trifluoromethyl)phenyl]-2,3,4,9-tetrahydro-1*H*-carbazol-3-amine (**Ie**);
(2*R*,3*R*,4*R*)-4-{2-[(Cyclopropylmethyl)amino]ethyl}-2-[4-(trifluoromethoxy)phenyl]-2,3,4,9-tetrahydro-1*H*-carbazol-3-amine (**If**);
(2*R*,3*S*,4*R*)-4-{2-[(Cyclopropylmethyl)amino]ethyl}-2-(2-fluorophenyl)-2,3,4,9-tetrahydro-1*H*-carbazol-3-amine (**Ig**);
(2*R*,3*R*,4*R*)-2-(3-Chlorophenyl)-4-{2-[(cyclopropylmethyl) amino]ethyl}-2,3,4,9-tetrahydro-1*H*-carbazol-3-amine (**Ih**);
(2*R*,3*R*,4*R*)-2-(4-Chlorophenyl)-4-{2-[(cyclopropylmethyl)amino]ethyl}-2,3,4,9-tetrahydro-1*H*-carbazol-3-amine (**Ii**);
(2*R*,3*R*,4*R*)-4-{2-[(Cyclopropylmethyl)amino]ethyl}-2-(3-methoxyphenyl)-2,3,4,9-tetrahydro-1*H*-carbazol-3-amine (**Ij**);
(2*R*,3*S*,4*R*)-4-{2-[(Cyclopropylmethyl)amino]ethyl}-2-(2-methoxyphenyl)-2,3,4,9-tetrahydro-1*H*-carbazol-3-amine (**Ik**);
(2*R*,3*R*,4*R*)-4-{2-[(Cyclopropylmethyl)amino]ethyl}-2-(3-methylphenyl)-2,3,4,9-tetrahydro-1*H*-carbazol-3-amine (**Il**);
(2*R*,3*R*,4*R*)-4-{2-[(Cyclopropylmethyl)amino]ethyl}-2-(2-methylphenyl)-2,3,4,9-tetrahydro-1*H*-carbazol-3-amine (**Im**);
(2*R*,3*S*,4*R*)-2-(2-Chlorophenyl)-4-{2-[(cyclopropylmethyl)amino]ethyl}-2,3,4,9-tetrahydro-1*H*-carbazol-3-amine (**In**);
(2*R*,3*R*,4*R*)-4-{2-[(Cyclopropylmethyl)amino]ethyl}-2-(4-fluorophenyl)-2,3,4,9-tetrahydro-1*H*-carbazol-3-amine (**Io**);
(2*S*,3*S*,4*S*)-4-{2-[(Cyclopropylmethyl)amino]ethyl}-2-(4-fluorophenyl)-2,3,4,9-tetrahydro-1*H*-carbazol-3-amine (**Ip**);
(2*R*,3*R*,4*R*)-4-{2-[(Cyclopropylmethyl)amino]ethyl}-2-(3-fluorophenyl)-2,3,4,9-tetrahydro-1*H*-carbazol-3-amine (**Iq**);
(2*R*,3*R*,4*R*)-4-{2-[(Cyclopropylmethyl)amino]ethyl}-2-(4-methoxyphenyl)-N-methyl-2,3,4,9-tetrahydro-1*H*-carbazol-3-amine (**Ir**);
(2*S*,3*S*,4*S*)-4-{2-[(Cyclopropylmethyl)amino]ethyl}-2-(4-methoxyphenyl)-N-methyl-2,3,4,9-tetrahydro-1*H*-carbazol-3-amine (**Is**);
*N*-[(2*R*,3*R*,4*R*)-4-{2-[(Cyclopropylmethyl)amino]ethyl}-2-(4-methoxyphenyl)-2,3,4,9-tetrahydro-1*H*-carbazol-3-yl]acetamide (**It**);
(2*R*,3*R*,4*R*)-4-(2-{[(3,3-Difluorocyclobutyl)methyl]amino}ethyl)-2-(4-methylphenyl)-2,3,4,9-tetrahydro-1*H*-carbazol-3-amine (**Iu**);
(2*R*,3*R*,4*R*)-9-Methyl-2-(4-methylphenyl)-4-(2-{[(oxetan-3-yl)methyl]amino}ethyl)-2,3,4,9-tetrahydro-1*H*-carbazol-3-amine (**Iv**);
(2*R*,3*R*,4*R*)-2-(4-Methoxyphenyl)-4-[2-(methylamino)ethyl]-2,3,4,9-tetrahydro-1*H-*carbazol-3-amine (**Iw**);
(2*R*,3*R*,4*R*)-2-(4-Methylphenyl)-4-{2-[(2-methylpropyl)amino]ethyl}-2,3,4,9-tetrahydro-1*H*-carbazol-3-amine (**Ix**);
(2*R*,3*R*,4*R*)-2-(4-Methylphenyl)-4-{2-[(propan-2-yl)amino]ethyl}-2,3,4,9-tetrahydro-1*H-*carbazol-3-amine (**Iy**);
(2*R*,3*R*,4*R*)-4-[2-(Ethylamino)ethyl]-2-(4-methylphenyl)-2,3,4,9-tetrahydro-1*H-*carbazol-3-amine (**Iz**);
(2*R*,3*R*,4*R*)-4-[2-(Cyclopropylamino)ethyl]-2-(4-methylphenyl)-2,3,4,9-tetrahydro-1*H-*carbazol-3-amine (**Iaa**);
(2*R*,3*R*,4*R*)-6-Chloro-4-(2-{[(3,3-difluorocyclobutyl)methyl]amino}ethyl)-2-(4-methylphenyl)-2,3,4,9-tetrahydro-1*H*-carbazol-3-amine (**Iab**);
(2*R*,3*R*,4*R*)-6-Chloro-4-(2-{[(3,3-difluorocyclobutyl)methyl]amino}ethyl)-*N*-methyl-2-(4-methylphenyl)-2,3,4,9-tetrahydro-1*H*-carbazol-3-amine (**Iac**);
2-[(2*R*,3*R*,4*R*)-3-amino-2-(4-methylphenyl)-2,3,4,9-tetrahydro-1*H*-carbazol-4-yl]ethan-1-ol (**Iad**);
(2*R*,3*R*,4*R*)-4-(2-Methoxyethyl)-2-(4-methoxyphenyl)-2,3,4,9-tetrahydro-1*H*-carbazol-3-amine (**Iae**);
(2*R*,3*R*,4*R*)-4-{2-[(Cyclopropylmethyl)amino]ethyl}-6-methoxy-2-(4-methylphenyl)-2,3,4,9-tetrahydro-1*H*-carbazol-3-amine (**Iaf**);
(2*R*,3*R*,4*R*)-6-Chloro-4-{2-[(cyclopropylmethyl)amino]ethyl}-2-(4-methylphenyl)-2,3,4,9-tetrahydro-1*H*-carbazol-3-amine (**Iag**);
(2*R*,3*R*,4*R*)-6-Chloro-4-{2-[(cyclopropylmethyl)amino]ethyl}-2-(4-methoxyphenyl)-2,3,4,9-tetrahydro-1*H*-carbazol-3-amine (**Iah**);
(2*R*,3*R*,4*R*)-4-{2-[(Cyclopropylmethyl)amino]ethyl}-6-methyl-2-(4-methylphenyl)-2,3,4,9-tetrahydro-1*H*-carbazol-3-amine (**Iai**);
(2*R*,3*R*,4*R*)-4-{2-[(Cyclopropylmethyl)amino]ethyl}-6-fluoro-2-(4-methylphenyl)-2,3,4,9-tetrahydro-1*H*-carbazol-3-amine (**Iaj**);
(2*R*,3*R*,4*R*)-6-Chloro-4-{2-[(cyclopropylmethyl)amino]ethyl}-*N*-methyl-2-(4-methylphenyl)-2,3,4,9-tetrahydro-1*H*-carbazol-3-amine (**Iak**); and
(2*R*,3*R*,4*R*)-4-{2-[(Cyclopropylmethyl)amino]ethyl}-9-methyl-2-(4-methylphenyl)-2,3,4,9-tetrahydro-1*H*-carbazol-3-amine (**Ial**).

10. A pharmaceutical composition which comprises a therapeutically effective amount of a compound of formula (**I**) or a pharmaceutically acceptable salt thereof, or any enantiomer or mixtures of enantiomers, either of the compound of formula (**I**) or of its pharmaceutically acceptable salt as defined in any of the claims 1-9, together with one or more pharmaceutically acceptable excipients or carriers.

11. A compound of formula (**I**) according to any of the claims 1-9 or a pharmaceutical composition according to claim 10, for use as a medicament.

12. A compound of formula (**I**) according to any of the claims 1-9 or a pharmaceutical composition according to claim 10, for use in the treatment and/or prevention of cancer mediated by NPM1 dysregulation.

13. The compound for use according to claim 12, wherein the cancer is selected from the group consisting of mixed lineage leukemia (MLL), MLL-related leukemia, MLL-associated leukemia, MLL-positive leukemia, MLL-induced leukemia, rearranged mixed lineage leukemia (MLL-r), leukemia associated with a MLL rearrangement or a rearrangement of the MLL gene, acute leukemia, chronic leukemia, indolent leukemia, lymphoblastic leukemia, lymphocytic leukemia, myeloid leukemia, myelogenous leukemia, childhood leukemia, acute lymphocytic leukemia (ALL), acute myeloid leukemia (AML), anaplastic large cell lymphoma (ALCL), acute promyelocytic leukemia (APL), acute granulocytic leukemia, acute nonlymphocytic leukemia, chronic lymphocytic leukemia (CLL), chronic myelogenous leukemia (CML), therapy related leukemia, myelodysplastic syndrome (MDS), myeloproliferative disease (MPD), myeloproliferative neoplasia (MPN), plasma cell neoplasm, multiple myeloma, myelodysplasia, cutaneous T-cell lymphoma, lymphoid neoplasm, AIDS-related lymphoma, thymoma, thymic carcinoma, mycosis fungoides, Alibert-Bazin syndrome, granuloma fungoides, Sézary Syndrome, hairy cell leukemia, T-cell prolymphocytic leukemia (T-PLL), large granular lymphocytic leukemia, meningeal leukemia, leukemic leptomeningitis, leukemic meningitis, multiple myeloma, Hodgkin's lymphoma, non Hodgkin's lymphoma (malignant lymphoma), or Waldenstrom's macroglobulinemia in a mammal in need thereof.

14. The compound for use according to any of claims 12-13 wherein the cancer is selected from acute myeloid leukemia (AML), anaplastic large cell lymphoma (ALCL), and acute promyelocytic leukemia (APL).

15. A process for the preparation of the compound of formula (I) or a pharmaceutically acceptable salt thereof, or any enantiomer or mixtures of enantiomers, either of the compound of formula (I) or of its pharmaceutically acceptable salt, as defined in any of the claims 1-9, which comprises:
a) reductive amination of intermediate of formula (3) with an amine of formula R₄NH₂ to afford an intermediate of formula (4) wherein R₁, R₂ and R₄ are as defined in claim 1, and Y is methyl, acetyl or a Protecting group; 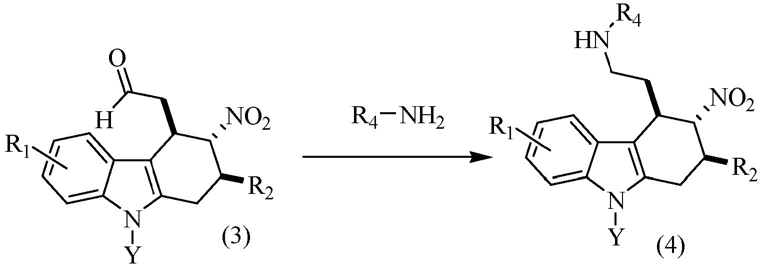
b) subsequent reduction of the nitro group of the intermediate (4), optionally followed by removal of the Protecting-group, resulting in final compounds of formula (**I**) wherein X is NH, R₃ is H, and R₁, R₂, R₄ and R₅ are as defined in claim 1;
or alternatively
a') reductive amination of intermediate of formula (3) with an amine of formula R₄NH₂ to afford an intermediate of formula (4) wherein R₁, R₂ and R₄ are as defined in claim 1, and Y is methyl, acetyl or a Protecting group; 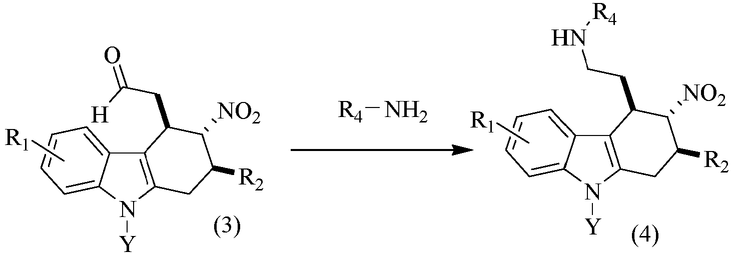
b') reduction of the nitro group preceded or followed by *N*-protection to afford intermediate of formula (5); 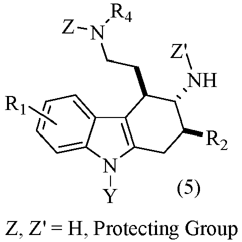
c') N-alkylation or N-acylation of intermediate (5) to afford intermediate (6); 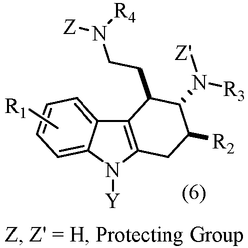
d') final deprotection provides compounds of formula (**I**) wherein X is NH, R₃ is different from H, and R₁, R₂, R₄ and R₅ are as defined in claim 1;
or
a") reduction of the nitro group of intermediate of formula (7) followed by optional *N*-alkylation or *N*-acylation and deprotection afford the compounds of formula (**I**) wherein X is O, R₄ is H, and R₁, R₂, R₃ and R₅ are as defined in claim 1; 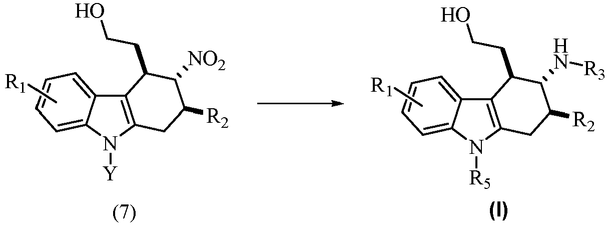 or alternatively
a‴) reduction of the nitro group and in-situ *N*-protection of intermediate of formula (7) provide intermediate of formula (8); 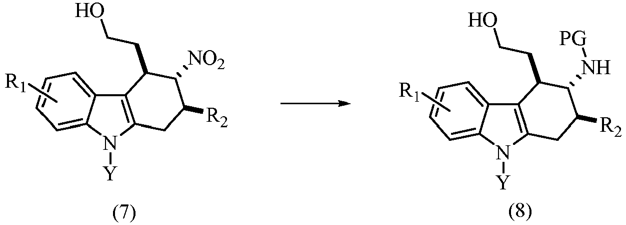
b‴) *O*-alkylation with a halogenated derivative of formula R₄-halogen wherein R₄ is as defined in claim 1, to afford intermediate (9); 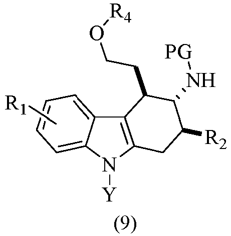
c‴) final deprotection and optional *N*-alkylation or *N*-acylation of intermediate (9) provides compounds of formula (**I**) wherein wherein X is O, R₄ is different from H, and R₁, R₂, R₃ and R₅ are as defined in claim 1.

## Patentansprüche

1. Eine Verbindung der Formel (I), ein pharmazeutisch akzeptables Salz davon, oder ein Enantiomer oder Mischungen aus Enantiomeren, entweder der Verbindung der Formel (I) oder eines ihrer pharmazeutisch akzeptablen Salze 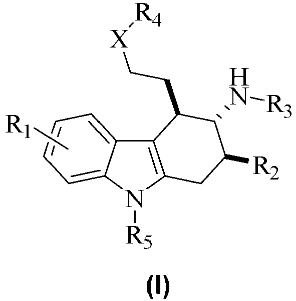 wobei:
sich die "fettgedruckten Bindungen" und die "gestrichelten Bindungen" in der Formel **(I)** auf die relative Stereochemie der chiralen Zentren beziehen;
X = NH oder O ist;
R₁ ausgewählt ist aus der Gruppe bestehend aus H, -(C₁-C₃)-Alkyl, -O(C₁-C₃)-Alkyl, Halogen, -CF₃ und -OCF₃;
R₂ = Phenyl ist, das wahlweise substituiert durch eine Gruppe ist, die ausgewählt ist aus -(C₁-C₃)-Alkyl, -O(-C ₁C₃)-Alkyl, Halogen, -CF₃, -OCF₃, -(C₃-C₆)-Cycloalkyl, -NH-(C₁-C₃)-Alkyl, -NH-CO-(C₁-C₃)-Alkyl, und -O(C₃-C₆)-Cycloalkyl;
R₃ ausgewählt ist aus der Gruppe bestehend aus H, -(C₁-C₄)-Alkyl, -(C₃-C₆)-Cycloalkyl, -CO-(C₁-C₆)-Alkyl und -CO-(C₃-C₆)-Cycloalkyl;
R₄ ausgewählt ist aus der Gruppe bestehend aus H, -(C₁-C₄)-Alkyl, -(C₃-C₆)-Cycloalkyl, -(CH₂)-(C₃-C₆)-Cycloalkyl, -(CH₂CH₂)-(C₃-C₆)-Cycloalkyl;
und wobei R₄ wahlweise durch Halogen substituiert ist;
und R₅ ausgewählt ist aus der Gruppe bestehend aus H, -CH₃ und -COCH₃.

2. Die Verbindung der Formel **(I)** nach Anspruch 1, wobei
R₂ = Phenyl ist, das wahlweise durch Methyl, Ethyl, -OCH₃, -OCH₂CH₃, -OCH(CH₃)₂, F, Cl, Br, -CF₃, -OCF₃, -(C₃-C₆)-Cycloalkyl, -NHCH₃, -NHCOCH₃ und -O(C₃-C₆)-Cycloalkyl substituiert ist;
R₄ ausgewählt ist aus der Gruppe bestehend aus H, Methyl, Ethyl, Propyl, Isopropyl, Isobutyl, Cyclopropyl, Cyclobutyl, Cyclopentyl, Cyclohexyl und 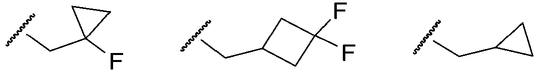 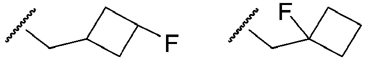
wobei die wellenförmige Linie die Bindungsstelle darstellt.

3. Die Verbindung der Formel (**I**) nach einem der Ansprüche 1 bis 2, wobei
R₁ ausgewählt ist aus der Gruppe bestehend aus H, Methyl, Ethyl, -OCH₃, -OCH₂CH₃; F, Cl, Br, -CF₃ und -OCF₃;
R₂ = Phenyl ist, das wahlweise durch Methyl, Ethyl, -OCH₃, -OCH₂CH₃, -OCH(CH₃)₂, F, Cl, Br, -CF₃, -OCF₃, -NHCH₃ und -NHCOCH₃ substituiert ist;
R₃ ausgewählt ist aus der Gruppe bestehend aus H, Methyl, Ethyl, -COCH₃ und Cyclopropyl;
R₄ ausgewählt ist aus der Gruppe bestehend aus H, Methyl, Ethyl, Propyl, Isopropyl, Isobutyl, Cyclopropyl, Cyclobutyl und 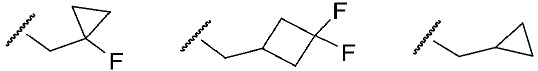 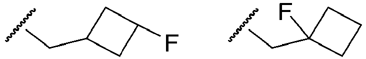
wobei die wellenförmige Linie die Bindungsstelle darstellt.

4. Die Verbindung der Formel (**I**) nach einem der Ansprüche 1 bis 3, wobei
R₁ ausgewählt ist aus der Gruppe bestehend aus H, Methyl, Ethyl, -OCH₃, -OCH₂CH₃, F, Cl, Br, -CF₃ und -OCF₃;
R₂ = Phenyl ist, das wahlweise durch Methyl, Ethyl, -OCH₃, -OCH₂CH₃, F, Cl, -CF₃ und -OCF₃ substituiert ist;
R₃ ausgewählt ist aus der Gruppe bestehend aus H und Methyl;
R₄ ausgewählt ist aus der Gruppe bestehend aus H, Methyl, Ethyl, Propyl, Isopropyl, Isobutyl, Cyclopropyl, Cyclobutyl und 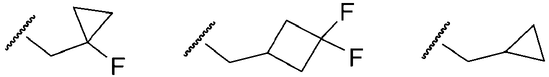 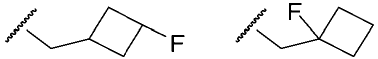
wobei die wellenförmige Linie die Bindungsstelle darstellt.

5. Die Verbindung der Formel (**I**) nach einem der Ansprüche 1 bis 4, wobei
X = NH ist;
R₁ ausgewählt ist aus der Gruppe bestehend aus H, F, Cl, Methyl und -OCH₃;
R₂ = Phenyl ist, das wahlweise durch Methyl, Ethyl, -OCH₃, -OCH₂CH₃, F, Cl, -CF₃ und -OCF₃ substituiert ist;
R₃ ausgewählt ist aus der Gruppe bestehend aus H und Methyl;
R₄ ausgewählt ist aus der Gruppe bestehend aus H, Methyl, Ethyl, Propyl, Isopropyl, Isobutyl, Cyclopropyl, Cyclobutyl und 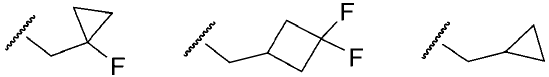 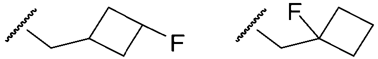
wobei die wellenförmige Linie die Bindungsstelle darstellt;
und R₅ ausgewählt ist aus der Gruppe bestehend aus H, CH₃ und -COCH₃.

6. Die Verbindung der Formel (**I**) nach einem der Ansprüche 1 bis 5, wobei
X = NH ist;
R₁ ausgewählt ist aus der Gruppe bestehend aus H, F, Cl, Methyl und -OCH₃;
R₂ = Phenyl ist, das wahlweise durch Methyl, Ethyl; -OCH₃, -OCH₂CH₃, F, Cl, -CF₃ und -OCF₃ substituiert ist;
R₃ ausgewählt ist aus der Gruppe bestehend aus H und Methyl;
R₄ ausgewählt ist aus der Gruppe bestehend aus 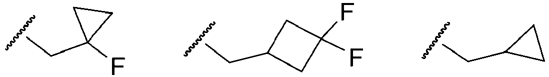 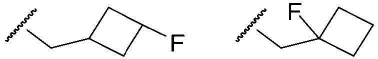
wobei die wellenförmige Linie die Bindungsstelle darstellt;
und R₅ ausgewählt ist aus der Gruppe bestehend aus H, CH₃ und -COCH₃.

7. Die Verbindung der Formel (**I**) nach einem der Ansprüche 1 bis 6, wobei
X = O ist;
R₁ ausgewählt ist aus der Gruppe bestehend aus H, F, Cl, Methyl und -OCH₃;
R₂ = Phenyl ist, das wahlweise durch Methyl, Ethyl; -OCH₃, -OCH₂CH₃, F, Cl, -CF₃ und -OCF₃ substituiert ist;
R₃ ausgewählt ist aus der Gruppe bestehend aus H und Methyl;
R₄ ausgewählt ist aus der Gruppe bestehend aus H, Methyl, Ethyl, Propyl, Isopropyl, Isobutyl, Cyclopropyl, Cyclobutyl und 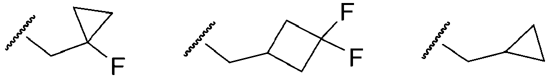 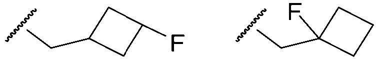
wobei die wellenförmige Linie die Bindungsstelle darstellt;
und R₅ ausgewählt ist aus der Gruppe bestehend aus H, CH₃ und -COCH₃.

8. Die Verbindung der Formel (**I**) nach einem der Ansprüche 1 bis 7, wobei
X = O ist;
R₁ ausgewählt ist aus der Gruppe bestehend aus H, F, Cl, Methyl und -OCH₃;
R₂ = Phenyl ist, das wahlweise durch Methyl, Ethyl; -OCH₃, -OCH₂CH₃, F, Cl, -CF₃ und -OCF₃ substituiert ist;
R₃ ausgewählt ist aus der Gruppe bestehend aus H und Methyl;
R₄ ausgewählt ist aus der Gruppe bestehend aus 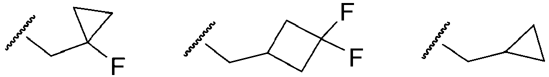 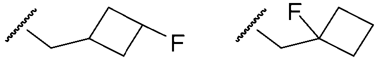
wobei die wellenförmige Linie die Bindungsstelle darstellt;
und R₅ ausgewählt ist aus der Gruppe bestehend aus H, CH₃ und -COCH₃.

9. Die Verbindung der Formel (**I**) nach einem der Ansprüche 1 bis 8, die ausgewählt ist aus
(2*R*,3*R*,4*R*)-4-{2-[(Cyclopropylmethyl)amino]ethyl}-2-phenyl-2,3,4,9-tetrahydro-1*H-*carbazol-3-amin (**Ia**);
(2*R*,3*R*,4*R*)-4-{2-[(Cyclopropylmethyl)amino]ethyl}-2-(4-methylphenyl)-2,3,4,9-tetrahydro-1*H*-carbazol-3-amin (**Ib**);
(2*R*,3*R*,4*R*)-4-{2-[(Cyclopropylmethyl)amino]ethyl}-2-(4-methoxyphenyl)-2,3,4,9-tetrahydro-1*H*-carbazol-3-amin (**Ic**);
(2*S*,3*S*,4*S*)-4-{2-[(Cyclopropylmethyl)amino]ethyl}-2-(4-methoxyphenyl)-2,3,4,9-tetrahydro-1*H*-carbazol-3-amin (**Id**);
(2*R*,3*R*,4*R*)-4-{2-[(Cyclopropylmethyl)amino]ethyl}-2-[4-(trifluormethyl)phenyl]-2,3,4,9-tetrahydro-1*H*-carbazol-3-amin (**Ie**);
(2*R*,3*R*,4*R*)-4-{2-[(Cyclopropylmethyl)amino]ethyl}-2-[4-(trifluormethoxy)phenyl]-2,3,4,9-tetrahydro-1*H*-carbazol-3-amin (**If**);
(2*R*,3*S*,4*R*)-4-{2-[(Cyclopropylmethyl)amino]ethyl}-2-(2-fluorphenyl)-2,3,4,9-tetrahydro-1*H*-carbazol-3-amin (**Ig**);
(2*R*,3*R*,4*R*)-2-(3-Chlorphenyl)-4-{2-[(cyclopropylmethyl)amino]ethyl}-2,3,4,9-tetrahydro-1*H*-carbazol-3-amin (**Ih**);
(2*R*,3*R*,4*R*)-2-(4-Chlorophenyl)-4-{2-[(cyclopropylmethyl)amino]ethyl}-2,3,4,9-tetrahydro-1*H*-carbazol-3-amin (**Ii**);
(2*R*,3*R*,4*R*)-4-{2-[(Cyclopropylmethyl)amino]ethyl}-2-(3-methoxyphenyl)-2,3,4,9-tetrahydro-1*H*-carbazol-3-amin (**Ij**);
(2*R*,3*S*,4*R*)-4-{2-[(Cyclopropylmethyl)amino]ethyl}-2-(2-methoxyphenyl)-2,3,4,9-tetrahydro-1*H*-carbazol-3-amin (**Ik**);
(2*R*,3*R*,4*R*)-4-{2-[(Cyclopropylmethyl)amino]ethyl}-2-(3-methylphenyl)-2,3,4,9-tetrahydro-1*H*-carbazol-3-amin (**Il**);
(2*R*,3*R*,4*R*)-4-{2-[(Cyclopropylmethyl)amino]ethyl}-2-(2-methylphenyl)-2,3,4,9-tetrahydro-1*H*-carbazol-3-amin (**Im**);
(2*R*,3*S*,4*R*)-2-(2-Chlorophenyl)-4-{2-[(cyclopropylmethyl)amino]ethyl}-2,3,4,9-tetrahydro-1*H*-carbazol-3-amin (**In**);
(2*R*,3*R*,4*R*)-4-{2-[(Cyclopropylmethyl)amino]ethyl}-2-(4-fluorophenyl)-2,3,4,9-tetrahydro-1*H*-carbazol-3-amin (**Io**);
(2*S*,3*S*,4*S*)-4-{2-[(Cyclopropylmethyl)amino]ethyl}-2-(4-fluorophenyl)-2,3,4,9-tetrahydro-1*H*-carbazol-3-amin (**Ip**);
(2*R*,3*R*,4*R*)-4-{2-[(Cyclopropylmethyl)amino]ethyl}-2-(3-fluorophenyl)-2,3,4,9-tetrahydro-1*H*-carbazol-3-amin (**Iq**);
(2*R*,3*R*,4*R*)-4-{2-[(Cyclopropylmethyl)amino]ethyl}-2-(4-methoxyphenyl)-N-methyl-2,3,4,9-tetrahydro-1*H*-carbazol-3-amin (**Ir**);
(2*S*,3*S*,4*S*)-4-{2-[(Cyclopropylmethyl)amino]ethyl}-2-(4-methoxyphenyl)-N-methyl-2,3,4,9-tetrahydro-1*H*-carbazol-3-amin (**Is**);
*N*-[(2*R*,3*R*,4*R*)-4-{2-[(Cyclopropylmethyl)amino]ethyl}-2-(4-methoxyphenyl)-2,3,4,9-tetrahydro-1*H*-carbazol-3-yl]acetamid (**It**);
(2*R*,3*R*,4*R*)-4-(2-{[(3,3-Difluorocyclobutyl)methyl]amino}ethyl)-2-(4-methylphenyl)-2,3,4,9-tetrahydro-1*H*-carbazol-3-amin (**Iu**);
(2*R*,3*R*,4*R*)-9-Methyl-2-(4-methylphenyl)-4-(2-{[(oxetan-3-yl)methyl]amino}ethyl)-2,3,4,9-tetrahydro-1*H*-carbazol-3-amin (**Iv**);
(2*R*,3*R*,4*R*)-2-(4-Methoxyphenyl)-4-[2-(methylamino)ethyl]-2,3,4,9-tetrahydro-1*H-*carbazol-3-amin (**Iw**);
(2*R*,3*R*,4*R*)-2-(4-Methylphenyl)-4-{2-[(2-methylpropyl)amino]ethyl}-2,3,4,9-tetrahydro-1*H*-carbazol-3-amin (**Ix**);
(2*R*,3*R*,4*R*)-2-(4-Methylphenyl)-4-{2-[(propan-2-yl)amino]ethyl}-2,3,4,9-tetrahydro-1*H-*carbazol-3-amin (**Iy**);
(2*R*,3*R*,4*R*)-4-[2-(Ethylamino)ethyl]-2-(4-methylphenyl)-2,3,4,9-tetrahydro-1*H-*carbazol-3-amin (**Iz**);
(2*R*,3*R*,4*R*)-4-[2-(Cyclopropylamino)ethyl]-2-(4-methylphenyl)-2,3,4,9-tetrahydro-1*H-*carbazol-3-amin (**Iaa**);
(2*R*,3*R*,4*R*)-6-Chloro-4-(2-{[(3,3-difluorocyclobutyl)methyl]amino}ethyl)-2-(4-methylphenyl)-2,3,4,9-tetrahydro-1*H*-carbazol-3-amin (**Iab**);
(2*R*,3*R*,4*R*)-6-Chloro-4-(2-{[(3,3-difluorocyclobutyl)methyl]amino}ethyl)-*N*-methyl-2-(4-methylphenyl)-2,3,4,9-tetrahydro-1*H*-carbazol-3-amin (**Iac**);
2-[(2*R*,3*R*,4*R*)-3-Amino-2-(4-methylphenyl)-2,3,4,9-tetrahydro-1*H*-carbazol-4-yl]ethan-1-ol (**Iad**);
(2*R*,3*R*,4*R*)-4-(2-Methoxyethyl)-2-(4-methoxyphenyl)-2,3,4,9-tetrahydro-1*H*-carbazol-3-amin (**Iae**);
(2*R*,3*R*,4*R*)-4-{2-[(Cyclopropylmethyl)amino]ethyl}-6-methoxy-2-(4-methylphenyl)-2,3,4,9-tetrahydro-1*H*-carbazol-3-amin (**Iaf**);
(2*R*,3*R*,4*R*)-6-Chloro-4-{2-[(cyclopropylmethyl)amino]ethyl}-2-(4-methylphenyl)-2,3,4,9-tetrahydro-1*H*-carbazol-3-amin (**Iag**);
(2*R*,3*R*,4*R*)-6-Chloro-4-{2-[(cyclopropylmethyl)amino]ethyl}-2-(4-methoxyphenyl)-2,3,4,9-tetrahydro-1*H*-carbazol-3-amin (**Iah**);
(2*R*,3*R*,4*R*)-4-{2-[(Cyclopropylmethyl)amino]ethyl}-6-methyl-2-(4-methylphenyl)-2,3,4,9-tetrahydro-1*H*-carbazol-3-amin (**Iai**);
(2*R*,3*R*,4*R*)-4-{2-[(Cyclopropylmethyl)amino]ethyl}-6-fluoro-2-(4-methylphenyl)-2,3,4,9-tetrahydro-1*H*-carbazol-3-amin **(Iaj);**
(2*R*,3*R*,4*R*)-6-Chloro-4-{2-[(cyclopropylmethyl)amino]ethyl}-*N*-methyl-2-(4-methylphenyl)-2,3,4,9-tetrahydro-1*H*-carbazol-3-amin **(Iak);** und
(2*R*,3*R*,4*R*)-4-{2-[(Cyclopropylmethyl)amino]ethyl}-9-methyl-2-(4-methylphenyl)-2,3,4,9-tetrahydro-1*H*-carbazol-3-amin **(Ial).**

10. Eine pharmazeutische Zusammensetzung, die eine therapeutisch wirksame Menge einer Verbindung der Formel (**I**) oder eines pharmazeutisch akzeptablen Salzes davon oder eines Enantiomers oder Mischungen aus Enantiomeren, entweder der Verbindung der Formel (**I**) oder ihres pharmazeutisch akzeptablen Salzes, wie in einem der Ansprüche 1 bis 9 definiert, zusammen mit einem oder mehreren pharmazeutisch akzeptablen Hilfsstoffen oder Trägersubstanzen umfasst.

11. Eine Verbindung der Formel (**I**) nach einem der Ansprüche 1 bis 9 oder eine pharmazeutische Zusammensetzung nach Anspruch 10 zur Verwendung als Medikament.

12. Eine Verbindung der Formel (**I**) nach einem der Ansprüche 1 bis 9 oder eine pharmazeutische Zusammensetzung nach Anspruch 10, zur Verwendung bei der Behandlung und/oder Prävention von einer Krebserkrankung, die durch eine NPM1-Fehlfunktion verursacht wird.

13. Die Verbindung zur Verwendung nach Anspruch 12, wobei die Krebserkrankung ausgewählt ist aus der Gruppe bestehend aus: Mixed-Lineage-Leukämie (MLL), MLL-bedingter Leukämie, MLL-assoziierter Leukämie, MLL-positiver Leukämie, MLLinduzierter Leukämie, Mixed-Lineage-Leukämie mit Rearrangements (MLL-r), Leukämie, die in Verbindung mit einem MLL-Rearrangement bzw. einem Rearrangement des MLL-Gens steht, akuter Leukämie, chronischer Leukämie, indolenter Leukämie, lymphoblastischer Leukämie, lymphozytischer Leukämie, myeloischer Leukämie, myelogener Leukämie, Leukämie im Kindesalter, akuter lymphozytischer Leukämie (ALL), akuter myeloischer Leukämie (AML), anaplastischem großzelligem Lymphom (ALCL), akuter promyelozytärer Leukämie (APL), akuter granulozytischer Leukämie, akuter nicht-lymphatischer Leukämie, chronischer lymphatischer Leukämie (CLL), chronischer myeloischer Leukämie (CML), therapiebedingter Leukämie, myelodysplastischem Syndrom (MDS), myeloproliferativer Erkrankung (MPD), myeloproliferativer Neoplasie (MPN), Plasmazellneoplasma, Multiplem Myelom, Myelodysplasie, kutanem T-Zell-Lymphom, lymphatischem Neoplasma, AIDS-assoziiertem Lymphom, Thymom, Thymuskarzinom, Mycosis fungoides, Alibert-Bazin-Syndrom, Granuloma fungoides, Sézary-Syndrom, Haarzell-Leukämie, T-Zell-Prolymphocyten-Leukämie (T-PLL), großzelliger granulärer Lymphozyten-Leukämie, meningealer Leukämie, leukämischer Leptomeningitis, leukämischer Meningitis, multiplem Myelom, Hodgkin-Lymphom, Non-Hodgkin-Lymphom (malignem Lymphom) oder Waldenström-Makroglobulinämie bei einem Säugetier, das sie benötigt.

14. Die Verbindung zur Verwendung nach einem der Ansprüche 12 bis 13, wobei die Krebserkrankung ausgewählt ist aus akuter myeloischer Leukämie (AML), anaplastischem großzelligem Lymphom (ALCL) und akuter promyelozytärer Leukämie (APL).

15. Ein Verfahren zur Herstellung der Verbindung der Formel (I) oder eines pharmazeutisch akzeptablen Salzes davon, oder eines Enantiomers oder Mischungen aus Enantiomeren, entweder der Verbindung der Formel (I) oder ihres pharmazeutisch akzeptablen Salzes, wie in einem der Ansprüche 1 bis 9 definiert, umfassend:
a) reduktive Aminierung des Zwischenprodukts der Formel (3) mit einem Amin der Formel R₄NH₂, um ein Zwischenprodukt der Formel (4) zu ergeben, wobei R₁, R₂ und R₄ wie in Anspruch 1 definiert sind und Y Methyl, Acetyl oder eine Schutzgruppe ist; 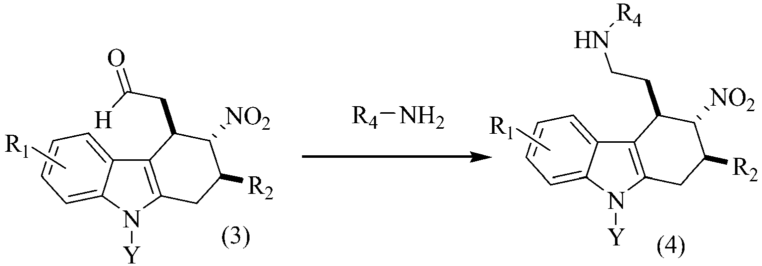
b) anschließende Reduktion der Nitrogruppe des Zwischenprodukts (4), wahlweise gefolgt von der Entfernung der Schutzgruppe, was zu Endverbindungen der Formel (**I**) führt, wobei X = NH ist, R₃ = H ist und R₁, R₂, R₄ und R₅ wie in Anspruch 1 definiert sind;
oder, ersatzweise,
a') reduktive Aminierung des Zwischenprodukts der Formel (3) mit einem Amin der Formel R₄NH₂, um ein Zwischenprodukt der Formel (4) zu ergeben, wobei R₁, R₂ und R₄ wie in Anspruch 1 definiert sind und Y Methyl, Acetyl oder eine Schutzgruppe ist; 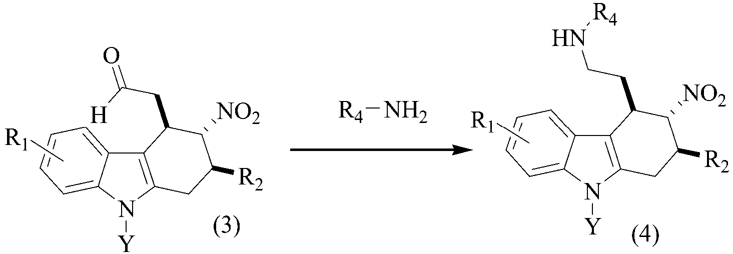
b') Reduktion der Nitrogruppe, der eine N-Schützung vorausgeht oder folgt, um ein Zwischenprodukt der Formel (5) zu ergeben; 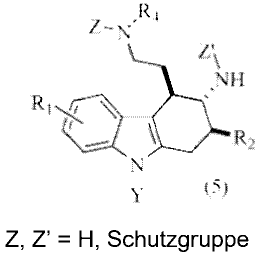
c') N-Alkylierung oder N-Acylierung des Zwischenprodukts (5), um das Zwischenprodukt (6) zu ergeben; 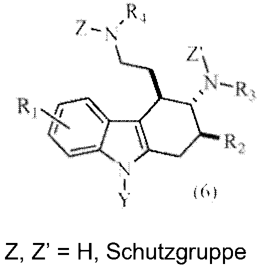
d') eine endgültige Entschützung stellt die Verbindungen der Formel (I) bereit, wobei X = NH ist, R₃ von H verschieden ist und R₁, R₂, R₄ und R₅ wie in Anspruch 1 definiert sind;
oder
a") die Reduktion der Nitrogruppe des Zwischenprodukts der Formel (7), gefolgt von einer optionalen *N*-Alkylierung oder *N*-Acylierung und Entschützung, ergibt die Verbindungen der Formel (**I**), wobei X = O ist, R₄ = H ist und R₁, R₂, R₃ und R₅ wie in Anspruch 1 definiert sind; 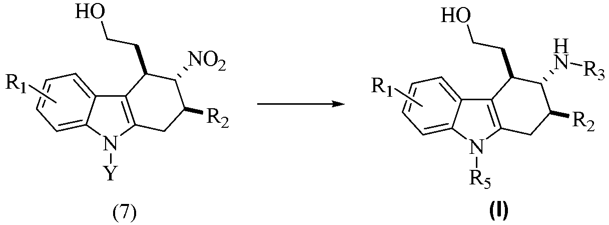 oder, ersatzweise,
a‴) die Reduktion der Nitrogruppe und die in-situ *N*-Schützung des Zwischenprodukts der Formel (7) stellen ein Zwischenprodukt der Formel (8) bereit; 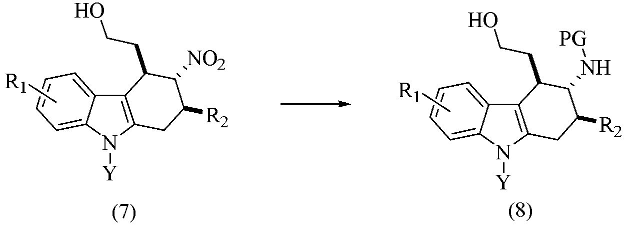
b‴) die O-Alkylierung mit einem halogenierten Derivat der Formel R₄-Halogen, wobei R₄ wie in Anspruch 1 definiert ist, um ein Zwischenprodukt (9) zu ergeben; 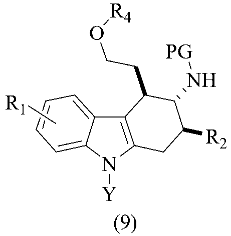
c‴) die endgültige Entschützung und wahlweise *N*-Alkylierung oder *N*-Acylierung des Zwischenprodukts (9) stellt Verbindungen der Formel (**I**) bereit, wobei X = O ist, R₄ von H verschieden ist und R₁, R₂, R₃ und R₅ wie in Anspruch 1 definiert sind.

## Revendications

1. Un composé de formule (I), un sel pharmaceutiquement acceptable de celui-ci, ou tout énantiomère ou mélange d'énantiomères, soit du composé de formule (**I**), soit de l'un de ses sels pharmaceutiquement acceptables 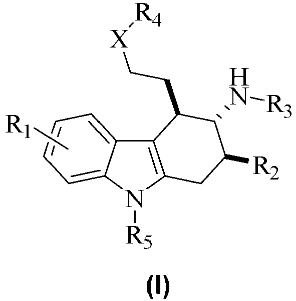 dans lequel :
dans la formule (**I**), les « liaisons en gras » et les « liaisons en pointillés » font référence à la stéréochimie relative des centres chiraux ;
X est NH ou O ;
R₁ est choisi dans le groupe constitué par H, alkyle en -(C₁-C₃), alkyle en -O(C₁-C₃), halogène, -CF₃ et -OCF₃ ;
R₂ est phényle facultativement substitué par un groupe choisi parmi alkyle en -(C₁-C₃), alkyle en -O-(C₁-C₃), halogène, -CF₃, -OCF₃, cycloalkyle en -(C₃-C₆), alkyle en -NH-(C₁-C₃), alkyle en -NH-CO-(C₁-C₃) et cycloalkyle en -O(C₃-C₆) ;
R₃ est choisi dans le groupe constitué par H, alkyle en -(C₁-C₄), cycloalkyle en -(C₃-C₆)-, alkyle en -CO-(C₁-C₆) et cycloalkyle en -CO-(C₃-C₆) ;
R₄ est choisi dans le groupe constitué par H, alkyle en -(C₁-C₄), cycloalkyle en -(C₃-C₆), cycloalkyle en -(CH₂)-(C₃-C₆), cycloalkyle en -(CH₂CH₂)-(C₃-C₆) ;
et où R₄ est facultativement substitué par un halogène ;
et R₅ est choisi dans le groupe constitué par H, -CH₃ et -COCH₃.

2. Le composé de formule (**I**) selon la revendication 1, où
R₂ est phényle, facultativement substitué par méthyle, éthyle, -OCH₃, -OCH₂CH₃, -OCH(CH₃)₂, F, Cl, Br, -CF₃, -OCF₃, cycloalkyle en -(C₃-C₆), -NHCH₃, -NHCOCH₃ et cycloalkyle en -O(C₃-C₆) ;
R₄ est choisi dans le groupe constitué par H, méthyle, éthyle, propyle, isopropyle, isobutyle, cyclopropyle, cyclobutyle, cyclopentyle, cyclohexyle et 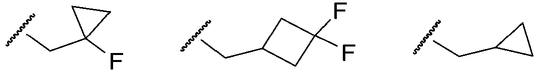 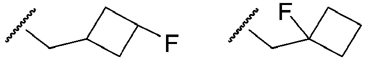
où la ligne ondulée représente le point de fixation.

3. Le composé de formule (**I**) selon l'une quelconque des revendications 1 à 2, où
R₁ est choisi dans le groupe constitué par H, méthyle, éthyle, -OCH₃, -OCH₂CH₃ ; F, Cl, Br, -CF₃ et -OCF₃ ;
R₂ est phényle facultativement substitué par méthyle, éthyle, -OCH₃, -OCH₂CH₃, -OCH(CH₃)₂, F, Cl, Br, -CF₃, -OCF₃, -NHCH₃ et -NHCOCH₃ ;
R₃ est choisi dans le groupe constitué par H, méthyle, éthyle, -COCH₃ et cyclopropyle ;
R₄ est choisi dans le groupe constitué par H, méthyle, éthyle, propyle, isopropyle, isobutyle, cyclopropyle, cyclobutyle et 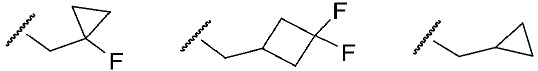 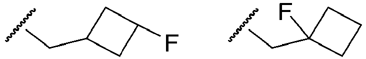
où la ligne ondulée représente le point de fixation.

4. Le composé de formule (**I**) selon l'une quelconque des revendications 1 à 3, où
R₁ est choisi dans le groupe constitué par H, méthyle, éthyle, -OCH₃, -OCH₂CH₃, F, Cl, Br, -CF₃ et -OCF₃ ;
R₂ est phényle facultativement substitué par méthyle, éthyle, -OCH₃, -OCH₂CH₃, F, Cl, -CF₃ et -OCF₃ ;
R₃ est choisi dans le groupe constitué par H et méthyle ;
R₄ est choisi dans le groupe constitué par H, méthyle, éthyle, propyle, isopropyle, isobutyle, cyclopropyle, cyclobutyle et 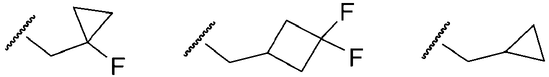 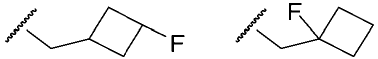
où la ligne ondulée représente le point de fixation.

5. Le composé de formule (**I**) selon l'une quelconque des revendications 1 à 4, où
X est NH ;
R₁ est choisi dans le groupe constitué par H, F, Cl, méthyle et -OCH₃ ;
R₂ est phényle facultativement substitué par méthyle, éthyle, -OCH₃, -OCH₂CH₃, F, Cl, -CF₃ et -OCF₃ ;
R₃ est choisi dans le groupe constitué par H et méthyle ;
R₄ est choisi dans le groupe constitué par H, méthyle, éthyle, propyle, isopropyle, isobutyle, cyclopropyle, cyclobutyle et 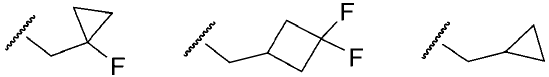 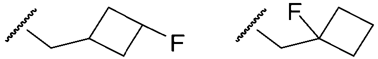
où la ligne ondulée représente le point de fixation ;
et R₅ est choisi dans le groupe constitué par H, CH₃ et -COCH₃.

6. Le composé de formule (**I**) selon l'une quelconque des revendications 1 à 5, où
X est NH ;
R₁ est choisi dans le groupe constitué par H, F, Cl, méthyle et -OCH₃ ;
R₂ est phényle facultativement substitué par méthyle, éthyle ; -OCH₃, -OCH₂CH₃, **F,** Cl, -CF₃ et -OCF₃ ;
R₃ est choisi dans le groupe constitué par H et méthyle ;
R₄ est choisi dans le groupe constitué par 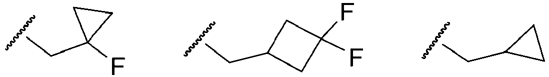 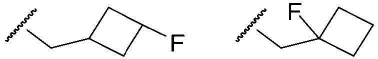
où la ligne ondulée représente le point de fixation ;
et R₅ est choisi dans le groupe constitué par H, CH₃ et -COCH₃.

7. Le composé de formule (**I**) selon l'une quelconque des revendications 1 à 6, où
X est O ;
R₁ est choisi dans le groupe constitué par H, F, Cl, méthyle et -OCH₃ ;
R₂ est phényle facultativement substitué par méthyle, éthyle ; -OCH₃, -OCH₂CH₃, **F,** Cl, -CF₃ et -OCF₃ ;
R₃ est choisi dans le groupe constitué par H et méthyle ;
R₄ est choisi dans le groupe constitué par H, méthyle, éthyle, propyle, isopropyle, isobutyle, cyclopropyle, cyclobutyle et 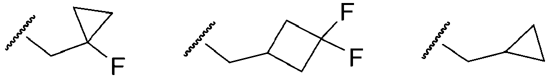 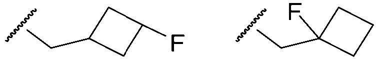
où la ligne ondulée représente le point de fixation ;
et R₅ est choisi dans le groupe constitué par H, CH₃ et -COCH₃.

8. Le composé de formule (**I**) selon l'une quelconque des revendications 1 à 7, où
X est O ;
R₁ est choisi dans le groupe constitué par H, F, Cl, méthyle et -OCH₃ ;
R₂ est phényle, facultativement substitué par méthyle, éthyle ; -OCH₃, -OCH₂CH₃, **F,** Cl, -CF₃ et -OCF₃ ;
R₃ est choisi dans le groupe constitué par H et méthyle ;
R₄ est choisi dans le groupe constitué par 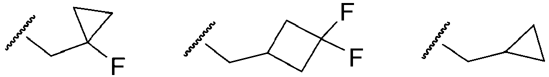 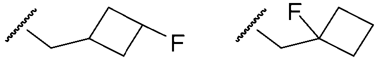
où la ligne ondulée représente le point de fixation ;
et R₅ est choisi dans le groupe constitué par H, CH₃ et -COCH₃.

9. Le composé de formule (**I**) selon l'une quelconque des revendications 1 à 8, qui est choisi parmi
(2*R*,3*R*,4*R*)-4-{2-[(Cyclopropylméthyl)amino]éthyl}-2-phényl-2,3,4,9-tétrahydro-1H-carbazol-3-amine **(Ia)** ;
(2*R*,3*R*,4*R*)-4-{2-[(Cyclopropylméthyl)amino]éthyl}-2-(4-méthylphényl)-2,3,4,9-tétrahydro-1*H*-carbazol-3-amine **(Ib)** ;
(2*R*,3*R*,4*R*)-4-{2-[(Cyclopropylméthyl)amino]éthyl}-2-(4-méthoxyphényl)-2,3,4,9-tétrahydro-1*H*-carbazol-3-amine **(Ic)** ;
(2*S*,3*S*,4*S*)-4-{2-[(Cyclopropylméthyl)amino]éthyl}-2-(4-méthoxyphényl)-2,3,4,9-tétrahydro-1*H*-carbazol-3-amine **(Id);**
(2*R*,3*R*,4*R*)-4-{2-[(Cyclopropylméthyl)amino]éthyl}-2-[4-(trifluorométhyl)phényl]-2,3,4,9-tétrahydro-1*H*-carbazol-3-amine **(Ie);**
(2*R*,3*R*,4*R*)-4-{2-[(Cyclopropylméthyl)amino]éthyl}-2-[4-(trifluorométhoxy)phényl]-2,3,4,9-tétrahydro-1*H*-carbazol-3-amine **(If);**
(2*R*,3*S*,4*R*)-4-{2-[(Cyclopropylméthyl)amino]éthyl}-2-(2-fluorophényl)-2,3,4,9-tétrahydro-1*H*-carbazol-3-amine (**Ig**);
(2*R*,3*R*,4*R*)-2-(3-Chlorophényl)-4-{2-[(cyclopropylméthyl)amino]éthyl}-2,3,4,9-tétrahydro-1*H*-carbazol-3-amine **(Ih);**
(2*R*,3*R*,4*R*)-2-(4-Chlorophényl)-4-{2-[(cyclopropylméthyl)amino]éthyl}-2,3,4,9-tétrahydro-1*H*-carbazol-3-amine (**Ii**);
(2*R*,3*R*,4*R*)-4-{2-[(Cyclopropylméthyl)amino]éthyl}-2-(3-méthoxyphényl)-2,3,4,9-tétrahydro-1*H*-carbazol-3-amine (**Ij**);
(2*R*,3*S*,4*R*)-4-{2-[(Cyclopropylméthyl)amino]éthyl}-2-(2-méthoxyphényl)-2,3,4,9-tétrahydro-1*H*-carbazol-3-amine **(Ik);**
(2*R*,3*R*,4*R*)-4-{2-[(Cyclopropylméthyl)amino]éthyl}-2-(3-méthylphényl)-2,3,4,9-tétrahydro-1*H*-carbazol-3-amine **(Il);**
(2*R*,3*R*,4*R*)-4-{2-[(Cyclopropylméthyl)amino]éthyl}-2-(2-méthylphényl)-2,3,4,9-tétrahydro-1*H*-carbazol-3-amine **(Im);**
(2*R*,3*S*,4*R*)-2-(2-Chlorophényl)-4-{2-[(cyclopropylméthyl)amino]éthyl}-2,3,4,9-tétrahydro-1*H*-carbazol-3-amine **(In);**
(2*R*,3*R*,4*R*)-4-{2-[(Cyclopropylméthyl)amino]éthyl}-2-(4-fluorophényl)-2,3,4,9-tétrahydro-1*H*-carbazol-3-amine (**Io**);
(2*S*,3*S*,4*S*)-4-{2-[(Cyclopropylméthyl)amino]éthyl}-2-(4-fluorophényl)-2,3,4,9-tétrahydro-1*H*-carbazol-3-amine **(Ip);**
(2*R*,3*R*,4*R*)-4-{2-[(Cyclopropylméthyl)amino]éthyl}-2-(3-fluorophényl)-2,3,4,9-tétrahydro-1*H*-carbazol-3-amine **(Iq);**
(2*R*,3*R*,4*R*)-4-{2-[(Cyclopropylméthyl)amino]éthyl}-2-(4-méthoxyphényl)-N-méthyl-2,3,4,9-tétrahydro-1*H*-carbazol-3-amine **(Ir);**
(2*S*,3*S*,4*S*)-4-{2-[(Cyclopropylméthyl)amino]éthyl}-2-(4-méthoxyphényl)-*N*-méthyl-2,3,4,9-tétrahydro-1*H*-carbazol-3-amine **(Is);**
*N*-[(2*R*,3*R*,4*R*)-4-{2-[(Cyclopropylméthyl)amino]éthyl}-2-(4-méthoxyphényl)-2,3,4,9-tétrahydro-1*H*-carbazol-3-yl]acétamide **(It);**
(2*R*,3*R*,4*R*)-4-(2-{[(3,3-Difluorocyclobutyl)méthyl]amino}éthyl)-2-(4-méthylphényl)-2,3,4,9-tétrahydro-1*H*-carbazol-3-amine (**Iu**);
(2*R*,3*R*,4*R*)-9-Méthyl-2-(4-méthylphényl)-4-(2-{[(oxétan-3-yl)méthyl]amino}éthyl)-2,3,4,9-tétrahydro-1*H*-carbazol-3-amine **(Iv);**
(2*R*,3*R*,4*R*)-2-(4-Méthoxyphényl)-4-[2-(méthylamino)éthyl]-2,3,4,9-tétrahydro-1*H-*carbazol-3-amine **(Iw);**
(2*R*,3*R*,4*R*)-2-(4-Méthylphényl)-4-{2-[(2-méthylpropyl)amino]éthyl}-2,3,4,9-tétrahydro-1*H*-carbazol-3-amine **(Ix);**
(2*R*,3*R*,4*R*)-2-(4-Méthylphényl)-4-{2-[(propan-2-yl)amino]éthyl}-2,3,4,9-tétrahydro-1*H-*carbazol-3-amine (**Iy**);
(2*R*,3*R*,4*R*)-4-[2-(Éthylamino)éthyl]-2-(4-méthylphényl)-2,3,4,9-tétrahydro-1*H-*carbazol-3-amine **(Iz);**
(2*R*,3*R*,4*R*)-4-[2-(Cyclopropylamino)éthyl]-2-(4-méthylphényl)-2,3,4,9-tétrahydro-1*H-*carbazol-3-amine (**Iaa**);
(2*R*,3*R*,4*R*)-6-Chloro-4-(2-{[(3,3-difluorocyclobutyl)méthyl]amino}éthyl)-2-(4-méthylphényl)-2,3,4,9-tétrahydro-1*H*-carbazol-3-amine (**Iab**);
(2*R*,3*R*,4*R*)-6-Chloro-4-(2-{[(3,3-difluorocyclobutyl)méthyl]amino}éthyl)-*N*-méthyl-2-(4-méthylphényl)-2,3,4,9-tétrahydro-1*H*-carbazol-3-amine **(Iac);**
2-[(2*R*,3*R*,4*R*)-3-amino-2-(4-méthylphényl)-2,3,4,9-tétrahydro-1*H*-carbazol-4-yl]éthan-1-ol **(Iad);**
(2*R*,3*R*,4*R*)-4-(2-Méthoxyéthyl)-2-(4-méthoxyphényl)-2,3,4,9-tétrahydro-1*H*-carbazol-3-amine (**Iae**);
(2*R*,3*R*,4*R*)-4-{2-[(Cyclopropylméthyl)amino]éthyl}-6-méthoxy-2-(4-méthylphényl)-2,3,4,9-tétrahydro-1*H*-carbazol-3-amine (**Iaf**);
(2*R*,3*R*,4*R*)-6-Chloro-4-{2-[(cyclopropylméthyl)amino]éthyl}-2-(4-méthylphényl)-2,3,4,9-tétrahydro-1*H*-carbazol-3-amine (**Iag**);
(2*R*,3*R*,4*R*)-6-Chloro-4-{2-[(cyclopropylméthyl)amino]éthyl}-2-(4-méthoxyphényl)-2,3,4,9-tétrahydro-1*H*-carbazol-3-amine (**Iah**);
(2*R*,3*R*,4*R*)-4-{2-[(Cyclopropylméthyl)amino]éthyl}-6-méthyl-2-(4-méthylphényl)-2,3,4,9-tétrahydro-1*H*-carbazol-3-amine **(Iai);**
(2*R*,3*R*,4*R*)-4-{2-[(Cyclopropylméthyl)amino]éthyl}-6-fluoro-2-(4-méthylphényl)-2,3,4,9-tétrahydro-1*H*-carbazol-3-amine (**Iaj**);
(2*R*,3*R*,4*R*)-6-Chloro-4-{2-[(cyclopropylméthyl)amino]éthyl}-*N*-méthyl-2-(4-méthylphényl)-2,3,4,9-tétrahydro-1*H*-carbazol-3-amine (**Iak**); et
(2*R*,3*R*,4*R*)-4-{2-[(Cyclopropylméthyl)amino]éthyl}-9-méthyl-2-(4-méthylphényl)-2,3,4,9-tétrahydro-1*H*-carbazol-3-amine **(Ial).**

10. Une composition pharmaceutique qui comprend une quantité thérapeutiquement efficace d'un composé de formule (**I**) ou d'un sel pharmaceutiquement acceptable de celui-ci, ou de tout énantiomère ou mélanges d'énantiomères, soit du composé de formule **(I),** soit de son sel pharmaceutiquement acceptable tel que défini dans l'une quelconque des revendications 1 à 9, avec un ou plusieurs excipients ou véhicules pharmaceutiquement acceptables.

11. Un composé de formule **(I)** selon l'une quelconque des revendications 1 à 9 ou une composition pharmaceutique selon la revendication 10, pour son utilisation en tant que médicament.

12. Un composé de formule **(I)** selon l'une quelconque des revendications 1 à 9 ou une composition pharmaceutique selon la revendication 10, pour son utilisation dans le traitement et/ou la prévention d'un cancer médié par une dérégulation de NPM1.

13. Le composé pour son utilisation selon la revendication 12, dans lequel le cancer est choisi parmi le groupe constitué de la leucémie à lignage mixte (MLL), la leucémie liée à MLL, la leucémie associée à MLL, la leucémie MLL-positive, la leucémie induite par MLL, la leucémie à lignage mixte réarrangée (MLL-r), la leucémie associée à un réarrangement de MLL ou à un réarrangement du gène MLL, la leucémie aiguë, la leucémie chronique, la leucémie indolente, la leucémie lymphoblastique, la leucémie lymphoïde, la leucémie myéloïde, la leucémie myélogène, la leucémie infantile, la leucémie aiguë lymphoblastique (LLA), la leucémie aigüe myéloïde (LMA), le lymphome anaplasique à grandes cellules (LAGC), la leucémie aiguë promyélocytaire (LPA), la leucémie granulocytaire aiguë, la leucémie non lymphoïde aiguë, la leucémie lymphoïde chronique (LLC), la leucémie myéloïde chronique (LMC), la leucémie liée à la thérapie, le syndrome myélodysplasique (SMD), la maladie myéloproliférative (MPD), la néoplasie myéloproliférative (MPN), la néoplasie à plasmocytes, le myélome multiple, la myélodysplasie, le lymphome cutané à cellules T, la néoplasie lymphoïde, le lymphome associé au SIDA, le thymome, le carcinome thymique, le mycosis fongoïde, le syndrome d'Alibert-Bazin, le granulome fongoïde, le syndrome de Sézary, la leucémie à tricholeucocytes, la leucémie prolymphocytaire à cellules T (T-PLL), la leucémie à grands lymphocytes granuleux, la leucémie méningée, la leptoméningite leucémique, la méningite leucémique, le myélome multiple, le lymphome de Hodgkin, le lymphome non hodgkinien (lymphome malin) ou la macroglobulinémie de Waldenström chez un mammifère qui en a besoin.

14. Le composé pour son utilisation selon l'une quelconque des revendications 12 et 13, dans lequel le cancer est choisi parmi la leucémie aigüe myéloïde (LMA), le lymphome anaplasique à grandes cellules (LAGC) et la leucémie aiguë promyélocytaire (LPA).

15. Un procédé de préparation du composé de formule **(I)** ou d'un sel pharmaceutiquement acceptable de celui-ci, ou de tout énantiomère ou mélange d'énantiomères, soit du composé de formule **(I),** soit de son sel pharmaceutiquement acceptable, tel que défini dans l'une quelconque des revendications 1 à 9, qui comprend :
a) l'amination réductrice de l'intermédiaire de formule (3) avec une amine de formule R₄NH₂ pour donner un intermédiaire de formule (4), où R₁, R₂ et R₄ sont tels que définis dans la revendication 1, et Y est un groupe méthyle, acétyle ou un groupe protecteur ; 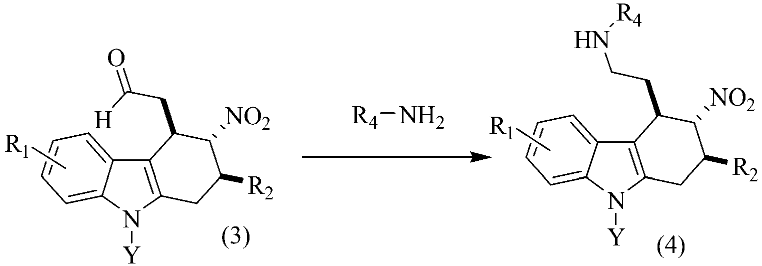
b) la réduction ultérieure du groupe nitro de l'intermédiaire (4), facultativement suivie de l'élimination du groupe protecteur, conduisant à des composés finaux de formule **(I),** où X est NH, R₃ est H, et R₁, R₂, R₄ et R₅ sont tels que définis dans la revendication 1 ;
ou, en variante,
a') l'amination réductrice de l'intermédiaire de formule (3) avec une amine de formule R₄NH₂ pour obtenir un intermédiaire de formule (4), où R₁, R₂ et R₄ sont tels que définis dans la revendication 1, et Y est un méthyle, un acétyle ou un groupe protecteur ; 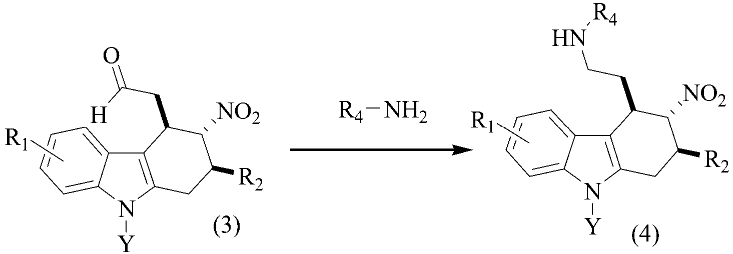
b') la réduction du groupe nitro précédée ou suivie d'une protection de *N* pour obtenir l'intermédiaire de formule (5) ; 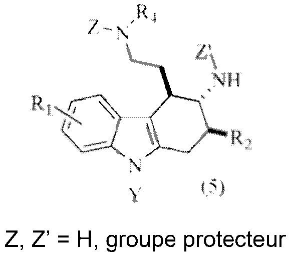
c') la N-alkylation ou la N-acylation de l'intermédiaire (5) pour obtenir l'intermédiaire (6) ; 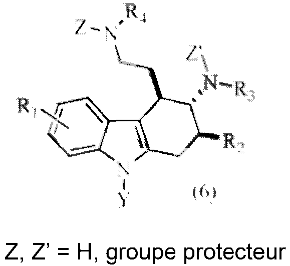
d') la déprotection finale fournit des composés de formule **(I),** où X est NH, R₃ est différent de H, et R₁, R₂, R₄ et R₅ sont tels que définis dans la revendication 1 ;
ou
a") la réduction du groupe nitro de l'intermédiaire de formule (7) suivie d'une N-alkylation ou d'une *N*-acylation et d'une déprotection facultatives donnent les composés de formule **(I),** où X est O, R₄ est H, et R₁, R₂, R₃ et R₅ sont tels que définis dans la revendication 1 ; 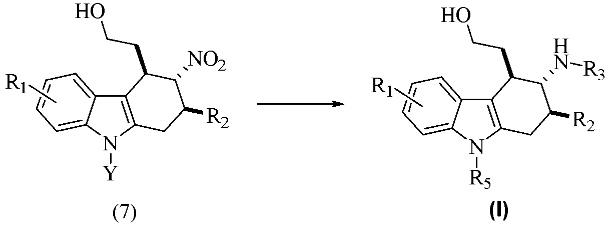 ou, en variante,
a‴) la réduction du groupe nitro et la protection de *N* in situ de l'intermédiaire de formule (7) fournissent l'intermédiaire de formule (8) ; 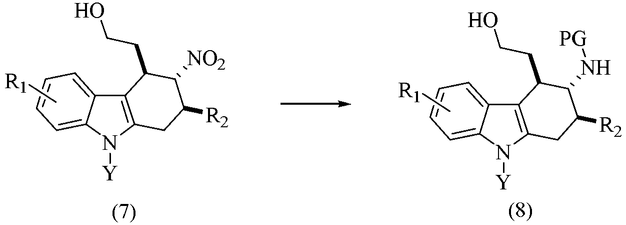
b‴) la O-alkylation avec un dérivé halogéné de formule R₄-halogène, où R₄ est tel que défini dans la revendication 1, pour obtenir l'intermédiaire (9) ; 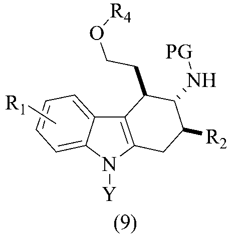
c‴) la déprotection finale et la *N*-alkylation ou la *N*-acylation facultative de l'intermédiaire (9) fournissent des composés de formule **(I),** où X est O, R₄ est différent de H, et R₁, R₂, R₃ et R₅ sont tels que définis dans la revendication 1.
